(19) Europäisches Patentamt
European Patent Office
Office européen des brevets

(11) EP 3 161 144 B1

(12) # EUROPÄISCHE PATENTSCHRIFT

(45) Veröffentlichungstag und Bekanntmachung des Hinweises auf die Patenterteilung:
**18.09.2019 Patentblatt 2019/38**

(21) Anmeldenummer: **15731902.1**

(22) Anmeldetag: **24.06.2015**

(51) Int Cl.:
***C12P 33/00*** *(2006.01)* ***C12N 9/04*** *(2006.01)*

(86) Internationale Anmeldenummer:
**PCT/EP2015/064264**

(87) Internationale Veröffentlichungsnummer:
**WO 2015/197698 (30.12.2015 Gazette 2015/52)**

(54) **VERFAHREN ZUR BIOKATALYTISCHEN GANZZELLREDUKTION VON DEHYDROCHOLSÄUREVERBINDUNGEN, SOWIE 7BETA-HYDROXYSTEROID DEHYDROGENASE-MUTANTEN**

METHOD FOR THE BIOCATALYTIC WHOLE CELL REDUCTION OF DEHYDROCHOLIC ACID COMPOUNDS, AND 7BETA-HYDROXYSTEROID DEHYDROGENASE MUTANTS

PROCÉDÉ DE RÉDUCTION BIO-CATALYTIQUE PAR DES CELLULES ENTIÈRES DE COMPOSÉS DU TYPE ACIDE DÉHYDROCHOLIQUE, ET MUTANTES DE 7BÉTA-HYDROXYSTÉROÏDE DÉSHYDROGÉNASES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.06.2014 EP 14173747**
**26.08.2014 EP 14182290**

(43) Veröffentlichungstag der Anmeldung:
**03.05.2017 Patentblatt 2017/18**

(73) Patentinhaber: **PharmaZell GmbH**
**83064 Raubling (DE)**

(72) Erfinder:
- **WEUSTER-BOTZ, Dirk**
  **80634 München (DE)**
- **SUN, Boqiao**
  **80339 München (DE)**
- **QUIRIN, Christian**
  **83098 Brannenburg (DE)**

(74) Vertreter: **Reitstötter Kinzebach**
**Patentanwälte**
**Sternwartstrasse 4**
**81679 München (DE)**

(56) Entgegenhaltungen:
**WO-A1-2012/080504**

- **BOQIAO SUN ET AL: "Multi-enzymatic one-pot reduction of dehydrocholic acid to 12-keto-ursodeoxycholic acid with whole-cell biocatalysts", BIOTECHNOLOGY AND BIOENGINEERING, Bd. 110, Nr. 1, 18. Januar 2013 (2013-01-18), Seiten 68-77, XP055169454, ISSN: 0006-3592, DOI: 10.1002/bit.24606**
- **H.-J. MOON ET AL: "Molecular Determinants of the Cofactor Specificity of Ribitol Dehydrogenase, a Short-Chain Dehydrogenase/Reductase", APPLIED AND ENVIRONMENTAL MICROBIOLOGY, Bd. 78, Nr. 9, 17. Februar 2012 (2012-02-17), Seiten 3079-3086, XP055233711, US ISSN: 0099-2240, DOI: 10.1128/AEM.07751-11**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 161 144 B1

## Beschreibung

Die Erfindung betrifft neuartige 7β-Hydroxysteroid Dehydrogenase-Mutanten, die für diese Enzym-Mutanten kodierenden Sequenzen, und deren Verwendung bei enzymatischen Umsetzungen von Cholsäureverbindungen (Gallensäure-Derivaten), und insbesondere bei der Herstellung von Ursodesoxycholsäure (UDCS); Gegenstand der Erfindung sind auch neuartige Verfahren zur Synthese von UDCS unter Verwendung der Enzym-Mutanten; sowie insbesondere ein weiter verbessertes Verfahren zur Herstellung von UDCS unter Verwendung rekombinanter Ganzzell-Biokatalysatoren.

## Hintergrund der Erfindung:

Zur medikamentösen Behandlung von Gallensteinleiden werden seit vielen Jahren u. a. die Gallensalz-Wirkstoffe Ursodesoxycholsäure (UDCS oder UDCA) und das zugehörige Diastereomer Chenodesoxycholsäure (CDCS oder CDCA) eingesetzt. Beide Verbindungen unterscheiden sich lediglich durch die Konfiguration der Hydroxygruppe am C-Atom 7 (UDCS: β -Konfiguration, CDCS: α-Konfiguration). Zur Herstellung von UDCS sind im Stand der Technik verschieden Verfahren beschreiben, welche rein chemisch durchgeführt werden oder aus einer Kombination chemischer und enzymatischer Verfahrensschritte bestehen. Ausgangspunkt ist jeweils Cholsäure (CS oder CA) oder die aus Cholsäure hergestellte CDCS.

So kann die klassisch chemische Methode zur UDCS Herstellung wie folgt schematisch dargestellt werden:

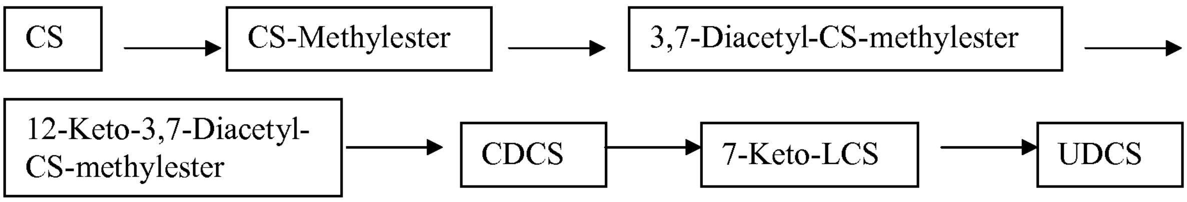


Ein gravierender Nachteil ist unter anderem folgender: da die chemische Oxidation nicht selektiv ist, müssen die Carboxy-Gruppe und die 3α und 7α-Hydroxy-Gruppe durch Veresterung geschützt werden.

Ein alternatives chemisch/enzymatische Verfahren basierend auf der Verwendung des Enzyms 12α-Hydroxysteroid Dehydrogenase (12α-HSDH) kann wie folgt dargestellt werden und ist z.B. beschrieben in der Patentanmeldung PCT/EP2009/002190 (publiziert als WO2009118176) der vorliegenden Anmelderin.

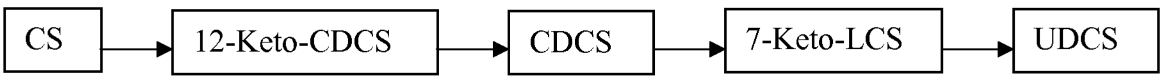


Die 12α-HSDH oxidiert dabei CS selektiv zu 12-Keto-CDCS. Die zwei nach der klassisch chemischen Methode erforderlichen Schützschritte entfallen dabei.

Weiterhin wird von Monti, D., et al., (One-Pot Multienzymatic Synthesis of 12-Ketoursodeoxycholic Acid: Subtle Cofactor Specificities Rule the Reaction Equilibria of Five Biocatalysts Working in a Row. Advanced Synthesis & Catalysis, 2009) ein alternatives enzymatisch-chemisches Verfahren beschrieben, das wie folgt schematisch darstellbar ist:

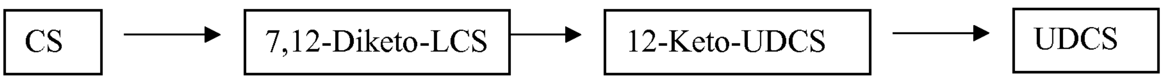


Die CS wird zuerst von 7α-HSDH aus *Bacteroides fragilis* ATCC 25285 (Zhu, D., et al., Enzymatic enantioselective reduction of -ketoesters by a thermostable 7 -hydroxysteroid dehydrogenase from Bacteroides fragilis. Tetrahedron, 2006. 62(18): p. 4535-4539) und 12α-HSDH zu 7,12-Diketo-LCS oxidiert. Diese beiden Enzyme sind jeweils NADH abhängig. Nach der Reduktion durch 7β-HSDH (NADPH abhängig) aus *Clostridium absonum* ATCC 27555 (DSM 599) (MacDonald, I.A. and P.D. Roach, Bile induction of 7 alpha- and 7 beta-hydroxysteroid dehydrogenases in Clostridium absonum. Biochim Biophys Acta, 1981. 665(2): p. 262-9) entsteht 12-Keto-UDCS. Durch Wolff-Kishner-Reduktion wird das Endprodukt erhalten. Nachteilig bei diesem Verfahren ist, dass wegen der Gleichgewichtslage der katalysieren Reaktion eine komplette Umsetzung nicht möglich ist, und dass für die erste Stufe der Umsetzung zwei unterschiedliche Enzyme eingesetzt werden müssen, was das Verfahren verteuert. Zur Kofaktor-Regenerierung werden Lactat Dehydrogenase (LDH; zur Regenerierung von $NAD^+$) und Glucose Dehydrogenase (GlcDH oder GDH, zur Regenerierung

von NADPH) eingesetzt. Nachteilig bei der dort verwendeten Kofaktor-Regenerierung ist, dass das entstehende Ko-Produkt nur sehr schwer aus dem Reaktionsgemisch zu entfernen ist, so dass das Reaktionsgleichgewicht nicht positiv beeinflusst werden kann, was eine unvollständige Umsetzung des Edukts bedingt.

Eine 7β-HSDH aus Stamm *Collinsella aerofaciens* ATCC 25986 (DSM 3979; ehemalige *Eubacterium aerofaciens*) wurde im Jahr 1982 von Hirano und Masuda beschrieben (Hirano, S. and N. Masuda, Characterization of NADP-dependent 7 beta-hydroxysteroid dehydrogenases from Peptostreptococcus productus and Eubacterium aerofaciens. Appl Environ Microbiol, 1982. 43(5): p. 1057-63). Sequenzinformationen wurden zu diesem Enzym nicht offenbart. Das durch Gelfiltration bestimmte Molekulargewicht betrug 45,000 Da (vgl. Hirano, Seite 1059, linke Spalte). Weiterhin konnte für das dortige Enzym die Reduktion der 7-Oxo-Gruppe zur 7β-Hydroxygruppe nicht beobachtet werden (vgl. Hirano, Seite 1061, Diskussion 1. Absatz). der Fachmann erkennt somit, dass das von Hirano et al beschriebene Enzym nicht zur Katalyse der Reduktion von Dehydrocholsäure (DHCS oder DHCA) in 7-Position zu 3,12-Diketo-UDCS geeignet ist.

In der WO2011/064404 der Anmelderin wird eine neuartige 7β-HSDH aus *Collinsella aerofaciens* ATCC 25986 beschrieben, welche unter anderem ein Molekulargewicht (bei SDS-Gelelektrophorese) von etwa 28-32 kDa, ein Molekulargewicht (bei Gelfiltration, unter nicht denaturierenden Bedingungen, wie insbesondere ohne SDS): von etwa 53 bis 60 kDa, und die Befähigung zur stereoselektiven Reduktion der 7-Carbonyl-Gruppe von 7-Keto-LCS in eine 7β-Hydroxy-Gruppe aufweist.

Außerdem wird in der WO2011/064404 ein Verfahren zur UDCS Herstellung bereitgestellt, welches schematisch wie folgt darstellbar ist:

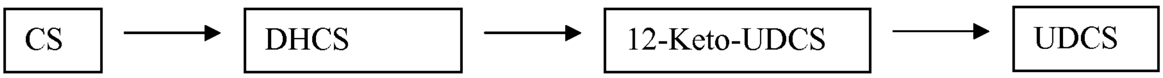


Dabei erfolgt die Oxidation von CS auf klassisch chemischem Weg in einfacher Weise. Die DHCS wird von Enzympaar 7β-HSDH und 3α-HSDH einzeln nacheinander oder in einem Topf zu 12-Keto-UDCS reduziert. Kombiniert mit der Wolff-Kishner-Reduktion kann UDCS somit in nur drei Schritte aus CS synthetisiert werden. Während die 7β-HSDH vom Kofaktor NADPH abhängig ist, benötigt die 3α-HSDH den Kofaktor NADH. Die Verfügbarkeit von Enzympaaren mit Abhängigkeit vom gleichen Kofaktor oder erweiterter Anhängigkeit, (z.B. von den Kofaktoren NADH und NADPH) wäre von Vorteil, weil damit die Kofaktor-Regenerierung vereinfacht werden könnte.

In der WO2012/080504 der Anmelderin wird insbesondere ein Ganzzellverfahren zur biokatalytischen Reduktion von Dehydrocholsäure-Verbindungen (DHCS) und insbesondere ein neuartiges Verfahren zur Herstellung von UDCS unter Verwendung von rekombinanten Ganzzell-Katalysatoren beschrieben, welche 7β-HSDH und 3α-HSDH exprimieren und wobei die enzymatischen Reduktionsschritte zur Cofaktor-Regenerierung mit einem cofaktorregenerierenden Enzym, wie z.B. einer geeigneten Glucosedehydrogenase (GDH) gekoppelt sind.

Entscheidend für die Effizienz einer enzymatischen Synthese ist die Art und Weise, in der die benötigten Enzyme eingesetzt werden. Die Ganzzellbiokatalyse stellt hierbei einen probaten Ansatz dar. Dabei werden die oftmals heterologen Enzyme innerhalb des Wirtsorganismus überexprimiert und die Zelle wird als Ganzes als Biokatalysator eingesetzt. Ein spezieller Ganzzellbiokatalysator der WO2012/080504, exprimiert heterolog eine, z.B. NADPH-abhängige, 7β-HSDH aus *Collinsella aerofaciens,* eine, z.B. NADH-abhängige 3α-HSDH aus *Comamonas testosteroni* und eine sowohl NADH als auch NADPH utilisierende GDH aus *Bacillus subtilis* und wird als Ganzzellbiokatalysator für die Reduktion von DHCS zu 12-keto-Ursodesoxycholsäure (12-keto-UDCS) eingesetzt. Dort wurden z.B. 17,7 $g_{BTM}$ $L^{-1}$ Biokatalysator eingesetzt, um 100 mM DHCS zu 98 % zu 12-keto-UDCS umzusetzen. WO2012/080504 offenbart auch die Herstellung von Mutanten des Enzyms 7β-HSDH aus C. *aerofaciens*, welche die Änderung der Kofaktorspezifität von NADPH zu NADH bedingen, z.B. die Mutanten G39A, G39D oder die Doppelmutante G39D/R40I. Da Biokatalysatoren einen Hauptkostenfaktor in diesem Produktionsprozess darstellen, besteht die aktuelle technische Herausforderung im Auffinden von technischen Lösungen, bei denen die Verfahrenskosten gesenkt werden können, z.B. indem die Biokatalysatoren durch andere Stoffe teilweise ersetzt werden.

Eine Aufgabe besteht daher in der Bereitstellung weiter verbesserter 7β-HSDHs. Insbesondere sollten Enzym-Mutanten bereitgestellt werden, welche noch vorteilhafter zur enzymatischen oder mikrobiellen (biokatalytischen) Herstellung von UDCS über die stereospezifische Reduktion von DHCS in 7-Position eingesetzt werden können, und insbesondere eine veränderte Kofaktor-Nutzung (insbesondere eine verbesserte NADH-Spezifität) aufweisen.

## Kurzfassung der Erfindung:

Obige Aufgabe konnte durch Erzeugung und Charakterisierung von Mutanten der 7β-HSDH aus aeroben Bakterien der Gattung *Collinsella*, insbesondere des Stammes *Collinsella aerofaciens*, mit verbesserter NADH-Spezifität gelöst, wobei die Mutante ebenfalls bei der (enzymatischen oder mikrobiellen) Umsetzung, insbesondere im Rahmen eines Ganzzellprozesses, von Cholsäureverbindungen, insbesondere bei der Herstellung von UDCS vorteilhaft Verwendung finden.

## Figurenbeschreibung:

Figur 1a zeigt die Aminosäuresequenz der 7β-HSDH aus *Collinsella aerofaciens* und Figur 1b die kodierende Nukleinsäuresequenz zur Aminosäuresequenz von Figur 1a; Figur 1c zeigt die Aminosäuresequenz der 3α-HSDH aus *Comanomonas testosteroni* und Figur 1d die kodierende Nukleinsäuresequenz zur Aminosäuresequenz von Figur 1c; Figur 1 e zeigt im Vergleich die Aminosäuresequenzen von 7β-HSDH Wildtyp (WT) und verschiedener Mutanten: 7β-HSDH D, 7β-HSDH DF, 7β-HSDH DFK und 7β-HSDH DFKG

Figur 2 zeigt eine schematische Darstellung der zweistufigen enzymatischen Reduktion von Dehydrocholsäure zu 12-keto-Ursodesoxycholsäure unter Verwendung eines Ganzzellbiokatalysators, wobei die Teilschritte der Reduktion durch eine 7β-HSDH und einen 3α-HSDH katalysiert werden. Der verbrauchte Kofaktor (NADH bzw. NADPH) wird von einer ebenfalls vom Ganzzellkatalysator exprimierten GDH unter Verbrauch von Glucose (Bildung von Glucono-1,5-lacton) regeneriert.

Figur 3 zeigt die Vektorkarten der Expressionsplasmide p7(A)T3rG (SEQ ID NO: 18), p7(A)T3rG-K (SEQ ID NO: 19) und p7(A)T3TG (SEQ ID NO: 20).

Figur 4 zeigt die Reaktionsverläufe der Biokatalysereaktion mit den Ganzzellbiokatalysatoren *E. coli* BL49 p7(A)T3rG, *E. coli* BL21 ΔhdhA p7(A)T3rG-K und *E. coli* BL49 p7(A)T3TG bei Einsatz von 1 $g_{BTM}$ $L^{-1}$ Biokatalysator, 0,05 mM NAD und 0,01 mM NADP (X = 67).

Figur 5 zeigt den Reaktionsverlauf der Biokatalysereaktion mit den Ganzzellbiokatalysator *E. coli* BL49 p7(A)T3rG bei Einsatz von 3,5 $g_{BTM}$ $L^{-1}$ Biokatalysator und 0,025 mM NAD (X = 147,5).

Figur 6 zeigt den Reaktionsverlauf der Biokatalysereaktion mit den Ganzzellbiokatalysator *E. coli* BL49 p7(A)T3rG bei Einsatz von 1,75 $g_{BTM}$ $L^{-1}$ Biokatalysator, 0,025 mM NAD und 0,01 mM NADP (X = 89,5).

Figur 7 zeigt den Reaktionsverlauf der Biokatalysereaktion mit den Ganzzellbiokatalysator *E. coli* BL21 ΔhdhA p7(A)T3rG-K bei Einsatz von 1 $g_{BTM}$ $L^{-1}$ Biokatalysator, 0,04 mM NAD und 0,0075 mM NADP (X = 61).

Figur 8 zeigt das Aminosäuresequenz-Alignment der unmodifizierten 7β-HSDH, der aus dem Stand der Technik bekannten Enzymmutanten 7β-HSDH G39D und 7β-HSDH G39D R40I mit speziellen erfindungsgemäßen Enzymmutanten. Positionen, die von der ursprünglichen Sequenz abweichen sind unterstrichen dargestellt.

Figur 9 zeigt die enzymkinetische Untersuchung erfindungsgemäßer 7β-HSDH-Mutanten. Auftragung der spezifischen Enzymaktivität gegen unterschiedliche eingesetzte Substratkonzentrationen (DHCA/DHCS) bei einer konstanten Kofaktorkonzentration 0,5 mM NADH.

Figur 10 zeigt die enzymkinetische Untersuchung erfindungsgemäßer 7β-HSDH-Mutanten. Auftragung der spezifischen Enzymaktivität gegen unterschiedliche eingesetzte Kofaktorkonzentrationen (NADH) bei einer konstanten Substratkonzentration von 10 mM DHCA/DHCS.

Figur 11 zeigt Ganzzellbiotransformationen mit bei -20 °C und bei Raumtemperatur/4 °C gelagerten Zellen des Ganzzellbiokatalysators E. coli BLLiu p7(A)T3TG-K. Die oberen zwei Ansätze wurden nach Standardbedingungen durchgeführt: 70 mM DHCA, 350 mM Glucose, OD 2 Zellen, 50 μM NAD, 10 μM NADP, 1 mM MgCl2, 50 mM KPi-Puffer (pH 7,0), 30 °C. Bei dem unteren Ansatz wurde bei sonst gleichbleibenden Bedingungen die NAD-Konzentration auf 100 μM verdoppelt. Der pH wurde halbstündlich manuell mit NaOH-Lösung (5 M) auf den Ausgangswert eingestellt. Gezeigt sind Mittelwerte aus Dreifachansätzen, die Standardabweichungen sind durch Fehlerindikatoren dargestellt.

Figur 12 zeigt den Vergleich der NADH-abhängigen 7β-HSDH-Mutanten D, DF, DFK und DKFG mit dem Wildtyp (WT) Enzym.. Die Reaktionsbedingungen waren jeweils 0,2 mg mL-1 7β-HSDH, 10 U mL-1 GDH, 0,5 mM NAD, 50 mM DHCA, 200 mM Glucose, 500 mM Kaliumphosphat, pH 8,0, 30 °C. Die Reaktionen wurden in geschüttelten DeepWell-Platten ohne pH-Kontrolle im stark gepufferten System durchgeführt. Standardabweichungen der Dreifachansätze sind durch Fehlerindikatoren gekennzeichnet.

Figur 13 zeigt die Reaktionsverläufe von zweistufigen Biotransformationen mit den NADH-abhängigen 7β-HSDH-Mutanten DFK und DFKG. Die Reaktionsbedingungen waren jeweils 0,2 mg mL-1 7β-HSDH, 1 U mL-1 3α-HSDH, 10 U mL-1 GDH, 0,5 mM NAD, 50 mM DHCA, 200 mM Glucose, 500 mM Kaliumphosphat, pH 8,0, 30 °C. Die Reaktionen wurden in geschüttelten DeepWell-Platten ohne pH-Kontrolle im stark gepufferten System durchgeführt. Standardabweichungen der Dreifachansätze sind durch Fehlerindikatoren gekennzeichnet.

## Spezielle Ausführungsformen der Erfindung

Die Erfindung betrifft insbesondere folgende spezielle Ausführungsformen:

1. 7β-Hydroxysteroiddehydrogenase (7β-HSDH), welche wenigstens die stereospezifische enzymatische Reduktion eines 7-Ketosteroids zum korrespondierenden 7-Hydroxysteroid katalysiert, wobei das Enzym je eine Mutation in den Positionen G39 und R40 von SEQ ID NO:2 sowie gegebenenfalls in der Positionen R41 und/ gegebenenfalls der Position K44 von SEQ ID NO:2 oder in den jeweils korrespondierenden Sequenzpositionen einer davon abgeleiteten Aminosäuresequenz mit wenigstens 80% Sequenzidentität zu SEQ ID NO:2 aufweist, wobei das Enzym die Doppelmutation G39D/R40F umfasst.

2. 7β-Hydroxysteroiddehydrogenase (7β-HSDH), welche wenigstens die stereospezifische enzymatische Reduktion eines 7-Ketosteroids zum korrespondierenden 7- Hydroxysteroid katalysiert, wobei das Enzym je eine Mutation in den Positionen G39, R40 und R41, sowie gegebenenfalls in der Position K44 von SEQ ID NO:2 oder in einer davon abgeleiteten Aminosäuresequenz mit wenigstens 80% Sequenzidentität zu SEQ ID NO:2 aufweist, wobei das Enzym umfasst:

a) die Dreifachmutation G39D/R40F/R41$X_1$, wobei $X_1$ für einen beliebigen anderen Aminosäurerest steht; oder
b) die Vierfachmutation G39D/R40F/R41 $X_1$/K44$X_2$, wobei $X_1$ für einen beliebigen anderen Aminosäurerest und $X_2$ für einen beliebigen anderen Aminosäurerest steht.

3. 7β-HSDH nach Ausführungsform 2, umfassend die Dreifachmutation G39D/R40F/R41 K.

4. 7β-HSDH nach Ausführungsform 2, umfassend die Vierfachmutation G39D/R40F/R41 K/K44G.

5. Nukleotidsequenz kodierend für eine 7β-HSDH nach einer der Ausführungsformen 1 bis 4.

6. Expressionskassette, umfassend unter der Kontrolle wenigstens einer regulativer Sequenz wenigstens eine Nukleotidsequenz nach Ausführungsform 5.

7. Expressionsvektor, umfassend wenigstens eine Expressionskassette nach Ausführungsform 6.

8. Rekombinanter Mikroorganismus, der wenigstens eine Nukleotidsequenz gemäß Ausführungsform 5 oder wenigstens eine Expressionskassette nach Ausführungsform 6 oder wenigstens einen Expressionsvektor nach Ausführungsform 7 trägt.

9. Rekombinanter Mikroorganismus nach Ausführungsform 8, der zusätzlich gegebenenfalls die kodierende Sequenz für wenigstens ein weiteres Enzym, ausgewählt unter Hydroxysteroiddehydrogenasen (HSDH) und zur Cofaktorregenerierung geeignete Dehydrogenasen, trägt.

10. Rekombinanter Mikroorganismus nach Ausführungsform 9, wobei die weitere HSDH ausgewählt ist unter 3α-HSDHs; und die Dehydrogenase ausgewählt ist unter NADH-regenerierenden Enzymen, wie NADH Dehydrogenasen, Alkoholdehydrogenasen (ADH), und NADH regenerierenden Formiatdehydrogenasen (FDH), sowie Glucosedehydrogenase (GDH), Glucose-6-Phosphat-Dehydrogenase (G-6-PDH), oder Phosphit-Dehydrogenasen (PtDH), sowie NADH regenerierenden Glucosedehydrogenasen (GDH).

11. Rekombinanter Mikroorganismus nach einer der Ausführungsformen 8 bis 10 welcher ein 7α-HSDH knock-out Stamm ist.

12. Verfahren zur Herstellung von Ursodesoxycholsäure (UDCS) der Formel (1)

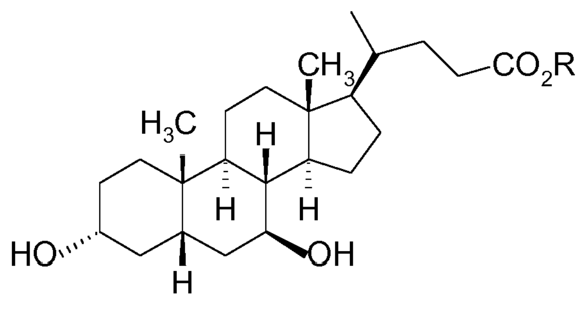

(1)

worin
R für Alkyl, H, ein Alkalimetallion oder $N(R^3)_4^+$ steht, worin die Reste $R^3$ gleich oder verschieden sind und für H oder Alkyl stehen, oder die Gruppe $-CO_2R$ durch die Säureamid-Gruppe $-CONR^1R^2$, ersetzt ist, worin $R^1$ und $R^2$ unabhängig voneinander für einen Alkylrest stehen;
wobei man

a) gegebenenfalls eine Cholsäure (CS) der Formel (2)

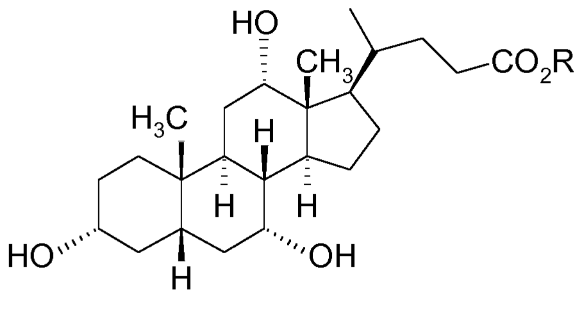

(2)

worin R die oben angegebenen Bedeutungen besitzt oder die Gruppe $-CO_2R$ durch die Säureamid-Gruppe $-CONR^1R^2$, wie oben definiert ersetzt ist,
zur Dehydrocholsäure (DHCS) der Formel (3)

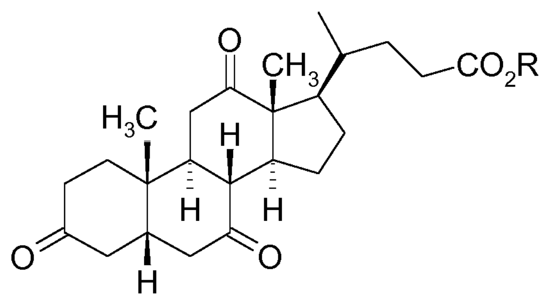

(3)

worin R die oben angegebenen Bedeutungen besitzt, oder die Gruppe $-CO_2R$ durch die Säureamid-Gruppe $-CONR^1R^2$, wie oben definiert ersetzt ist, oxidiert, wie z.B. chemisch oder enzymatisch, insbesondere chemisch oxidiert;
b) DHCS in Gegenwart wenigstens einer 7β-HSDH-Mutante gemäß der Definition in einer der Ausführungsformen 1 bis 4 und in Gegenwart wenigstens einer 3α-HSDH zur korrespondierenden 12-Keto-ursodesoxycholsäure (12-Keto-UDCS) der Formel (5)

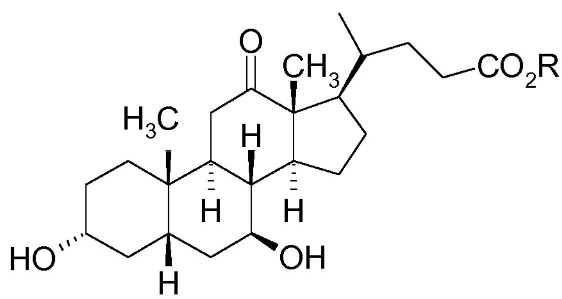

(5)

worin R die oben angegebenen Bedeutungen besitzt, oder die Gruppe $-CO_2R$ durch die Säureamid-Gruppe $-CONR^1R^2$, wie oben definiert ersetzt ist, insbesondere in Gegenwart und unter Verbrauch von NADH und /oder NADPH
reduziert und anschließend
c) 12-Keto-UDCS der Formel (5) chemisch zu UDCS reduziert; und
d) das Reaktionsprodukt gegebenenfalls weiter aufreinigt.

13. Verfahren zur Herstellung von UDCS der Formel (1)

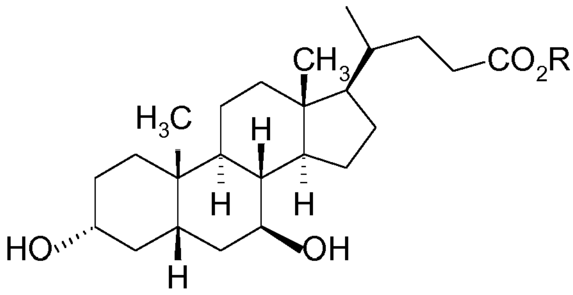

(1)

worin
R für Alkyl, $NR^1R^2$, H, ein Alkalimetallion oder $N(R^3)_4^+$ steht, worin die Reste $R^3$ gleich oder verschieden sind und für H oder Alkyl stehen oder die Gruppe $-CO_2R$ durch die Säureamid-Gruppe $-CONR^1R^2$, wie oben definiert ersetzt ist wobei man

a) gegebenenfalls eine CS der Formel (2)

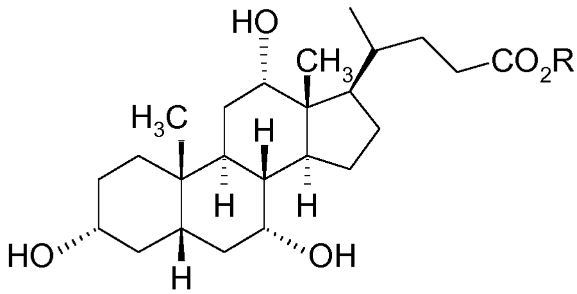

(2)

worin R die oben angegebenen Bedeutungen besitzt, oder die Gruppe $-CO_2R$ durch die Säureamid-Gruppe $-CONR^1R^2$, wie oben definiert ersetzt ist, zur DHCS der Formel (3)

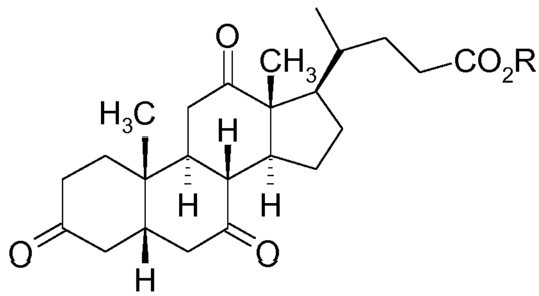

(3)

worin R die oben angegebenen Bedeutungen besitzt, oder die Gruppe $-CO_2R$ durch die Säureamid-Gruppe $-CONR^1R^2$ wie oben definiert ersetzt ist oxidiert, wie z.B. chemisch oder enzymatisch, insbesondere chemisch oxidiert;

b) DHCS in Gegenwart wenigstens einer 7β-HSDH und in Gegenwart wenigstens einer 3α-HSDH zur korrespondierenden 12-Keto UDCS der Formel (5)

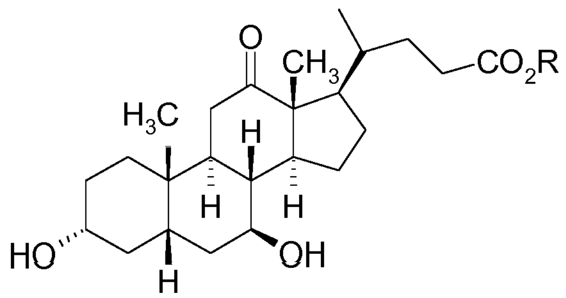

(5)

worin R die oben angegebenen Bedeutungen besitzt, oder die Gruppe $-CO_2R$ durch die Säureamid-Gruppe $-CONR^1R^2$ wie oben definiert ersetzt ist, reduziert und anschließend

c) 12-Keto-UDCS der Formel (5) chemisch zu UDCS reduziert; und

d) das Reaktionsprodukt gegebenenfalls weiter aufreinigt;

wobei die Umsetzungen des Schritts b) in Gegenwart eines rekombinanten Mikroorganismus nach einer der Ausführungsformen 8 bis 11 erfolgen.

## Detaillierte Beschreibung der Erfindung

1. Allgemeine Definitionen und verwendete Abkürzungen

Ein "Ganzzellkatalysator" umfasst sowohl lebensfähige (vermehrungsfähige, in jedem Wachstumsstadium befindliche) als auch nicht mehr lebensfähige Mikroorganismen, insbesondere rekombinante Mikroorganismen, welche die zur Durchführung eines erfindungsgemäßen Verfahrens erforderlichen Enzymaktivitäten vollständig oder wenigstens teilweise, in exprimierter Form enthalten. Der Ganzzellkatalysator kann dabei zusätzlich eine durch chemische, mechanische oder sonstige Einwirkung (Temperatur, Lagerung) perforierte Zellwand aufweisen, um den Stoffaustausch (insbesondere Substrat, Produkt, Kofaktoren) mit dem umgebenden Reaktionsmedium weiter zu fördern.

Werden keine anderen Angaben gemacht, so bezeichnet der Begriff "7β-HSDH" ein Dehydrogenase-Enzym, welches wenigstens die stereospezifische und/oder regiospezifische Reduktion von DHCS oder 7,12-Diketo-UDCS (7,12-Diketo-LCS) zu 3,12-Diketo-UDCS oder 12-Keto-UDCS insbesondere unter stöchiometrischem Verbrauch von NADPH, und gegebenenfalls die entsprechende Umkehrreaktion katalysiert. Das Enzym kann dabei ein natives bzw. rekombinant hergestelltes Enzym sein. Das Enzym kann grundsätzlich im Gemisch mit zellulären, wie z.B. Proteinverunreinigungen, vorzugsweise aber in Reinform vorliegen. Geeignete Nachweisverfahren sind z.B. im folgenden experimentellen Teil beschrieben oder aus der Literatur bekannt (z.B. Characterization of NADP-dependent 7 beta-hydroxysteroid dehydrogenases from Peptostreptococcus productus and Eubacterium aerofaciens. S Hirano and N Masuda. Appl Environ Microbiol. 1982). Enzyme dieser Aktivität sind unter der EC Nummer 1.1.1.201 klassifiziert.

Werden keine anderen Angaben gemacht, so bezeichnet der Begriff "3α-HSDH" ein Dehydrogenase-Enzym, welches wenigstens die stereospezifische und/oder regiospezifische Reduktion von 3,12-Diketo-UDCS oder DHCS zu 12-Keto-UDCS oder 7,12-Diketo-UDCS (7,12-Diketo-LCS), insbesondere unter stöchiometrischem Verbrauch von NADH und/oder NADPH, und gegebenenfalls die entsprechende Umkehrreaktion katalysiert. Geeignete Nachweisverfahren sind z.B. im folgenden experimentellen Teil beschrieben oder aus der Literatur bekannt. Geeignete Enzyme sind z.B. aus Comanomonas testosteroni (z.B. ATCC11996) erhältlich. Eine NADPH abhängige 3α-HSDH ist z.B. aus der Nagern bekannt und ebenfalls einsetzbar. (Cloning and sequencing of the cDNA for rat liver 3 alphahydroxysteroid/dihydrodiol dehydrogenase, J E Pawlowski, M Huizinga and T M Penning, May 15, 1991 The Journal of Biological Chemistry, 266, 8820-8825). Enzyme dieser Aktivität sind unter der EC Nummer 1.1.1.50 klassifiziert.

Werden keine anderen Angaben gemacht, so bezeichnet der Begriff "GDH" ein Dehydrogenase-Enzym, welches wenigstens die Oxidation von β-D-Glucose zu D-Glucono-1,5-Lacton unter stöchiometrischem Verbrauch von $NAD^+$ und/oder $NADP^+$ sowie ggf. die entsprechende Umkehrreaktion katalysiert. Geeignete Enzyme sind z.B. aus *Bacillus subtilis* oder *Bacillus megaterium* erhältlich. Enzyme dieser Aktivität sind unter der EC Nummer 1.1.1.47 klassifiziert.

Werden keine anderen Angaben gemacht, so bezeichnet der Begriff "FDH" ein Dehydrogenase-Enzym, welches wenigstens die Oxidation von Ameisensäure (oder korrespondierenden Formiat-Salzen) zu Kohlendioxid unter stöchiometrischem Verbrauch von $NAD^+$ und/oder $NADP^+$, und gegebenenfalls die entsprechende Umkehrreaktion katalysiert. Geeignete Nachweisverfahren sind z.B. im folgenden experimentellen Teil beschrieben oder aus der Literatur bekannt.

Geeignete Enzyme sind z.B. aus *Candida boidinii, Pseudomonas sp*, oder *Mycobacterium vaccae* erhältlich. Enzyme dieser Aktivität sind unter der EC Nummer 1.2.1.2 klassifiziert.

Unter einer "Reinform" oder einem "reinen" oder "im wesentlichen reinen" Enzym versteht man erfindungsgemäß ein Enzym mit einem Reinheitsgrad von mehr als 80, vorzugsweise mehr als 90, insbesondere mehr als 95, und vor allem mehr als 99 Gew.-%, bezogen auf den Gesamtproteingehalt, bestimmt mit Hilfe üblicher Proteinnachweismethoden, wie z.B. der Biuret-Methode oder dem Proteinnachweis nach Lowry et al. (vgl. Beschreibung in R.K. Scopes, Protein Purification, Springer Verlag, New York, Heidelberg, Berlin (1982)).

Unter einem "Redoxäquivalent" versteht man eine als Elektronendonor bzw. Elektronenakzeptor brauchbare, niedermolekulare organische Verbindung, wie beispielsweise Nikotinamidderivate wie $NAD^+$ und $NADH^+$ bzw. deren reduzierte Formen NADH bzw. NADPH. "Redoxäquivalent" und "Kofaktor" werden im Kontext der vorliegenden Offenbarung als Synonym verwendet. So kann ein "Kofaktor" auch als "redoxfähiger Kofaktor", d.h. als Kofaktor, der in reduzierter und oxidierter Form vorliegen kann, umschrieben werden.

Unter einem "verbrauchten" Kofaktor versteht man diejenige reduzierte bzw. oxidierte Form des Kofaktors, die im Verlauf einer vorgegebene Reduktions- bzw. Oxidationsreaktion eines Substrats in die korrespondierende oxidierte bzw. reduzierte Form überführt wird. Durch Regenerierung wird die bei der Reaktion gebildete oxidierte bzw. reduzierte Kofaktor-Form wieder in die reduzierte bzw. oxidierte Ausgangsform zurück überführt, so dass diese für die Umsetzung des Substrats wieder zur Verfügung steht.

Unter einer "veränderten Kofaktor-Nutzung versteht man im Rahmen der vorliegenden Offenbarung eine qualitative oder quantitative Veränderung im Vergleich zu einer Referenz. Insbesondere ist eine veränderte Kofaktor-Nutzung durch Vornahme von Aminosäuresequenzmutationen zu beobachten. Diese Veränderung ist dann im Vergleich zum nicht-mutierten Ausgangsenzym feststellbar. Dabei kann die Aktivität in Bezug auf einen bestimmten Kofaktor durch Vornahme einer Mutation erhöht oder erniedrigt oder vollständig unterbunden werden. Eine veränderte Kofaktor-Nutzung umfasst aber auch Änderungen dergestalt, dass anstelle einer Spezifität für einen einzelnen Kofaktor nunmehr wenigstens ein weiterer, vom ersten Kofaktor verschiedener, zweiter Kofaktor nutzbar ist (d.h. es liegt eine erweiterte Kofaktor-Nutzung vor) Umgekehrt kann aber auch eine ursprünglich vorhandene Befähigung zur Nutzung zweier verschiedener Kofaktoren so abgeändert werden, dass Spezifität nur für einen dieser Kofaktoren erhöht bzw. für einen dieser Kofaktoren verringert oder vollständig aufgehoben wird. So kann beispielsweise ein Enzym, das vom Kofaktor NAD (NADH) abhängig ist, aufgrund einer Veränderung der Kofaktor-Nutzung nunmehr sowohl von NAD (NADH) als auch vom Kofaktor NADP (NADPH) abhängig sein oder die ursprüngliche Abhängigkeit von NAD (NADH) kann vollständig in eine Abhängigkeit von NADP (NADPH) überführt werden und umgekehrt.

Die Begriffe "$NAD^+$/NADH-Abhängigkeit" bzw. "$NADP^+$/NADPH-Abhängigkeit" sind, wenn nicht anders definiert, weit auszulegen. Diese Begriffe umfassen sowohl "spezifische" Abhängigkeiten, d.h. ausschließlich Abhängigkeit von $NAD^+$/NADH bzw. $NADP^+$/NADPH, als auch die Abhängigkeit der verwendeten Enzyme von beiden Kofaktoren, d.h. Abhängigkeit von $NAD^+$/NADH und $NADP^+$/NADPH.

Entsprechendes gilt für die verwendeten Begriffe "$NAD^+$/NADH-akzeptierend" bzw. "$NADP^+$/NADPH-akzeptierend".

Die Begriffe "$NAD^+$/NADH-regenerierend" bzw. "$NADP^+$/NADPH-regenerierend" sind, wenn nicht anders definiert, weit auszulegen. Diese Begriffe umfassen sowohl "spezifische" d.h. ausschließliche Befähigung verbrauchten Kofaktor $NAD^+$/NADH bzw. $NADP^+$/NADPH zu regenerieren, als auch die Befähigung beide Kofaktoren, d.h. $NAD^+$/NADH und $NADP^+$/NADPH, zu regenerieren.

"Proteinogene" Aminosäuren umfassen insbesondere (Einbuchstabencode): G, A, V, L, I, F, P, M, W, S, T, C, Y, N, Q, D, E, K, R und H.

Unter einer "Immobilisierung" versteht man die kovalente oder nicht-kovalente Bindung eines verwendeten Biokatalysators, wie z.B. einer 7β-HSDH an einem festen, d.h. in dem umgebenden flüssigen Medium im Wesentlichen unlöslichen, Trägermaterial. Es können entsprechend auch ganze Zellen, wie die erfindungsgemäß verwendeten, rekombinanten Mikroorganismen, mit Hilfe derartiger Träger immobilisiert sein.

Eine "im Vergleich zum nicht-mutierten Enzym verringerte Substratinhibition" bedeutet, dass das die beim nicht-mutierten Enzym für ein bestimmtes Substrat beobachtete Substratinhibition nicht mehr zu beobachten ist, d.h. im Wesentlichen nicht mehr messbar ist, oder erst bei höherer Substratkonzentration einsetzt, d.h. der $K_i$-Wert erhöht ist.

Unter einer "Cholsäure-Verbindung" versteht man hierin Verbindungen mit dem Kohenstoffgrundgerüst, insbesondere der Steroidstruktur der Cholsäure und dem Vorhandensein von Keto- und/oder Hydroxy- oder Acyloxy-Gruppen in der Ringposition 7 und gegebenenfalls den Ringpositionen 3 und/oder 12.

Unter einer Verbindung eines speziellen Typs, wie z.B. einer "Cholsäure-Verbindung" oder einer "Ursodesoxycholsäure-Verbindung" versteht man insbesondere auch Derivate der zugrunde liegenden Ausgangsverbindung (wie z.B. Cholsäure oder Ursodesoxycholsäure).

Derartige Derivate umfassen "Salze", wie z.B. Alkalimetallsalze wie Lithium-, Natrium- und Kaliumsalze der Verbindungen; sowie Ammoniumsalze, wobei man unter einem Ammoniumsalz das $NH_4^+$-Salz bzw. solche Ammoniumsalze umfasst, worin wenigstens ein Wasserstoffatom durch einen $C_1$-$C_6$-Alkylrest ersetzt sein kann. Typische Al-

kylreste sind insbesondere $C_1$-$C_4$-Alkylreste, wie Methyl, Ethyl, n- oder i-Propyl-, n-, sec- oder tert-Butyl, sowie n-Pentyl und n-Hexyl und die ein- oder mehrfach verzweigten Analoga davon

"Alkylester" Verbindungen sind insbesondere Niedrigalkyl-Ester, wie z.B. $C_1$-$C_6$-Alkylester. Als nicht limitierende Beispiele sind zu nennen Methyl-, Ethyl-, n- oder i-Propyl-, n-, sec- oder tert-Butylester, oder längerkettige Ester, wie z.B. n-Pentyl- und n-Hexylester sowie die ein- oder mehrfach verzweigten Analoga davon.

"Amide" sind insbesondere Umsetzungsprodukte von Säuren mit Ammoniak oder primären oder sekundären Monoaminen. Derartige Amine sind beispielsweise Mono- oder Di-$C_1$-$C_6$-Alkyl-monoamine, wobei die Alkylreste unabhängig voneinander gegebenenfalls weiter substituiert sein können, wie z.B. durch Carboxy-, Hydroxy-, Halogen (wie F, Cl, Br, J)-, Nitro- und Sulfonatgruppen.

"Acylgruppen" sind insbesondere nichtaromatische Gruppen mit 2 bis 4 Kohlenstoffatomen, wie z.B. Acetyl, Propionyl und Butyryl, sowie aromatische Gruppen mit gegebenenfalls substituiertem einkernigen aromatischem Ring, wobei geeignete Substituenten z.B. ausgewählt sind unter Hydroxy-, Halogen (wie F, Cl, Br, J)-, Nitro- und $C_1$-$C_6$-Alkylgruppen, wie z.B. Benzoyl oder Toluoyl.

Die erfindungsgemäß eingesetzten bzw. hergestellten Hydroxysteroidverbindungen, wie z.B. Cholsäure, Ursodesoxycholsäure, 12-Keto-Chenodesoxycholsäure, Chenodesoxycholsäure und 7-Keto-Lithocholsäure können in stereoisomerenreiner Reinform oder im Gemisch mit anderen Stereoisomeren im erfindungsgemäßen Verfahren eingesetzt oder daraus erhalten werden. Vorzugsweise werden jedoch die eingesetzten bzw. die hergestellten Verbindungen in im Wesentlichen stereoisomerenreiner Form eingesetzt bzw. isoliert.

Hierin verwendete Synonyme sind CDCS und CDCA; UDCS und UDCA; DHCS und DHCA; NAD und $NAD^+$; NADP und $NADP^+$.

"BTM" steht für Biotrockenmasse.

In folgender Tabelle 1 sind die Strukturformeln, deren chemischen Namen und die verwendeten Abkürzungen wesentlicher chemischer Verbindungen tabellarisch zusammengefasst:

**Tabelle 1**

| Formel | Abkürzung | Chemischer Name |
|---|---|---|
| 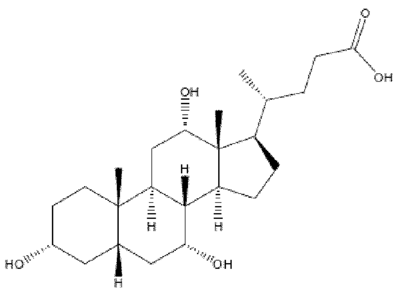 Cholsäure | CS | Cholsäure |
| 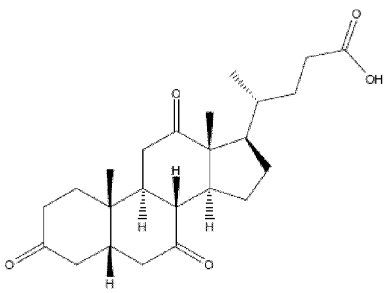 Dehydrocholsäure | DHCS | Dehydrocholsäure |

(fortgesetzt)

| Formel | Abkürzung | Chemischer Name |
|---|---|---|
| 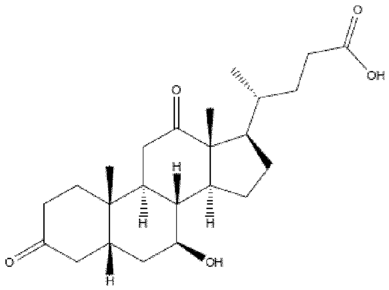 3,12-Diketo-7ß-Cholansäure | 3,12-Diketo-7β-CS | 3,12-Diketo-7β-Cholansäure |
| 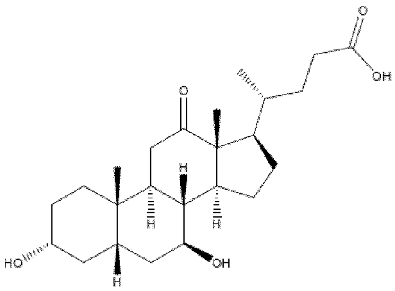 12keto-Ursodeoxycholsäure | 12Keto-UDCS | 12Keto-Ursodeoxycholsäure |
| 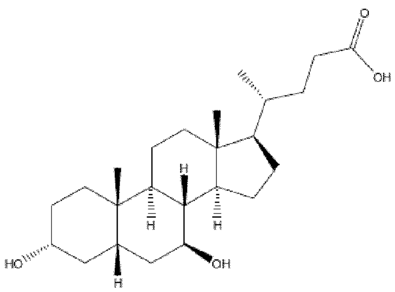 Ursodeoxycholsäure | UDCS | Ursodeoxycholsäure |
| 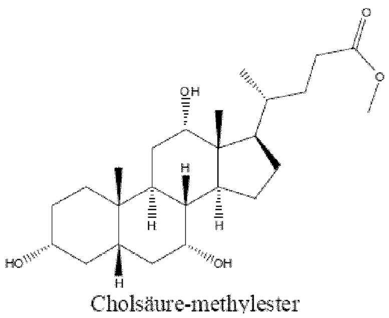 Cholsäure-methylester | CS-Methylester | Cholsäure-methylester |

(fortgesetzt)

| Formel | Abkürzung | Chemischer Name |
|---|---|---|
| 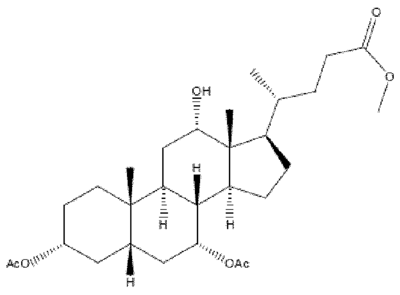<br>3,7-Diacetyl-Cholsäure-methylester | 3,7-Diacetyl-CS-methylester | 3,7-Diacetyl-Cholsäure-methylester* |
| 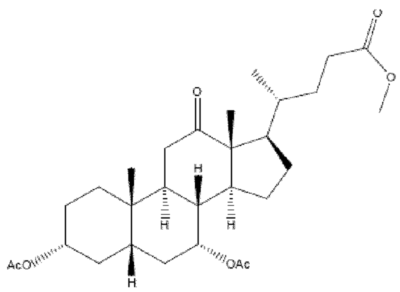<br>12-Keto-3,7-Diacetyl-Cholansäure-methylester | 12-Keto-3,7-Diacetyl-CS-methylester | 12-Keto-3,7-Diacetyl-Cholansäure-methylester* |
| 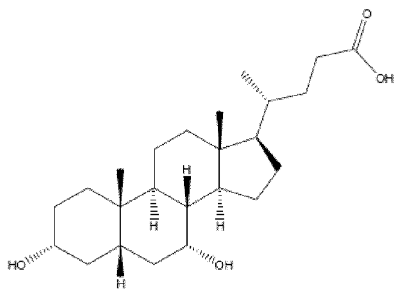<br>Chenodeoxycholsäure | CDCS | Chenodeoxycholsäure |
| 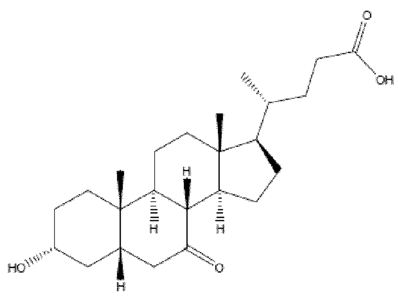<br>7-Keto-Lithocholsäure | 7-Keto-LCS | 7-Keto-Lithocholsäure |

(fortgesetzt)

| Formel | Abkürzung | Chemischer Name |
|---|---|---|
| 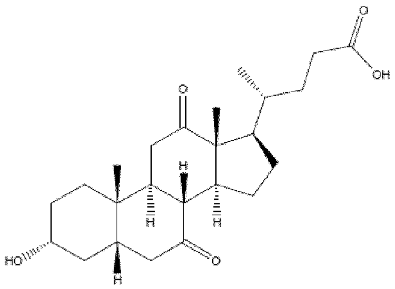 7,12-Diketo-Lithocholsäure | 7,12-Diketo-LCS | 7,12-Diketo-Lithocholsäure |
| 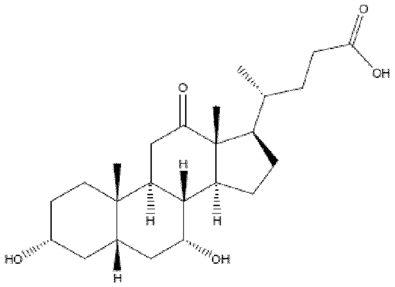 12-Keto-Chenodeoxycholsäure | 12-Keto-CDCS | 12-Keto-Chenodeoxycholsäure |

3,12-Diketo-7beta-Cholansäure ist ein Synonym für 3,12-Diketo-Ursodesoxycholsäure (3,12-Diketo-UDCS).
7,12-Diketo-Lithocholsäure ist ein Synonym für 7,12-Diketo-Ursodesoxycholsäure (7,12-Diketo-UDCS).

2. Proteine

Die hierin konkret offenbarten Proteine bzw. Enzyme insbesondere mit 7β-HSDH-, FDH-, GDH- oder 3α-HSDH Aktivität bzw. deren Mutanten schließen auch funktionale Äquivalente davon ein.

"Funktionale Äquivalente" oder Analoga der konkret offenbarten Enzyme sind davon verschiedene Polypeptide, welche weiterhin die gewünschte biologische Aktivität, wie z.B. 7β HSDH-Aktivität, besitzen.

So versteht man beispielsweise unter "funktionalen Äquivalenten" Enzyme, die im verwendeten Test auf 7β-HSDH-, FDH-, GDH- oder 3α-HSDH-Aktivität eine um mindestens 1%, wie z.B. mindestens 10% oder 20%, wie z.B. mindestens 50% oder 75% oder 90% höhere oder niedrigere Aktivität eines Ausgangs-Enzyms, umfassend eine hierin definierte Aminosäuresequenz aufweisen.

Funktionale Äquivalente sind außerdem vorzugsweise zwischen pH 4 bis 11 stabil und besitzen vorteilhaft ein pH-Optimum in einem Bereich von pH 6 bis 10, wie insbesondere 8,5 bis 9,5, sowie ein Temperaturoptimum im Bereich von 15°C bis 80°C oder 20°C bis 70°C, wie z.B. etwa 45 bis 60°C oder etwa 50 bis 55°C.

Die 7β-HSDH-Aktivität kann mit Hilfe verschiedener bekannter Tests nachgewiesen werden. Ohne darauf beschränkt zu sein, sei ein Test unter Verwendung eines Referenzsubstrates, wie z. B. CS oder DHCS, unter standardisierten Bedingungen wie im experimentellen Teil definiert, zu nennen.

Tests zur Bestimmung der FDH-, GDH- oder 3α-HSDH -Aktivität sind ebenfalls an sich bekannt.

Unter "funktionalen Äquivalenten" versteht man insbesondere auch "Mutanten", welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere, wie z.B. 1, 2, 3, 4, 5, 6, 7, 8, 9, 10, 11, 12, 13, 14 oder 15, Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden. Beispiele für geeignete Aminosäuresubstitutionen sind in folgender Tabelle 2 zusammengefasst:

Tabelle 2

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls mit umfasst.

"Funktionale Derivate" der hier offenbarten Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxylgruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der hier offenbarten Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

"Funktionale Äquivalente" sind außerdem Homologe zu den konkret offenbarten Proteinen. Diese besitzen wenigstens 60 %, vorzugsweise wenigstens 75% ins besondere wenigsten 85 %, wie z.B. 90, 91, 92, 93, 94, 95, 96, 97,98 oder 99%, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäßen homologen Polypeptids d.h. mit wenigstens 80% Sequenzidentität zu SEQ ID NO:2, bedeutet insbesondere prozentuale Identität der Aminosäurereste bezogen auf die Gesamtlänge einer

der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal Einstellungen ermittelt werden.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B. durch Punktmutation, Verlängerung oder Verkürzung des Proteins.

Homologe der erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Hierin offenbart ist weiterhin die Anwendung des 7β-HSDH-Wildtyps aus *Collinsella aerofaciens* ATCC 25986, wie er in der älteren internationalen Patentanmeldung WO2011/064404 (PCT/EP2010/068576) der Anmelderin beschrieben ist, worauf hiermit ausdrücklich Bezug genommen wird.

## 3. Nukleinsäuren und Konstrukte

### 3. 1 Nukleinsäuren

Gegenstand der Erfindung sind auch Nukleinsäuresequenzen, die für ein erfindungsgemäßes, mutiertes Enzym mit 7β-HSDH-Aktivität kodieren.

Die vorliegende Offenbarung nennt auch Nukleinsäuren mit einem bestimmten Identitätsgrad zu den hierin beschriebenen konkreten Sequenzen.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

| Multiple alignment parameters: | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |

Pairwise alignment parameter:

| | |
|---|---|
| FAST algorithm | on |
| K-tuplesize | 1 |
| Gap penalty | 3 |
| Window size | 5 |
| Number of best diagonals | 5 |

Alternativ dazu kann die Identität auch nach Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Internetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

Alle hierin erwähnten Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben. Gegenstand der Erfindung sind auch Nukleinsäuresequenzen (einzel- und doppelsträngige DNA- und RNA-Sequenzen, wie z.B. cDNA und mRNA), kodierend für eines der erfindungsgemäßen, mutierten 7β-HSDH Enzyme. Auch offenbart sind deren funktionalen Äquivalente, welche z.B. unter Verwendung künstlicher Nukleotidanaloga zugänglich sind.

Offenbart sind sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die offenbarten Nukleinsäuremoleküle können zudem untranslatierte Sequenzen vom 3'- und/oder 5'-Ende des kodierenden Genbereichs enthalten Weiterhin offenbart sind die zu den konkret beschriebenen Nukleotidsequenzen komplementären Nukleinsäuremoleküle oder einen Abschnitt davon.

Die offenbarten Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold

Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Hier offenbarte Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den hier offenbarten Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100% komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca. 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42°C über Nacht in einer Lösung bestehend aus 50 % Formamid, 5 x SSC (750 mM NaCl, 75 mM Tri-Natrium-citrat), 50 mM Natrium Phosphat (pH7,6), 5x Denhardt Lösung, 10% Dextransulfat und 20 g/ml denaturierte, gescheerte Lachsspermien-DNA, gefolgt von einem Waschschritt der Filter mit 0,1x SSC bei 65°C.

Hier offenbart sind auch Derivate der konkret offenbarten oder ableitbaren Nukleinsäuresequenzen.

So können weitere Nukleinsäuresequenzen z.B. von SEQ ID NO:1, 3 oder 7, abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Offenbart sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Offenbart sind ebenso durch konservative Nukleotidsubstitutionen (d.h. die betreffende Aminosäure wird durch eine Aminosäure gleicher Ladung, Größe, Polarität und/oder Löslichkeit ersetzt) erhältliche Sequenzen.

Offenbart sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 %

in der Nukleotidsequenz eines Gens.

Unter Derivaten der hier offenbarten Nukleinsäuresequenzen sind beispielsweise Allelvarianten zu verstehen, die mindestens 60 % Homologie auf der abgeleiteten Aminosäureebene, bevorzugt mindestens 80 % Homologie, ganz besonders bevorzugt mindestens 90 % Homologie über den gesamten Sequenzbereich aufweisen (bezüglich Homologie auf Aminosäureebene sei auf obige Ausführungen zu den Polypeptiden verwiesen auf). Über Teilbereiche der Sequenzen können die Homologien vorteilhaft höher liegen.

Weiterhin sind unter Derivaten auch Homologe der hier offenbarten Nukleinsäuresequenzen, beispielsweise pilzliche oder bakterielle Homologe, verkürzte Sequenzen, Einzelstrang-DNA oder RNA der kodierenden und nichtkodierenden DNA-Sequenz, zu verstehen. So besitzen z.B. Homologe auf DNA-Ebene eine Homologie von mindestens 40 %, bevorzugt von mindestens 60 %, besonders bevorzugt von mindestens 70 %, ganz besonders bevorzugt von mindestens 80 % über den gesamten angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch wenigstens einen Nukleotidaustausch, wenigstens eine Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Dem Fachmann sind darüber hinaus Verfahren zur Erzeugung funktionaler Mutanten bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der durch diese codierten Protein sind dem Fachmann seit langem geläufig, wie beispielsweise

- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcärel R, Stunnenberg HG (1994) Nucleic Acids Res 22:541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739);
- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique u-sing an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747).

Unter Anwendung der sogenannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise können stufenweise eine begrenzte Anzahl von Mutationen , wie z.B. 1 bis 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die hier offenbarten Ergebnisse liefern wichtige Informationen in bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

3.2 Konstrukte

Gegenstand der Erfindung sind außerdem Expressionskonstrukte, enthaltend unter der genetischen Kontrolle regulativer Nukleinsäuresequenzen eine für wenigstens ein erfindungsgemäßes Polypeptid kodierende Nukleinsäuresequenz; sowie Vektoren, umfassend wenigstens eines dieser Expressionskonstrukte.

Unter einer "Expressionseinheit" wird eine Nukleinsäure mit Expressionsaktivität verstanden, die einen Promotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird erfindungsgemäß eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im Kontext der Erfindung die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird erfindungsgemäß eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Hier offenbarte Nukleinsäurekonstrukte umfassen insbesondere Sequenz SEQ ID NO:1, 3 oder 7oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz

ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, lpp-, lac-, lpp-lac-, $lacI^{q-}$, T7-, T5-, T3-, gal-, trc-, ara-, rhaP ($rhaP_{BAD}$)SP6-, lambda-$P_R$- oder im lambda-$P_L$-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, $pIN\text{-}III^{113}$-B1, λgt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, $pGHlac^+$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

4. Mikroorganismen

Je nach Zusammenhang kann unter dem Begriff "Mikroorganismus" der Ausgangsmikroorganismus (Wildtyp) oder ein genetisch veränderter, rekombinanter Mikroorganismus oder beides verstanden werden.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der erfindungsgemäßen Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben. Einen Überblick über bakterielle Expressionssysteme für die heterologe Expression von Proteinen liefertz.B. auch Terpe, K. Appl. Microbiol. Biotechnol. (2006) 72: 211-222.

Als rekombinante Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gramnegative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden

Der Wirtsorganismus oder die Wirtsorganismen gemäß der Erfindung enthalten dabei mindestens eine der in dieser Erfindung beschriebenen Nukleinsäuresequenzen, Nukleinsäurekonstrukte oder Vektoren, die für ein erfindungsgemäßes, mutiertes Enzym mit 7β-HSDH-Aktivität gemäß obiger Definition kodieren.

Die im erfindungsgemäßen Verfahren verwendeten Mikroorganismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

Die erfindungsgemäßen Mikroorganismen können bis zu ihrer Verwendung in geeigneter Weise gelagert werden, z.B. in gefrorenem Zustand bei -20°C; oder aber auch als Lyophilisat. Zur Verwendung werden gefrorene Kulturen auf Raumtermperatur gebracht; ggf. können auch ein oder mehrere Gefrier- /Auftauzyklen durchgeführt werden. Lyophilisierte Präparate können zur weiteren Verwendung in einem geeigneten Flüssigmedium, wie Pufferlösungen, gelöst/suspendiert werden.

5. Herstellung der UDCS

*1. Schritt: Chemische Umsetzung von CS zu DHCS*

Die Hydroxygruppen von CS werden zum Beispiel mit Chromsäure bzw. Chromaten in saurer Lösung (z.B. $H_2SO_4$) zu Carbonylgruppe in an sich bekannter Weise auf klassischchemischem Weg oxidiert. Dadurch entsteht DHCS.

*2. Schritt: Enzymatische oder mikrobielle Umsetzung von DHCS zu 12-Keto-UDCS*

In wässriger Lösung wird DHCS durch 3α-HSDH und 7β-HSDH bzw. Mutanten davon spezifisch zu 12-Keto-UDCS in Anwesenheit von NADPH bzw. NADH reduziert. Der Kofaktor NADPH bzw. NADH kann durch eine ADH bzw. FDH bzw. GDH bzw. Mutanten davon von Isopropanol bzw. Natium-formiat bzw. Glucose regeneriert werden. Die Reaktion läuft unter milder Bedingung. Beispielsweise kann die Reaktion bei pH = 6 bis 9, insbesondere etwa pH = 8 und bei etwa 10 bis 30, 15 bis 25 oder etwa 23°C durchgeführt werden.

Im falle eines mikrobiellen Umsetzungsschrittes können rekombinante Mikroorganismen, welche die erforderliche(n) Enzymaktivität(en) exprimieren in Gegenwart des umzusetzenden Substrates (DHCS) anaerob oder aerob in geeigneten Flüssigmedien kultiviert werden. Geeignete Kultivierungsbedingungen sind dem Fachmann an sich bekannt. Sie umfassen Umsetzungen im pH bereich von beispielsweise 5 bis 10 oder 6 bis 9, bei Temperaturen im Bereich von 10 bis 60 oder 15 bis 45 oder 25 bis 40 oder 37 °C. Geeignete Medien umfassen z,B, die unten beschriebenen LB und TB Medien. Die Umsetzungsdauer kann dabei z,B, batchweise oder kontinuierlich oder in sonstigen üblichen Verfahrensvarianten erfolgen (wie oben beschrieben). Die Unsetzungsdauer kann dabei z.B. im Bereich von Minuten bis mehreren Stunden oder tagen Liegen, und z.B. 1h bis 48 h betragen. Gegebenenfalls kann, wenn Enzymaktivität nicht kontinuierlich exprimiert wird, diese durch Zugabe eines geeigneten Induktors, nach Erreichen einer Zielzelldichte , z,B, von etwa $OD_{600}$ = 0,5 bis 1,0, eingeleitet werden.

Weitere mögliche geeignete Abwandlungen des mikrobiellen Herstellungsverfahrens hinsichtlich Fahrweise der Fermentation, Zusätze zum Medium, Enzymimmobilisierung und Isolierung der Wertstoffe sind auch dem folgenden Abschnitt betreffend "Herstellung der Enzyme bzw. Mutanten" zu entnehmen

*3. Schritt Chemische Umsetzung von 12-Keto-UDCS zu UDCS*

Die 12-Carbonylgruppe von 12-Keto-UDCS wird mittels Wolff-Kishner-Reduktion in an sich bekannter Weise

entfernt, dadurch entsteht UDCS aus 12-Keto-UDCS. In der Reaktion wird zuerst die Carbonylgruppe mit Hydrazin zum Hydrazon umgesetzt. Anschließend wird das Hydrazon in Gegenwart einer Base (z.B. KOH) auf 200 °C erhitzt, hierbei wird Stickstoff abgespaltet und UDCS entsteht.

6. Rekombinante Herstellung der Enzyme und Mutanten

Offenbart sind weiterhin Verfahren zur rekombinanten Herstellung erfindungsgemäßer Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die offenbarungsgemäß hergestellten Mikroorganismen können kontinuierlich oder diskontinuierlich im batch-Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese offenbarungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die offenbarungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich

von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff-haltige Gasmischungen, wie z. B. Umgebungsluft, in die Kultur eingetragen. Die Temperatur der Kultur liegt normalerweise bei 20°C bis 45°C und. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

7. Enzymimmobilisierung

Die erfindungsgemäßen Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als GanzzellKatalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" " in K. Drauz und H. Waldmann, Enzyme Catalysis in Organic Synthesis 2002, Vol.III, 991-1032, Wiley-VCH, Weinheim beschrieben.

## Experimenteller Teil:

### A. Allgemeine Angaben

#### 1. Materialien:

Die genomische DNA von *Collinsella aerofaciens* DSM 3979 (ATCC 25986, frühere Bezeichnung *Eubacterium aerofaciens*) wurde von der Deutschen Sammlung für Mikroorganismen und Zellkulturen (DSMZ) bezogen. UDCS und 7-Keto-LCS sind an sich bekannte und in der Literatur beschriebene Ausgangsverbindungen. Alle übrigen Chemikalien und Enzyme ware kommerziell erhältliche Handelsprodukte verschiedener Hersteller.

#### 2. Mikroorganismen und Vektoren:

**2.1 Mikroorganismen**

| | |
|---|---|
| E. coli BL21(DE3) | $F^-$ ompT gal dcm Ion $hsdS_B(r_B^- m_B^-)$ _(DE3 [lacI lacUV5-T7 gene 1 ind1 sam7 nin5]) |
| E. coli BL49 | $F^-$ ompT gal dcm Ion $hsdS_B(r_B^- m_B^-)$ _(DE3 [lacI lacUV5-T7 gene 1 ind1 sam7 nin5]) hdhA::KanR |
| E. coli BLLiu (=E. *coli* BL21ΔhdhA) | $F^-$ ompT gal dcm Ion $hsdS_B(r_B^- m_B^-)$ _(DE3 [lacI lacUV5-T7 gene 1 ind1 sam7 nin5]) _hdhA |
| E. coli NovaBlue(DE3) | endA1 hsdR17($r_{K12}^-$ $m_{K12}^+$) supE44 $thi^{-1}$ recA1 gyrA96 relA1lac [F' proA+B+ lacIqZ M15::Tn10($Tc^R$)] |
| E. coli NB13 | endA1 hsdR17($r_{K12}^-$ $m_{K12}^+$) supE44 $thi^{-1}$ recA1 gyrA96 relA1lac [F' $proA^+B^+$ laclqZ M15::Tn10($Tc^R$)] hdhA::KanR |

**2.2 Expressionsvektoren und Vektorkonstrukte**

Die Expressionsplasmide (vgl. Fig. 3)

p7(A)T3rG (= p7(A)T3rG-A) (vgl. WO2012/080504)
p7(A)T3rG-K und
p7(A)T3TG (=p7(A)T3TG-A)

verfügen jeweils über Expressionskassetten, in denen die Gene 7β-HSDH, 3α-HSDH und GDH codiert sind, allerdings mit unterschiedlicher Struktur der Expressionskassette und mit unterschiedlichen Antibiotikaresistenzen. Diese Plasmide wurden wahlweise in den Wirtsstamm *E. coli* BL49 oder *E. coli* BL21 ΔhdhA (beide aus der WO 2012/080504 bzw. der WO 2011/147957 der Anmelderin bekannt) eingesetzt.

Folgende so modifizierte Stämme wurden verwendet:

*E. coli* BL49 p7(A)T3rG,
*E. coli* BL21 ΔhdhA p7(A)T3rG-K
*E. coli* BL49 p7(A)T3TG

#### 3. Mikrobiologische Methoden

Molekularbiologische Arbeiten erfolgen, soweit keine anderen Angaben gemacht werden, in Anlehnung an etablierte Methoden, z.B. beschrieben in: Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989; Ausubel et al. (eds.), Current Protocols in Molecular Biology, John Wiley &Sons, NY (1993); Kriegler, Gene Transfer and Expression, A Laboratory Manual, Stockton Press, NY (1990).

**3.1 Kultivierung von Escherichia coli im Schüttelkolben**

Für die Expression rekombinanter Proteine wurden zunächst 5 mL LB-Medium mit Zugabe des entsprechenden

Antibiotikums mit einer Bakterienkolonie oder einer Kryokultur inokuliert und anschließend über Nacht bei 30 °C und 200 rpm inkubiert. Am nächsten Tag wurden 100-200 mL TB-Medium mit entsprechendem Antibiotikum mit 1-5 mL der Übernachtkultur inokuliert und bei 37 °C, 250 rpm inkubiert. Bei Erreichen einer OD600 von 0,6-0,8 wurde die Expression des rekombinanten Proteins durch Zugabe von 1 mM IPTG induziert und die Kultur bei 25 °C, 160 rpm weitere 21 h inkubiert.

**3.2 Kultivierung von Escherichia coli im 7,5 L Rührkesselreaktor**

Die Kultivierung von Ganzzellbiokatalysatoren im Litermaßstab erfolgte in einem Rührkesselreaktor (V = 7,5 L) der Firma Infors AG (Infors 3, Bottmingen, Schweiz). Der Reaktor war mit Sonden für Temperatur, pH und pO2 ausgestattet, so dass diese Parameter von einer Steuerungseinheit online abgelesen und gegebenenfalls geregelt werden konnten. Temperiert wurde der Reaktor über einen an die Steuerungseinheit angeschlossenen Doppelmantel. Die Begasung erfolgte über ein Tauchrohr und die Durchmischung erfolgte durch drei Sechsblatt-Scheibenrührer, die über einen Motor am Deckel des Reaktors angetrieben wurden. Zudem konnte über Zulaufpumpen Substrat und Base (Ammoniumhydroxidlösung, 25 % (w/v)) in den Reaktor zugegeben werden.

**Vorkultur**

Für die Kultivierung im 7,5 L Rührkesselreaktor wurden insgesamt zwei Vorkulturstufen angesetzt. Die erste Vorkultur erfolgte in einem Reagenzglas mit 5 mL LB-Medium mit entsprechendem Antibiotikum. Diese wurde morgens mit 100 µL einer Kryokultur angeimpft und 6-10 hbei 30 °C und 200 rpm inkubiert, bis eine sichtbare Trübung auftritt. Anschließend wurden 500-1000 µL der ersten Vorkultur in einen mit 200 mL Minimalmedium nach Wilms et al. (2001) Material und gefüllten 1 L-Enghals-Erlenmeyerkolben überführt, welcher über Nacht bei 37 °C, 250 rpm (Exzentrizität 5 cm) inkubiert wurde.

**Satzphase**

Für die Rührkesselkultivierung wurde ein mit 2,8-3,8 L Minimalmedium und 1,5 mL Antischaummittel (Antifoam 204, Sigma-Aldrich, München) befüllter, sterilisierter Rührkesselreaktor verwendet, dessen Sonden vor Beginn der Kultivierung nach gängigem Verfahren kalibriert wurden. Dieser wurde mit Satzglucose (Endkonzentration 2 g $L^{-1}$) und entsprechendem Antibiotikum versetzt und anschließend mit 200 mL der zweiten Vorkulturstufe inokuliert. Begast wurde zu Beginn mit 2 L $min^{-1}$ Druckluft und die Rührerdrehzahl betrug am Anfang der Kultivierung 200 rpm. Bei Unterschreiten eines Schwellenwerts von 30 % pO2 wurde die Rührerdrehzahl inkrementell um 5 rpm bis zu einem theoretischen Maximalwert von 1100 rpm erhöht. Der pH wurde einseitig durch Basenzugabe (25 % Ammoniumhydroxid, w/v) auf 7,0 geregelt, während die Temperatur auf 37 °C gehalten wurde. Nach Verbrauch der Satzglucose, was durch plötzlichen Anstieg des $pO_2$ festgestellt werden konnte, erfolgte der Übergang zur substratlimitierten Zulaufphase.

**Substratlimitierte Wachstumsphase**

Zu Beginn der Zulaufphase wurde die Begasung mit Druckluft auf 5 L $min^{-1}$ erhöht, die Temperatur auf 30 °C verringert und der Schwellenwert für die inkrementelle Rührerdrehzahlerhöhung auf 20 % pO2 verringert. Die Zudosierung des Substratzulaufs erfolgte basierend auf einer festgelegten Wachstumsrate von $\mu$ = 0,15 $h^{-1}$. Das Zulaufmedium enthielt 500 g $L^{-1}$ Glucose und 99 g $L^{-1}$ Diammoniumhydrogenphosphat. Der Biomassenertrag wurde mit 0,45 $g_{BTM}$ $g_{Glc}^{-1}$ angenommen.

Die Dauer der substratlimitierten Wachstumsphase betrug 19 h. Eine Stunde vor Ende dieser Phase wurde die Temperatur auf 20 °C verringert, weiterhin wurden 3 mL $L^{-1}$ Spurenelementlösung und 2 mL $L^{-1}$ Magnesiumsulfatlösung (1 M) zugegeben, jeweils bezogen auf V0. Wurde Ampicillin als Selektionsantibiotikum verwendet, wurden zudem jeweils zu Beginn und 1 h vor Ende der substratlimiterten Wachstumsphase 50 mg $L^{-1}$ Ampicillin zugegeben.

**Expressionsphase**

Nach Beendigung der substratlimiterten Wachstumsphase erfolgte zu Beginn der Expressionsphase die Zugabe von 0,5 mM IPTG (bezogen auf V0). Die Temperatur wurde auf 20 °C gehalten und die eingestellte Wachstumsrate auf $\mu$ = 0,06 $h^{-1}$ verringert. Nach 18 h wurde der Zulaufvolumenstrom auf den zuletzt eingestellten Wert konstant gehalten, da ansonsten eine Sauerstoffsättigung von pO2 $\geq$ 20 % nicht gewährleistet werden konnte. Eine weitere Zugabe von 3 mL $L^{-1}$ Spurenelementlösung, 2 mL $L^{-1}$ Magnesiumsulfatlösung (1 M) und gegebenenfalls 50 mg $L^{-1}$ Ampicillin erfolgte 8 h nach Beginn der Expressionsphase. Die Zellen wurden 24 h nach Beginn der Expressionsphase geerntet, gegebenenfalls mit 30 % Glycerin (v/v) versetzt und bei -20 °C gelagert.

### 3.3 Kultivierung von Escherichia coli-Bibliotheken in Deep Well-Platten

Für die Kultivierung von E. coli-Bibliotheken in Deep Well-Platten wurde zunächst eine Vorkultur in sterilen Mikrotiterplatten mit 96 Vertiefungen angesetzt. Hierzu werden jeweils 150 μL Vorkulturmedium (TB-Medium versetzt mit 5 % (v/v) DMSO) in die Vertiefungen gegeben. Anschließend wurden die Vertiefungen mit Kolonien von Agarplatten mit Hilfe von sterilen Zahnstochern inokuliert. Abschließend wurden die Mikrotiterplatten mit sterilen, atmungsaktiven Verschlussfolien (Breathe-Easy, Diversified Biotech, USA) versiegelt und bei 37 °C über Nacht bei 200-250 rpm inkubiert. Nach der Inkubation wurden die Mikrotiterplatten als Stammplatten steril bei -80 °C gelagert. Die Proteinexpression erfolgte in sterilen Deep Well-Platten mit 96 Vertiefungen bei 2,2 mL Vertiefungsvolumen und quadratischen Vertiefungsöffnungen. Hierzu wurden jeweils 600 μL steriles TB-Medium mit entsprechendem Antibiotikum in die Vertiefungen überfuhrt, anschließend wurden die Vertiefungen mit je 10 μL Vorkultur aus der Stammplatte inokuliert. Die Deep Well-Platten wurden mit sterilen, atmungsaktiven Verschlussfolien versiegelt und bei 37 °C für 9 h bei 200-250 rpm inkubiert. Nach 9 h Inkubation wurden die Kulturen mit jeweils 100 μL Induktionslösung versetzt. Anschließend wurden die DepWell-Platten mit sterilen, atmungsaktiven Verschlussfolien versiegelt und bei 30 °C für weitere 21 h bei 200-250 rpm inkubiert. Die Zellernte erfolgte durch Zentrifugation (30 min, 3000 g). Die Zellpellets wurden bis zur weiteren Verwendung bei -80 °C gelagert.

### 3.4 Stammhaltung

Die kurz- und mittelfristige Stammhaltung von E. coli erfolgte auf LB-Agarplatten mit entsprechenden Selektionsantibiotika bei 4 °C. Für eine langfristige Stammhaltung wurden Kryokulturen hergestellt, indem E. coli-Kulturen in LB-Medium angezogen wurden und in der exponenziellen Wachstumsphase (OD ≤ 0,8) mit 20 % sterilem Glycerin (v/v) versetzt und in sterilen 1,5 mL-Reaktionsgefäßen bei -80 °C gelagert wurden.

### 3.5 Zellaufschluss von Escherichia coli in der Schwingmühle

Der Zellaufschluss von E. coli in der Schwingmühle erfolgte in 2 mL-Reaktionsgefäßen. Hierzu wurden jeweils 1 mL Glasperlen (Durchmesser 0,25-0,5 mm, Carl Roth, Karlsruhe) und 1 mL der aufzuschließenden Bakteriensuspension in das Reaktionsgefäß gegeben, welches anschließend in eine Schwingmühle (MM 200, Retsch, Haan) eingespannt und 6 min bei 30 Hz geschüttelt wurde. Anschließend wurden die Gefäße 10 min bei 4 °C und 17880 g in einer Tischzentrifuge (Biofuge Stratos, Thermo Fischer Scientific,Waltham, USA) zentrifugiert. Der Überstand konnte für weitere Anwendungen verwendet werden.

### 3.6 Zellaufschluss von Escherichia coli im Hochdruckhomogenisator

Zellen aus Kultivierungen im Bioreaktor wurden mit einem Hochdruckhomogenisator (Ariete, GEA Niro Soavi, Lübeck) aufgeschlossen. Hierzu wurde die Zellsuspension zunächst in einen mit 50 L Kaliumphosphatpuffer (20 mM, pH 7,4) befüllten und auf 4 °C gekühlten 200 L-Edelstahltank überführt. Der Aufschluss erfolgte bei einem Druck von 950 bar und einem Volumenstrom von 300-350 L $h^{-1}$, welcher nach 15-20 min auf 150 L $h^{-1}$ gedrosselt wurde. Die Zellbrühe wurde nach der ersten Passage in einem zweiten, auf 4 °C gekühlten 200 L-Edelstahltank gesammelt, auf unter 20 °C gekühlt und in einen dritten, mit 50 L Kaliumphosphatpuffer befüllten und auf 4 °C gekühlten 200 L-Edelstahltank überführt. Anschließend wurde eine zweite Passage durch den Hochdruckhomogenisators nach obiger Beschreibung durchgeführt.

Diese Vorgehensweise sollte gewährleisten, dass die Temperatur der Zellbrühe während des Aufschlusses 35 °C nicht übersteigt, da eine Passage durch den Hochdruckhomogenisator zu einer Erwärmung des Mediums um 10-15 °C führte. Im Anschluss an den Zellaufschluss wurde in einem ersten Schritt Zelldebris mit einem Tellerseparator (CSA 08, GEA Westfalia, Oelde) abgetrennt. Dieser wurde bei einem Volumenstrom von 100 L $h^{-1}$, einem Gegendruck von 0-3 bar und einem Teilentleerungsintervall des Feststoffauswurfes von 999 s betrieben. Die geklärte Zellbrühe wurde in 10 L-Plastikkanister aliquotiert und bei -20 °C bis zur weiteren Verwendung gelagert.

### 3.7 Zellaufschluss von Escherichia coli mit Lysozym

Für den enzymatischen Zellaufschluss mit Lysozym wurden in Deep Well-Platten pelletierte Zellen in 600 μL Aufschlusspuffer (50 mM KPi, 10 mM $MgCl_2$, 70000 U $mL^{-1}$ Lysozym, 50 U $mL^{-1}$ DNAsel) resuspendiert und für 1 h bei 37 °C inkubiert. Anschließend wurde die Zelldebris durch Zentrifugation (30 min, 3000 g, 4 °C) in einer Standzentrifuge (Rotixa 50 RS, Hettich, Tuttlingen) abgetrennt. Der Überstand wurde für weitere Untersuchungen verwendet.

## 4. Molekularbiologische Methoden

### 4.1 Isolation von Plasmid-DNA

Die Isolation von Plasmid-DNA wurde mit dem GenElute™ Plasmid Miniprep Kit (Sigma-Aldrich, München) durchgeführt. Hierzu wurden 5 mL einer LB-Übernachtkultur von E. coli nach Herstellerangaben behandelt. Zur Elution der isolierten Plasmid-DNA wurden 100 μL auf 70 °C temperiertes, steriles, doppelt destilliertes Wasser verwendet.

### 4.2 Polymerasekettenreaktion

Polymerasekettenreaktionen (PCR) zur präparativen Amplifikation von DNA-Fragmenten wurden nach der Methode von Saiki R. K. et al. Science, 239:487-491, 1988, durchgeführt. Der Reaktionsansatz bestand aus1-2,5 μL Templat-DNA, je 0,5 μM Oligonukleotid, je 0,2 mM der Desoxyribonukleotid-Triphosphate (dNTPs) sowie 0,02 U $\mu L^{-1}$ Phusion DNA-Polymerase. Das Temperaturprogramm für die DNA-Amplifikation richtete sich nach den Angaben des Polymerasenherstellers und den Schmelztemperaturen der Oligonukleotide.

### 4.3 Analytische und präparative Agarose-Gelelektrophorese

Für die Trennung von DNA-Molekülen wurden Agarosegele mit einer Konzentration von 1 %Agarose (w/v) verwendet. Dabei wurde 1 g Agarose in 100 mL 1x TAE-Puffer durch Aufkochen gelöst, mit 5 μL Ethidiumbromid (≥98 %) oder alternativ 5 μL Roti®-GelStain (Carl Roth, Karlsruhe) versetzt und anschließend in eine Gelkammer (C.B.S. Scientific, San Diego, USA) gegossen. Die aufzutrennende DNA wurde vor dem Auftragen mit 5x-Agarose-Gelladepuffer nach Sambrook & Russell (2001) versetzt und auf das Gel aufgetragen. Als Längenstandard diente eine 100 bp DNA-Leiter extended (Carl Roth, Karlsruhe). Die Elektrophorese erfolgte in 1x TAE-Puffer bei einer konstanten Spannung von 120 V.

### 4.4 Aufreinigung von DNA-Fragmenten mittels Gelextraktion

Für die Aufreinigung von DNA-Fragmenten aus Agarosegelen wurde der GenElute™ Gel Extraction Kit (Sigma-Aldrich, München) verwendet. Hierzu wurde nach Herstellerangaben verfahren. Zur Elution der isolierten DNA-Fragmente wurden 50 μL auf 70 °C temperiertes, steriles, doppelt destilliertes Wasser verwendet.

### 4.5 Aufreinigung von DNA-Fragmenten mittels PCR Clean-Up Kit

Die Aufreinigung von DNA-Fragmenten mit dem GenElute™ PCR Clean-Up Kit (Sigma-Aldrich, München) erfolgte nach Herstellerangaben. Zur Elution der aufgereinigten DNA-Fragmente wurden 50 μL auf 70 °C temperiertes, steriles, doppelt destilliertes Wasser verwendet.

### 4.6 Restriktion mit Endonukleasen

Für die Restriktion von DNA wurden 40-45 μL der zu schneidenden DNA mit 10-20 U der jeweiligen Restriktionsenzyme versetzt und in dem vom Hersteller empfohlenen Reaktionspuffer mit entsprechenden Zusätzen für 2 h bei 37 °C inkubiert. Anschließend wurden die Fragmente entweder durch Agarosegelelektrophorese mit anschließender Gelextraktion oder unter Verwendung des PCR Clean-Up Kits aufgereinigt.

### 4.7 Ligation von DNA-Fragmenten

Für die Ligation von DNA-Fragmenten wurden zuvor bereits restringierte und aufgereinigte DNA-Fragmente verwendet. Die Ligation erfolgte unter Verwendung von 12 μL geschnittener Vektor-DNA, 4 μL geschnittener Insert-DNA, 20 U $mL^{-1}$ T4-DNA-Ligase (New England Biolabs, Frankfurt) unter Zusatz von 0,5 mM ATP in dem vom Hersteller dafür vorgesehenen Puffer bei 16 °C. Alternativ wurde die Ligation mit dem Quick Ligation™ Kit (New England Biolabs, Frankfurt) nach Herstellerangaben durchgeführt. Dabei wurden 6 μL geschnittene Vektor-DANN und 3 μL geschnittene Insert-DNA verwendet.

### 4.8 Positionsgerichtete Mutagenese

Positionsgerichtete Mutagenesen an Plasmid-DNA wurden wahlweise mit einer Methodenach Sanchis et al., Appl. Microbiol. Biotechnol., 81(2):387-97, 2008 oder Liu, H. & Naismith, J. H., BMC Biotechnol., 8:91, 2008 durchgeführt.

Sollte eine Sättigungsmutagenese durchgeführt werden, wurden Primer mit degenerierten Codons verwendet. Im Falle der Methode nach Sanchis et al., Appl. Microbiol. Biotechnol., 81(2):387-97, 2008 bestand der Reaktionsansatz aus 0,4 µL Templat-DNA, je 0,1 µM Oligonukleotid, je 0,2 mM der dNTPs sowie 0,02 U µL-1 Phusion Hot Start DNA-Polymerase. Das Temperaturprogramm für die DNA-Amplifikation richtete sich nach der publizierten Methode und wurde lediglich entsprechend den Angaben des Polymerasenherstellers und den Schmelztemperaturen der Oligonukleotide angepasst.

Bei der Methode nach Liu & Naismith (2008) bestand der Reaktionsansatz aus 0,2 µL Templat-DNA, je 1 µM Oligonukleotid, je 0,2 mM der dNTPs sowie 0,02 U $\mu L^{-1}$ Phusion Hot Start DNA-Polymerase. Das Temperaturprogramm für die DNA-Amplifikation richtete sich nach der publizierten Methode und wurde lediglich entsprechend den Angaben des Polymerasenherstellers und den Schmelztemperaturen der Oligonukleotide angepasst. Im Anschluss an die Mutagenese-PCR wurde die parentale DNA restringiert, indem zweimal hintereinander jeweils 0,5 U $\mu L^{-1}$ DpnI zugegeben und im Anschluss 1 h bei 37 °C inkubiert wurde.

### 4.9 Herstellung und Transformation chemisch kompetenter Zellen

Für die Herstellung von chemisch kompetenten E. coli-Zellen wurde eine 100 mL-LB-Flüssigkultur im exponentiellen Wachstumsstadium (OD 0,5) in 50 mL-Reaktionsgefäße überführt und durch Zentrifugation (3220 g, 4 °C, 10 min) pelletiert. Anschließend wurde der Überstand verworfen, das Zellpellet in 40 mL eisgekühltem TFB1-Medium resuspendiert und 15 min auf Eis inkubiert. Danach wurden die Zellen erneut durch Zentrifugation (3220 g, 4 °C,10 min) pelletiert, der Überstand verworfen, die Zellen mit 4 mL eisgekühltem TFB2-Medium resuspendiert und weitere 15 min auf Eis inkubiert. Im Anschluss wurden 200 µL-Aliquots in sterile 1,5 mL-Reaktionsgefäße gegeben und bei -80 °C eingefroren. Für die Transformation wurde jeweils ein Aliquot aufgetaut, mit 1-10 µL DNA-Lösung versetzt und 45 min auf Eis inkubiert. Nach einem Hitzeschock (42 °C, 1:30 min) im Thermomixer (RiO, QUANTIFOIL Instruments, Jena) wurden die Zellen erneut 1-2 min auf Eis gehalten. Anschließend wurden 600 µL steriles LB-Medium zugegeben und im Thermomixer weitere 45 min bei 37 °C und 600 rpm inkubiert. Nach einer milden Zentrifugation (3000 rpm, 1 min) wurde der Üerstand bis auf 50-100 µL verworfen, das Pellet im verbliebenen Überstand resuspendiert und auf entsprechende Agar-Platten ausplattiert. Diese wurden anschließend im Brutschrank bei 37 °C über Nacht inkubiert.

### 4.10 Herstellung und Transformation elektrokompetenter Zellen

Für die Herstellung von elektrokompetenten E. coli-Zellen wurde eine 200 mL-LB Flüssigkultur im exponentiellen Wachstumsstadium (OD 0,5) in eisgekühlte 50 mL-Reaktionsgefäße überführt, 20 min auf Eis inkubiert und durch Zentrifugation (4000 g, 4 °C, 15 min) pelletiert. Die Zellen wurden anschließend dreifach gewaschen, indem das Pellet nachfolgend in 200 mL, 100 mL und 8 mL eisgekühlter 10 %-iger Glycerinlösung (v/v) resuspendiert und erneut durch Zentrifugation (6000 g, 4 °C, 15 min) pelletiert wurden. Im Anschluss wurden die Zellen auf ein Gesamtvolumen von 0,4-0,8 mL mit eisgekühltem 10 %-igem (v/v) Glycerin resuspendiert und in Aliquots a' 20 µL in eisgekühlten, sterilen 1,5 mL-Reaktionsgefäßen abgefüllt und bei -80 °C eingefroren.

Für die Transformation wurden die Zellen mit 2-10 µL entsalzter DNA-Lösung versetzt und in Elektroporationsküvetten mit 1-2 mm Elektrodenabstand nach Herstellerprotokoll des Elektroporators (Gene Pulser Xcell™, Bio-Rad, M"unchen.) elektroporiert. Anschließend wurden sofort 1 mL LB-Medium zu den Zellen gegeben, die Suspension in sterile 1,5 mL-Reaktionsgefäße überführt und 60 min bei 37 °C und 600 rpm im Thermomixer (RiO, QUANTIFOIL Instruments, Jena) inkubiert. Nach einer milden Zentrifugation (3000 rpm, 1 min) wurde der Überstand bis auf 50-100 µL verworfen, das Pellet im verbliebenen Überstand resuspendiert und auf entsprechende Agar-Platten ausplattiert. Diese wurden anschließend im Brutschrank bei 37 °C über Nacht inkubiert.

### 4.11 Kolonie-Polymerasekettenreaktion

Präparative Kolonie-Polymerasekettenreaktionen (Kolonie-PCR) wurden durchgeführt, um das Gen für die Glucosedehydrogenase aus *Bacillus subtilis* zu isolieren. Die Vorgehensweise entspricht der in Abschnitt 4.2 angegebenen, mit der Abwandlung, dass anstelle von Templat-DNA ein Bakterienabstrich von einer auf Agarplatten kultivierten Einzelkolonie zum Reaktionsansatz zugegeben wurde. Analytische Kolonie-PCR dienten zur Kontrolle einer korrekt erfolgten Ligation. Auch hierbei diente ein Abstrich einer einzelnen Bakterienkolonie als Templat. Primer für die Kolonie-PCR wurden so gewählt, dass diese an der Ziel-DNA mit zur Insertionsstelle flankierenden Regionen hybridisieren, so dass anhand der Länge des Amplifikats abgeschätzt werden kann, ob die Insertion erfolgreich war. Der Reaktionsansatz bestand aus je 0,5 µM Oligonukleotid, je 0,2 mM der dNTPs sowie 0,05 U $\mu L^{-1}$ Taq DNA-Polymerase. Das Temperaturprogramm für die DNA-Amplifikation richtete sich nach den Angaben des Polymerasenherstellers und den Schmelztemperaturen der Oligonukleotide.

### 4.12 Spezifische Ausschaltung chromosomaler Gene

Die spezifische Ausschaltung der chromosomalen 7α-HSDH in E. coli erfolgte mit Hilfe des Kits TargeTron™ Gene Knockout System von Sigma Aldrich (München). Das für die Ausschaltung benötigte Plasmid pMB13 ist in Braun, M., PhD thesis, Technische Universität München, 2011 beschrieben. Dieses Plasmid wurde in chemisch kompetente E. coli transformiert und das Ausschalten des Zielgens nach Herstellerangaben durchgeführt. Durch Selektion auf LB-Agarplatten mit Kanamycin sowie Kolonie-PCR konnte das erfolgreiche Ausschalten nachgewiesen werden. Um das im Bakterium verbliebene Plasmid zu entfernen, wurde eine Über-Nacht-Kultur der Zellen in LB-Medium mit Kanamycin (50 mg $L^{-1}$) und Novobiocin (62 mg $L^{-1}$) bei 37 °C angesetzt. Anschließend wurde die Kultur auf LB-Agarplatten mit Kanamycin ausgestrichen und über Nacht bei 37 °C inkubiert. Das Vorhandensein des zu entfernenden Plasmids wurde untersucht, indem einzelne Kolonien auf Chloramphenicol-Sensitivität untersucht werden. Dies erfolgte in paralleler Über-Nacht-Kultivierung bei 37 °C in LB-Medium, einmal unter Zugabe von 33 mg $L^{-1}$ Chloramphenicol, einmal ohne dessen Zugabe.

## 5. Proteinchemische Methoden

### 5.1 Aufreinigung von Proteinen im mL-Maßstab

Die Aufreinigung der Proteine im mL-Maßstab erfolgte nach dem Prinzip der Immobilisierten Metallaffinitätschromatographie (IMAC). Das Trennprinzip beruht auf der spezifischen Wechselwirkung matrixgebundener Metallliganden mit Histidinresten am aufzureinigenden Zielprotein. Zu diesem Zweck wird bei der Expression des Zielproteins ein His6-Anker entweder N- oder C-terminal fusioniert. Für die Aufreinigung an der Fast Protein Liquid Chromatography (FPLC)-Anlage wurden HisTrap-Säulen (1 mL oder 5 mL Säulenvolumen) verwendet, deren Agarosematrix mit $Ni^{2+}$ -Ionen beladen war. Die Flussraten der mobilen Phase wurden jeweils auf ein Säulenvolumen (CV) pro Minute eingestellt. Die Proteinkonzentration im Eluatstrom konnte hierbei über die UV-Extinktion bei 280 nm verfolgt werden. Zunächst wurden die Säulen mit mindestens 5 CV Bindepuffer äquilibriert, anschließend erfolgte die Auftragung der Proben. Danach erfolgte das Waschen der Säule mit Bindepuffer zur Entfernung nicht spezifisch bindender Fremdproteine. Das Waschen wurde so lange durchgeführt, bis wieder eine Basislinie des UV-Signals erreicht wurde. Die Elution des Zielproteins wurde durch einen von 0 % auf 100 % über 20 min linear ansteigenden Elutionspuffergradienten erreicht, wobei Eluatfraktionen von jeweils 2 CV gesammelt wurden. Die Fraktionen, die das Zielprotein enthalten, können über das UV-Signal identifiziert werden. Anschließend wurde die Säule nochmal mit 10 CV Elutionspuffer nach gewaschen. Die das Zielprotein enthaltenden Fraktionen wurden anschließend über Vivaspin Zentrifugalkonzentratoren (Ausschlussgröße 10 kDa) aufkonzentriert und durch dreimaliges Auffüllen und Aufkonzentrieren mit dem Zielpuffer umgepuffert. Der Zielpuffer entsprach in der Regel dem für die weitere Verwendung des Proteins benötigten Reaktionspuffer.

Für die Aufreinigung mit Zentrifugaleinheiten wurden HisPur™Ni-NTA Resin Spin Columns (Thermo Fischer Scientific, Waltham, USA) mit einem Bettvolumen von 3 mL verwendet. Die Vorgehensweise entsprach dabei den Herstellerangaben. Die Aufkonzentrierung und Umpufferung entsprach der zuvor beschriebenen Durchführung.

### 5.2 Aufreinigung von Proteinen im L-Maßstab

Die Aufreinigung von Proteinen im L-Maßstab erfolgte ebenfalls auf Basis der IMAC. Hierfür wurde eine Chromatographie-Säule mit 600 mL-Volumen und einem Durchmesser von 50 mm verwendet, die mit Ni Sepharose 6 Fast Flow (GE Healthcare Life Science, Uppsala, Schweden) befüllt wurde. Vor dem Packen wurde das in 20 % Ethanol gelagerte Säulenmaterial durch dreimaliges Dekantieren, Wiederauffüllen mit Wasser, Aufschlämmen und Sedimentieren gewaschen. Anschließend wurde das aufgeschlämmte Medium in die leere Säule gegeben und die Säule bei einer maximalen Flussrate von 150 mL $min^{-1}$ und einem maximalen Druck von 1,5 bar gepackt. Die fertig gepackte Säule wurde danach mit 5 CV Bindepuffer bei einem maximalen Druck von 1,2 bar äquilibriert. Die aufzutragende Probe wurde nach dem Auftauen zunächst mit 500 mM NaCl versetzt und mit 1 M NaOH auf pH 7,4 eingestellt. Anschließend erfolgte eine Querstromfiltration mit zwei Sartocon® Slice Hydrosart® Filterkassetten (0,2 μm Ausschlussgröße, je 0,1 m2 Filterfläche, Sartorius Stedim Biotech, Göttingen) mit in Reihe geschaltetem Sterilfilter (0,2 μm Ausschlussgröße), um die Probe zu klären. Die Proben wurden danach mit einem maximalen Druck von 1,2 bar entgegen der Elutionsrichtung auf die Säule aufgetragen. Anschließend wurde die Säule in Elutionsrichtung mit Bindepuffer gewaschen, bis das UV-Signal des Eluatstroms wieder eine
Basislinie anzeigt. Die Elution des Proteins erfolgte über einen von 0 % bis 100 % über 180 min linear ansteigenden Elutionspuffergradienten, wobei Fraktionen von jeweils 2 L gesammelt wurden. Durch das UV-Signal des Detektors konnten die Fraktionen identifiziert werden, die das Zielprotein enthalten. Diese Fraktionen wurden im Anschluss mittels Querstromfiltration mit zwei Sartocon® Slice Hydrosart® Filterkassetten (10 kDa Ausschlussgröße, je 0,1 m2 Filterfläche, Sartorius Stedim Biotech, Göttingen) zunächst aufkonzentriert und anschließend durch Diafiltration mit 5-10-fachem

Austauschvolumen an Zielpuffer umgepuffert.

### 5.3 Natriumdodecylsulfat-Polyacrylamidgelelektrophorese (SDS-PAGE)

Die analytische Auftrennung von Proteingemischen erfolgte mittels einer diskontinuierlichenNatriumdodecylsulfat-Polyacrylamidgelelektrophorese (SDS-PAGE) mit 12,5 %-igem Trenn- und 3 %-igem Sammelgel (Laemmli, U. K., Nature, 227(5259);680-5, 1970; Fling, S. P.& Gregerson, D. S., Anal. Biochem., 155(1):83-8, 1986. Für das Trenngel wurde 17,5 mL destilliertes Wasser mit 10 mL Trenngelpuffer (4x) und 12,5 mL Acrylamid (40 %) vermischt und die Polymerisation mit 100 μL Ammoniumpersulfat (APS, 10 %) und 10 μL Tetramethylethylendiamin (TEMED) gestartet. Die Zusammensetzung des Sammelgels besteht aus 15 mL destilliertem Wasser, 20 mL Sammelgelpuffer (2x), 5 mL Acrylamid (40 %), 100 μL APS und 10 μL TEMED. Proteinproben wurden vor dem Auftragen mit Laemmli-Puffer versetzt und 5 min bei 95 °C inkubiert, um die Proteine zu denaturieren. Anschließend wurden diese auf das Gel aufgetragen, als Größenstandard diente Roti® -Mark Standard (14-212 kDa, Carl Roth, Karlsruhe). Die Elektrophorese erfolgte in einer Elektrophoresekammer (PEQLAB, Erlangen) mit Rotiphorese® SDS-PAGE (Carl Roth, Karlsruhe) als Laufmittel bei einer konstanten Stromstärke von 30 mA pro Gel. Zur Färbung der Proteinbanden wurde Roti®-Blue-Färbelösung (Carl Roth, Karlsruhe) gemäß Herstellerangaben verwendet.

### 5.4 Proteinkonzentrationsbestimmung mittels Bicinchoninsäure-Assay (BCA-Assay)

Gesamtproteinkonzentrationen wurden mit dem Pierce™ BCA Protein Assay Kit (Thermo Scientific, Rockford, USA) nach Herstellerangaben gemessen. Als Proteinstandard diente ein im Kit enthaltener Standard aus Bovinem Serumalbumin (BSA).

### 5.5 Bestimmung von Enzymaktivitäten im Mikrotiterplattenphotometer

Enzymaktivitätsbestimmungen wurden im Mikrotiterplattenphotometer mit einem Probenvolumen von 250 μL bei 30 °C durchgeführt, bei der die Änderung der NAD(P)H-Konzentration ($\varepsilon$ 340 = 6,22 mL $\mu mol^{-1}$ $cm^{-1}$) bei einer Wellenlänge von $\lambda$= 340 nm verfolgt wurde. Die Aktivitäten wurden hierbei durch lineare Regression im linear verlaufenden Teil der Reaktion ermittelt. Dabei wurde die zu untersuchenden Probe mit Kaliumphosphatpuffer (50 mM, pH 8,0) entsprechend verdünnt und mit Substrat und Kofaktor versetzt. Die Endkonzentrationen an Substrat und Kofaktor waren für 7β-HSDH 10 mM DHCA und 100 μM NADPH, für 3α-HSDH 10 mM DHCA und 100 μM NADH, und für GDH 200 mM Glucose und 1000 μM NAD(P).

Sämtliche Substrate und Kofaktoren waren in Kaliumphosphatpuffer (50 mM, pH 8,0) gelöst. Sämtliche Messungen wurden im Dreifachansatz durchgeführt, anschließend wurde der Mittelwert ermittelt. Bei der Bestimmung der Artificial Units (AU) für die mechanistische Modellierung der multienzymatischen Reduktion von DHCA zu 12-Keto-UDCA wurde das Protokoll zur Enzymaktivitätsbestimmung modifiziert. Hierbei diente als Reaktionspuffer ein Kaliumphosphatpuffer (100 mM, pH 7,0), dem 20 % (v/v) Glycerin, 0,6 % (w/v) BSA und 0,006 % (v/v) Antifoam 204 (Sigma-Aldrich, München) beigemengt wurde. Die Konzentrationen an Substrat und Kofaktor war 500 μM DHCA und 200 μM NADH für 3α-HSDH, 500 μM DHCA und 200 μM NADPH für 7β-HSDH sowie 200 mM Glucose und 1000 μM NAD für GDH. Die Enzyme wurden in Verdünnungen eingesetzt, bei denen die gemessene anfängliche Extinktionsveränderung bei der GDH im Bereich 0,0005 - 0,0020 $s^{-1}$ und bei den HSDH 0,00025 - 0,00100 $s^{-1}$ betrug. Sämtliche Messungen wurden im Achtfachansatz durchgeführt, anschließend wurde der 25 % gestutzte Mittelwert ermittelt. Ein AU wird hierbei als die Menge an aktivem Enzym definiert, die unter diesen genannten Reaktionsbedingungen die Umsetzung von 1 μmol Substrat bzw. Kofaktor innerhalb von 1 min katalysiert.

## 6. Analytische Methoden

### 6.1 Bestimmung der optischen Dichte von Bakteriensuspensionen

Die optische Dichte von E. coli-Suspensionen wurde in einem Küvettenphotometer in Küvetten mit 1 cm Schichtdicke bei einer Wellenlänge von $\lambda$ = 600 nm gemessen. Die Bakteriensuspension wurde gegebenenfalls mit entsprechendem Medium oder Puffer verdünnt, so dass die gemessene Extinktion 0,5 nicht überstieg.

### 6.2 Bestimmung der Biotrockenmassenkonzentration von Bakteriensuspensionen

Die Bestimmung der Biotrockenmassenkonzentration erfolgte gravimetrisch, sofern nicht anders angegeben. Hierzu wurde 1 mL der entsprechenden Bakteriensuspension in vorgetrocknete und vorgewogene 1,5 mL-Reaktionsgefäße gegeben, anschließend wurden die Zellen in einer Tischzentrifuge bei 13000 rpm 10 min bei Raumtemperatur

pelletiert und der Überstand verworfen. Danach wurden die Gefäße bis zur Gewichtskonstanz getrocknet und erneut gewogen. Die Biotrockenmassenkonzentration konnte anschließend mittels folgender Gleichung berechnet werden.

$$c_X = (m_{voll} - m_{leer})/V$$

mit:

$c_X$ = Biotrockenmassenkonzentration, gBTM $L^{-1}$
$m_{voll}$ = Masse des mit Probenmaterial gefüllten Reaktionsgefäßes nach der Trocknung, g
$m_{leer}$ = Masse des leeren Reaktionsgefäßes nach der Trocknung, g
V = Volumen der Zellsuspension vor der Sedimentation, L

**6.3 Photometrische Bestimmung von Kofaktorkonzentrationen**

Konzentrationen von NAD(P) und NADP(H) wurden photometrisch in einem Küvettenphotometer nach dem Lambert-Beer'schen-Gesetz bestimmt. Hierzu wurde eine Quarzküvette (Hellma Analytics, Mülheim) mit einer Schichtdicke von 1 cm verwendet, die mit 1 mL Probe gefüllt war. Die Bestimmung der NAD(P)-Konzentration wurde bei einer Wellenlänge von $\lambda$ = 259 nm durchgeführt, wobei der molare Extinktionskoeffizient $\varepsilon_{259\ nm}$ = 16,9 mL $\mu mol^{-1}$ $cm^{-1}$ betrug. Die Konzentrationen von NAD(P)H wurden bei einer Wellenlänge von $\lambda$ = 340 nm bestimmt, hierbei betrug der molare Extinktionskoeffizient $\varepsilon_{340\ nm}$ = 6,22 mL $\mu mol^{-1}$ $cm^{-1}$.

**6.4 Hochauflösende Flüssigkeitschromatographie (HPLC)**

Die qualitative und quantitative Analytik von Gallensalzen erfolgte durch eine Auftrennung der Substanzen mittels HPLC. Hierzu wurde das HPLC-System Finnigan Surveyor Plus (Thermo Fischer Scientific, Waltham, USA) mit einer Umkehrphasen-Chromatographiesäule des Typs Hibar® 125-4 RP-18e (5 $\mu$m) (Merck, Darmstadt) verwendet. Als mobile Phase diente ein Laufmittelgemisch aus phosphorsauren Wasser (pH 2,6) und Acetonitril, wobei für die Auftrennung ein Laufmittelgradient verwendet wurde. Die Flussrate der mobilen Phase beträgt 1 mL $min^{-1}$ und es wurden jeweils 20 $\mu$L Probe injiziert. Detektiert werden die Gallensalze mittels UV-Extinktion bei $\lambda$ = 200 nm. Kalibriert wurde die Methode mit Referenzsubstanzen nach gängigen Methoden.

Das Gradientenprofil war wie folgt:

0-3 min: konstanter Anteil an Acetonitril von 35 % (v/v), 3-7 min: lineare Zunahme des Acetonitrilanteils auf 39 % (v/v), 7-8 min: lineare Zunahme des Acetonitrilanteils auf 70 % (v/v),
8-9,5 min: konstanter Anteil an Acetonitril von 70 % (v/v), 9,5-10,5 min: lineare Abnahme des Acetonitrilanteils auf 35 % (v/v), 10,5-14 min: konstanter Anteil an Acetonitril von 35 % (v/v).

**6.5 Durchflusszytometrie (FACS)**

Die Durchflusszytometrie (engl. fluorescence-activated cell sorting, FACS) wurde verwendet, um die Zellintegrität von Ganzzellbiokatalysatoren zu untersuchen. Hierzu wurden die Zellen mit PBS auf eine Partikeldichte von ca. 109 $mL^{-1}$ verdünnt, was bei einer Flussrate von 1 mL $s^{-1}$ 1000 Signalen $s^{-1}$ entspricht. Diese Zellen wurden mit 0,75 mM des Farbstoffs bis- (1,3-Dibutylbarbituräure)-Trimethinoxonol (Dibac4[3]) versetzt, welcher depolarisierte Zellmembranen überwindet und durch Bindung an intrazelluläre Proteine und Membranen eine gesteigerte Fluoreszenz erzeugt. Durch Auftragung der Dibac4[3]-vermittelten Fluoreszenz gegen die Lichtstreuung der Partikel, welche ein Indikator für die Partikelgröße ist, konnten Hinweise auf die Zellintegrität bezogen werden (Suller, M. T.& Lloyd D., Cytometry, 35(3):235-41, 1999; Langemann et al., Bioeng. Bugs, 1(5):326-36, 2010).

**6.6 Standardisierte Prüfmethode zur Bestimmung der enzymatischen Umsetzung von DHCA zu 12-Keto-UDCA (IPC-Methode)**

**a. Ausrüstung**

Apparatur: HPLC mit UV-Detektor und Autosampler (Merck Hitachi, LaChrom Elite (Hochdruck-Gradientensystem) oder vergleichbar);

Säule: Merck, Purospher® STAR RP-18e, 125 mm X 4,0 mm, 5 μm, Art. #510 036; oder vergleichbar

**b. Reagenzien geeignet für Gradientenerzeugung**

| | |
|---|---|
| Acetonitril | Merck LiChroSolv® |
| Wasser | Reinstwasser |
| $H_3PO_4$ | ortho-Phosphorsäure 85,0%-ig; Merck |
| Methanol | Merck LiChroSolv® |

**c. HPLC-Parameter**

| | | | | |
|---|---|---|---|---|
| Flussrate | 1,0 ml/min | | | |
| Säulentemperatur | 25 °C | | | |
| Injektionsvolumen | 20 μl | | | |
| Gradient | Zeit (min) | Eluent A (%) | Eluent B (%) | Kurve |
| | | | | |
| | 0,0 | 80,0 | 20,0 | - |
| | 15,0 | 65,0 | 35,0 | linear |
| | 20,0 | 65,0 | 35,0 | linear |
| | 21,0 | 15,0 | 85,0 | linear |
| | 30,0 | 15,0 | 85,0 | linear |
| | 31,0 | 80,0 | 20,0 | linear |
| | 36,0 | 80,0 | 20,0 | linear |
| Detektion | UV 200 nm | | | |
| Laufzeit | 36,0 min | | | |
| Waschen | Methanol / Wasser: 9/1 (v/v) | | | |

**d. Herstellung der Lösungen und Proben**

| | |
|---|---|
| Mobile Phase A | $H_2O$ (eingestellt auf pH 2,6 mit $H_3PO_4$ (85%)) |
| Mobile Phase B | Acetonitril |
| Blank | Verdünnungsmittel: Methanol / Wasser: 9/1 (v/v) |

System Suitability Solution (SST) 5,00 mg DHCA, 5,00 mg 12-Keto-UDCA, 5,00 mg 3,12-Diketo-UDCA und 5,00 mg 7,12-Diketo-CA (jeweils genau eingewogen) in 10,0 ml Verdünnungsmittel

Testlösung 1 ml Reaktionslösung, verdünnt mit 9 ml Verdünnungsmittel; behandelt mit Ultraschall bei Zimmertemperatur und 10 min. zentrifugiert.

**e. Ablauf**

Analysensequenz:

| | |
|---|---|
| - Blank | 1 x Injektion |
| - System Suitability Solution (SST) | 1 x Injektion |
| - Testlösung | 2 x Injektion |
| - Blank | 1 x Injektion |

Regenerierung:

Nach jeder Sequenz wird die Säule mit Methanol / Wasser (4 / 6 bis 9 / 1 (v/v)) regeneriert. Dann wird die Säule mit Acetonitril / Wasser (4 / 6 (v/v)) gespült.

**f. Auswertung**

Bestimmung der Retentionszeiten (Rt) der Verbindungen aus Analyse der SST Probe.

Flächen- % Analyse von

- DHCA (Edukt)
- 12-Keto-UDCA (Produkt)
- 3,12-Diketo-UDCA (Intermediat)
- 7,12-Diketo-CA (Intermediat)

Retentionszeiten:

| Analyt | ca. RT (min) | RRT |
|---|---|---|
| 12-Keto-UDCA | 13,9 | 1,00 |
| 3,12-Diketo-UDCA | 14,5 | 1,04 |
| 7,12-Diketo-CA | 16,5 | 1,18 |
| DHCA | 17,9 | 1,28 |
| RT: Retentionszeit<br>RRT: Relative Retentionszeit | | |

**7. Stereoselektive Reduktion von Dehydrocholsäure**

**7.1 Satz-Reduktion im 2 mL-Maßstab**

Satz-Reduktionen im 2 mL-Maßstab wurden für die mechanistische Modellierung der multienzymatischen Reduktion von DHCA zu 12-Keto-UDCA als Validierungsexperimente durchgeführt. Hierzu wurden Deep Well-Platten mit quadratischen Vertiefungsöffnungen und V-förmigen Boden mit einem Nennvolumen von 2,0 mL pro Vertiefung verwendet. Zur Durchmischung wurden die Deep-Well-Platte auf einem Laborschüttler bei 500 rpm geschüttelt. Um Temperaturkonstanz zu gewährleisten, befand sich der gesamte Aufbau in einem auf 30 °C temperierten Inkubationsschrank. Die Reaktionsgemische, bestehend aus DHCA, NAD, NADP, 3α-HSDH, 7β- -HSDH und GDH lagen in Kaliumphosphatpuffer (100 mM, pH 7,0) mit Zusatz von 0,6 % ((w)/v) BSA vor und wurden direkt in den Vertiefungen angesetzt. Gestartet wurde die Reaktion durch Zugabe von Glucoselösung und anschließendem dreimaligen Invertieren der Deep-Well-Platte. Verwendet wurden Enzyme, die gemäß Abschnitt 5.2 aufgereinigt wurden. Die Probenahme erfolgte halbstündlich durch Entnahme von 100 μL Reaktionsansatz, welcher direkt mit 900 μL Methanol (77 %, v/v) versetzt wurde. Die mit Methanol versetzten Proben wurden anschließend durch Vortexen vermischt und 10 min bei 13000 rpm in einer Tischzentrifuge bei Raumtemperatur zentrifugiert. Der Überstand wurde danach mittels HPLC analysiert. Sämtliche

Reaktionen wurden im Dreifachansatz durchgeführt.

### 7.2 Satz-Reduktion im 20 mL-Maßstab

Stereoselektive Reduktionen von DHCA im 20 mL-Maßstab erfolgten in Enghals-Schraubverschlussflaschen (DURAN Group, Wertheim/Main) mit einem Nennvolumen von 50 mL, einem Innendurchmesser von 41 mm und einem GL32-Schraubverschlussgewinde. Für die Durchmischung sorgte ein kreuzförmiger Magnetrührer bei 450 rpm, der von einer Mehrfachrührplatte (Variomag Multipoint, Thermo Scientific, Waltham, USA) angetrieben wurde. Um Temperaturkonstanz zu gewährleisten, befand sich der gesamte Aufbau in einem temperierten Inkubationsschrank. Für die Reaktionsansätze wurde das Substrat DHCA zunächst mit äquimolarer Menge an NaOH vorgelöst. Der Reaktionsansatz, bestehend aus DHCA, Kosubstrat (Glucose oder Formiat), und gegebenenfalls weiteren Zusätzen wie NAD(P), Glycerin, $MgCl_2$ wurde vor Beginn der Reaktion zusammengemischt. Als Puffer diente Kaliumphosphat (50 mM) und der pH wurde wahlweise mit Natronlauge, Phosphorsäure oder Ameisensäure (jeweils 5 M) auf den gewünschten Wert eingestellt. Gestartet wurde die Reaktion durch die Zugabe von Ganzzellbiokatalysatoren. Wahrend der Reaktion wurde der pH in 30-min"utigen Abständen mit einem Hand-pH-Meter (pH-Tester Checker®, Carl Roth, Karlsruhe) gemessen und gegebenenfalls mit Natronlauge, Phosphorsäure oder Ameisensäure (jeweils 5 M) auf den Ausgangswert eingestellt. Die Probenahme erfolgte in Abständen von 30 oder 60 Minuten, indem 300 $\mu$L Reaktionsansatz entnommen und mit 700 $\mu$L Methanol ($\geq$ 99,9 %) vermischt wurden. Anschließend wurde das Proben-Methanol-Gemisch 3:10 mit 70 %-igem Methanol (v/v) verdünnt und 10 min bei 13000 rpm in einer Tischzentrifuge bei Raumtemperatur zentrifugiert. Vom Überstand wurde eine Probe entnommen und 1:10 mit Methanol (70 %, v/v) verdünnt, in Probiergläser gegeben und mittels HPLC analysiert. Sämtliche Reaktionen erfolgten im Dreifachansatz

### 7.3 Satz-Reduktion im 1 L-Maßstab

Stereoselektive Reduktionen von DHCA im 1 L-Maßstab erfolgten in einem 1,5 L Rührkesselreaktor der Firma Infors AG (Infors 3, Bottmingen, Schweiz) ohne Strömungsbrecher. Der Reaktor war mit Sonden für Temperatur und pH ausgestattet, so dass diese Parameter von einer Steuerungseinheit online abgelesen und gegebenenfalls geregelt werden konnten. Temperiert wurde der Reaktor über einen an die Steuerungseinheit angeschlossenen Doppelmantel und die Durchmischung erfolgte durch zwei Sechsblatt-Scheibenrührer bei 500 - 1000 rpm, die über einen Motor am Deckel des Reaktors angetrieben wurden. Zudem konnte über Zulaufpumpen Säure (Phosphorsäure, 5 M) oder Base (Natronlauge, 5 M) zur pH-Regulation in den Reaktor zugegeben werden. Das Substrat DHCA wurde entweder in äquimolarer Natronlauge vorgelöst oder direkt als freie Säure in Pulverform zugegeben. Sämtliche weitere Bestandteile wurden ebenfalls entweder als Feststoff oder als Stammlösung zugegeben. Nach Auffüllen auf das entsprechende Volumen und der Einstellung des gewünschten pH wurde die Reaktion mittels Zugabe von Ganzzellbiokatalysatoren gestartet. Die Probenahme erfolgte im Abstand von 30 oder 60 min wie unter Abschnitt 7.2 beschrieben.

### 7.4 Isolation von Gallensalzen durch Säurepräzipitation

Die Isolation von Gallensalzen beruht auf der geringen Löslichkeit von Gallensalzen in ihrer protonierten Form bei saurem pH. Hierzu wird ein Gemisch mit gelösten Gallensalzen im gerührten Zustand mit Salzsäure (6 M) tröpfchenweise auf pH <= 2,0 titriert. Die gelösten Gallensalze fallen hierbei nahezu vollständig als Feststoff aus. Dieser wurde anschließend mit Hilfe eines Büchnertrichters mit eingesetztem Filterpapier (Durchmesser 150 mm, Rückhaltebereich $\geq$ 4 $\mu$m) von der restlichen Lösung getrennt. Bei Bedarf konnte das Gallensalz gewaschen werden, indem es in Reinstwasser gegeben und durch Titration mit Natronlauge (5 M) auf pH 8 - 9 gelöst, mit dem Büchnertrichter filtriert und anschließend wieder durch Säurepräzipitation isoliert wurde. Für die Darstellung von 7,12-Diketo-UDCA und 12-Keto-UDCA erfolgte ein zweimaliger Waschvorgang, für die Darstellung von 3,12-Diketo-UDCA erfolgte der Waschvorgang einmal. Anschließend wurde das isolierte Gallensalz bei 60 °C bis zur Gewichtskonstanz getrocknet.

## 8. Standardisierte Prüfvorschrift für die Umsetzung von Dehydrocholsäure (DHCA) zu 12-Keto-ursodeoxycholsäure (12-Keto-UDCA) mittels Ganzzell-Katalysator (mit 3$\alpha$-HSDH, 7$\beta$-HSDH und GDH-Aktivität)

### 8.1 Reagenzien

$K_2HPO_4$ * 3 $H_2O$ ($\geq$ 99 %, p.a.); Fa. Roth
$KH_2PO_4$ (kristallin, reinst); Fa. Merck
$C_6H_{12}O_6$ * $H_2O$ ($\geq$ 99,5%, Ph. Eur.); Fa. Roth

$MgCl_2$ * 6 $H_2O$ (≥ 99 %, p.a.); Fa. Roth
DHCA Fa. PharmaZell
β-NAD: Fa. Roth
β-NADP-$Na_2$ Fa. Merck,
Ganzzell-Katalysator (mit 3α-HSDH, 7β-HSDH und GDH-Aktivität) bei -20 °C gelagert)
HCl (37%)
NaOH (10%)

**8.2 Herstellung der Pufferlösung (Vorratslösung)**

Zur Herstellung der Puffer-Vorratslösung werden nacheinander 2,54 g Di-Kaliumhydrogenphosphat Trihydrat und 0,18 g Kaliumdihydrogenphosphat eingewogen und in 1000 ml VE-Wasser gelöst; pH-Wert der Lösung: 7,8 bei 25 °C.

**8.3 Herstellung der NAD/NADP-Lösung (Vorratslösung)**

Zur Herstellung der NAD/NADP-Vorratslösung werden nacheinander 158 mg β-NADP-$Na_2$ und 663 mg β-NAD in einen 1000ml-Messkolben eingewogen, mit VE-Wasser aufgefüllt und gelöst.

**8.4 Probenvorbereitung des Ganzzell-Katalysators (Zellsuspension)**

Vor Entnahme der Zell-Suspension ist die Probe auf Raumtemperatur (RT) zu erwärmen. Dauer der Erwärmung ca. 30 min bis ca. 3 Stunden.

**8.5 Reaktionsansatz (Ganzzell-Umsetzung und DHCA zu 12-Keto-UDCA)**

180 ml der o.g. Puffer-Vorratslösung werden im Erlenmeyerkolben auf 26 - 28 °C vorgewärmt. Anschließend werden 13,87 g (0,07 mol) α-D(+)-Glucosemonohydrat im 250ml-Dreihalskolben mit ca. 2/3 des Volumens des vorgewärmten Puffers gelöst. 5,64 g (0,014 mol) Dehydrocholsäure (DHCA) zusammen mit dem restlichen Puffer in der Glucose-Pufferlösung suspendiert und auf 26 - 28 °C im Wasserbad erwärmt, dabei sinkt der pH-Wert bereits und wird auf 6,8 eingestellt. Ohne Zeitverzug werden der Suspension nacheinander 36 mg (0,18 mmol) Magnesiumchloridhexahydrat, 10 ml der NAD/NADP-Vorratslösung und 5 ml aufgetaute Zellsuspension hinzugefügt.
Die Reaktionssuspension wird 8 h im Wasserbad (bei 26 - 28 "C) gerührt. Der pH-Wert der Suspension soll bei Verwendung eines pH-Meters und manueller pH-Nachführung (Titration mit 10%-iger NaOH-Lösung) stets zwischen pH 6,70 und 6,90 liegen. Beim alternativen Einsatz eines pH-Stat soll der pH stets bei pH 6,8 liegen.

**8.6 HPLC-Analyse**

Nach 0,5h, 1h, 1,5h, 2h, 2,5h, 3h, 4h, 5h, 6h, 7h und 8h Reaktionszeit werden zur Reaktionsverfolgung Proben (1 ml Volumen) der Reaktionssuspension mit einer Pipette entnommen und mittels HPLC analysiert (vgl. IPC-Methode oben):
1 ml Probenvolumen wird hierzu in einem 20 ml Schnappdeckelglas mit 9 ml Lösungsmittelmix aus Methanol/$H_2O$ (9:1; v/v) verdünnt und gut durchmischt. Das Schnappdeckelglas wird verschlossen. Vor Injektion auf die HPLC-Säule muss die trübe, verdünnte Lösung zentrifugiert werden. Anschließend wird der klare Überstand abgehoben und hieraus das Injektionsvolumen entnommen.
Die Reaktion wird nach geeigneter Zeit, z.B. 7 bis 10 h, wie z.B. nach 8h Reaktionszeit beendet. Hierzu wird die trübe Reaktionslösung mit ca. 4 - 5 ml konz. HCl angesäuert (pH ≤ 1,5) und für 30 min. nachgerührt.

**8.7 Auswertung**

Ermittlung der Reaktionskinetik:

Die Reaktionsverfolgung/Reaktionskinetik setzt sich aus dem (nicht analysierten) Startpunkt "0h" (für DHCA: 100 Area %, für alle weiteren Analyten: 0 Area %) und z.B. 11 weiteren Messpunkten / HPLC-Analysen (z.B. nach 0,5h, 1h, 1,5h, 2h, 2,5h, 3h, 4h, 5h, 6h, 7h und 8h Reaktionszeit) zusammen.
Die Zusammensetzung der Analyten im aktuellen Probenzug (zum Zeitpunkt: x h Reaktionsdauer) ist zu ermitteln. Hierzu werden die Area-%-Angaben im HPLC-UV-Chromatogramm zu den Analyten DHCA (Edukt), 12-Keto-UDCA (Endprodukt), 3,12-Diketoursodeoxycholsäure (3,12-Diketo-UDCA; Intermediat) und 7,12-Diketo-cholsäure (7,12-Diketo-CA; Intermediat) ausgewertet und protokolliert.

## B. Ganzzell-Reduktion

***Beispiel B.1: Zweistufige Ganzzellreduktion von DHCA mit drei verschiedenen Ganzzellbiokatalysatoren (X = 67).***

Für die Umsetzungen wurden die Biokatalysatorstämme *E. coli* BL49 p7(A)T3rG, *E. coli* BL21 ΔhdhA p7(A)T3rG-K und *E. coli* BL49 p7(A)T3TG verwendet. Die Plasmide p7(A)T3rG, p7(A)T3rG-K und p7(A)T3TG verfügen jeweils über Expressionskassetten, in denen die Gene 7β-HSDH, 3α-HSDH und GDH codiert sind, allerdings mit unterschiedlicher Struktur der Expressionskassette und mit unterschiedlichen Antibiotikaresistenzen. Diese Plasmide wurden wahlweise in den Wirtsstamm *E. coli* BL49 oder *E. coli* BL21 ΔhdhA (beide aus der WO 2012/080504 bzw der WO 2011/147957 der Anmelderin bekannt) transformiert, bei denen es sich unterschiedliche Knock-out-Stämme handelt, bei denen jeweils die genomische 7α-HSDH ausgeknockt wurde. Die Expressionsplasmide sind in Figur 3 dargestellt.

Mit den genannten Biokatalysatoren wurden Umsetzungen im 1 L-Maßstab durchgeführt. Dabei wurden jeweils 1 $g_{BTM}$ $L^{-1}$ Biokatalysator, 0,05 mM NAD und 0,01 mM NADP eingesetzt, somit ergibt sich für alle drei Ansätze X = 67. Weitere Bedingungen der Umsetzung waren: 70 mM DHCA, 350 mM Glucose, 10 mM $MgCl_2$, 50 mM Kaliumphosphatpuffer, pH 7, 30 °C. Maßgeblich für die Beurteilung der Umsetzung ist die Menge des gebildeten Produktes (12-keto-UDCA) im Reaktionsansatz. Die Konzentrationen des Substrats DHCA, der Intermediate 3,12-diketo-UDCA und 7,12-diketo-UDCA sowie des Produkts 12-keto-UDCA im Reaktionsansatz kann durch Hochleistungsflüssigkeitschromatographie (HPLC) ermittelt werden.

Die Verläufe der Reaktionen sind in Figur 4 dargestellt. Mit sämtlichen Ansätzen konnten nach 5-6 h ein Umsatz von > 99 % erzielt werden. Somit ist ersichtlich, dass die Formel für verschiedene Ganzzellbiokatalysatorstämme gültig ist, so lange alle drei Enzyme 7β-HSDH, 3α-HSDH und GDH exprimiert werden.

***Beispiel B.2 Zweistufige Ganzzellreduktion von DHCA mit 3,5 $g_{BTM}L^{-1}$ Biokatalysator und 0,025 mM NAD (X = 147,5).***

Für die Umsetzung wurde der Biokatalysatorstamm *E. coli* BL49 p7(A)T3rG verwendet. Dabei wurden 3,5 $g_{BTM}$ $L^{-1}$ Biokatalysator, 0,025 mM NAD und kein NADP eingesetzt, somit ergibt sich für diesen Ansatz X = 147,5. Weitere Bedingungen der Umsetzung waren: 70 mM DHCA, 350 mM Glucose, 10 mM $MgCl_2$, 50 mM Kaliumphosphatpuffer, pH 7, 30 °C, 20 mL Reaktionsvolumen.

Der Verlauf der Reaktion ist in Figur 5 dargestellt. Mit diesem Ansatz kann nach 24 h ein Umsatz von > 99 % erzielt werden. Somit hat die Formel Gültigkeit für diesen Ansatz. Weiterhin wird gezeigt, dass nicht zwingend notwendig NAD und NADP zugegeben werden muss, sondern auch die Zugabe eines der Substanzen ausreichend sein kann.

***Beispiel B.3 Zweistufige Ganzzellreduktion von DHCA mit 1,75 $g_{BTM}$ $L^{-1}$ Biokatalysator, 0,025 mM NAD und 0,01 mM NADP (X = 89,5).***

Für die Umsetzung wurde der Biokatalysatorstamm *E. coli* BL49 p7(A)T3rG verwendet. Dabei wurden 1,75 $g_{BTM}$ $L^{-1}$ Biokatalysator, 0,025 mM NAD und 0,01 mM NADP eingesetzt, somit ergibt sich für diesen Ansatz X = 89,5. Weitere Bedingungen der Umsetzung waren: 70 mM DHCA, 350 mM Glucose, 10 mM $MgCl_2$, 50 mM Kaliumphosphatpuffer, pH 7, 30 °C, 20 mL Reaktionsvolumen.

Der Verlauf der Reaktion ist in Figur 6 dargestellt. Mit diesem Ansatz kann nach 24 h ein Umsatz von > 98 % erzielt werden. Somit hat die Formel Gültigkeit für diesen Ansatz.

***Beispiel B.4 Zweistufige Ganzzellreduktion von DHCA mit 1 $g_{BTM}$ $L^{-1}$ Biokatalysator, 0,04 mM NAD und 0,0075 mM NADP (X = 61).***

Für die Umsetzung wurde der Biokatalysatorstamm *E. coli* BL21 ΔhdhA p7(A)T3rG-K verwendet. Dabei wurden 1 $g_{BTM}$ $L^{-1}$ Biokatalysator, 0,04 mM NAD und 0,0075 mM NADP eingesetzt, somit ergibt sich für diesen Ansatz X = 61. Weitere Bedingungen der Umsetzung waren: 70 mM DHCA, 200 mM Glucose, 5 mM $MgCl_2$, 4 % (v/v) Glycerin, 50 mM Kaliumphosphatpuffer, pH 7, 30 °C, 20 mL Reaktionsvolumen.

Der Verlauf der Reaktion ist in Figur 7 dargestellt. Mit diesem Ansatz kann nach 24 h ein Umsatz von > 99 % erzielt werden. Somit hat die Formel Gültigkeit für diesen Ansatz.

***Beispiel B.5 Zweistufige Ganzzellreduktion von DHCA mit dem Ganzzellbiokatalysators E. coli BLLiu p7(A)T3TG-K***

Es wurde ein kanamycinresistender Ganzzellbiokatalysatorstamm mit erhöhter GDH-Expression hergestellt. Das Plasmid p7(A)T3rG-K wurde dabei dahingehend modifiziert, dass in die Expressionskassette ein zusätzlicher T7-Promotor vor der GDH eingefügt wurde. Das resultierende Plasmid trägt die Bezeichnung p7(A)T3TG-K und wurde in den Wirtsstamm *E. coli* BLLiu transformiert. Der resultierende Ganzzellbiokatalysator trägt die Bezeichnung *E. coli* BLLiu p7(A)T3TG-K.

**a) Kultivierung des Ganzzellbiokatalysators *E. coli* BLLiu p7(A)T3TG-K**

Der Stamm *E. coli* BLLiu p7(A)T3TG-K wurde im Rührkesselreaktor nach Standardprotokoll kultiviert und hinsichtlich des Wachstums- und Expressionsverhaltens mit verwandten Stämmen verglichen.

Der Stamm *E. coli* BLLiu p7(A)T3TG-K wurde dazu nach Standardprotokoll im Rührkesselreaktor bei einer Expressionstemperatur von 25 °C kultiviert. Die bei Erntezeitpunkt vorliegenden Zellkonzentrationen und Enzymaktivitäten sind in folgender Tabelle 3 angegeben. Dem gegenübergestellt sind die Daten des ursprünglichen kanamycinresistenten Stamms *E.coli* BLLiu p7(A)T3rG-K und die Daten des ampicillinresistenten Stamms BL49 p7(A)T3TG mit ebenfalls erhöhter GDH-Aktivität

Aus der Tabelle lässt sich entnehmen" dass der neue Stamm *E. coli* BLLiu p7(A)T3TG-K über deutlich höhere 7β-HSDH-Aktivitäten verfügt als die jeweiligen Vergleichsstämme (Faktor 2,4-2,7). Die 3α-HSDH-Aktivität ist auf dem Niveau des höchsten Wertes unter den Vergleichsstämmen. Die GDH-Aktivität ist hingegen um den Faktor 4-10 höher als die des kanamycinresistenten Stamms *E. coli* BLLiu p7(A)T3rG-K, beträgt jedoch nur 70 % der GDH-Aktivität des ampicillinresistenten Stamms BL49 p7(A)T3TG. Die erzielte Zellkonzentration zum Erntezeitpunkt war durchgehend höher als bei den Vergleichsstämmen.

**Tabelle 3:** Vergleich der Wachstums und Expressionsdaten des Stamms *E. coli* BLLiu p7(A)T3TG-K mit den Stämmen *E. coli* BLLiu p7(A)T3rG-K und *E. coli* BL49 p7(A)T3TG

| Stamm | BLLiu p7(A)T3rG-K | | | BL49 p7(A)T3TG | BLLiu p7(A)T3TG-K |
|---|---|---|---|---|---|
| $T_{Expression}$, °C | 20 | 25 | 30 | 20 | 25 |
| OD, - | 69 | 64 | 66 | 62 | 78 |
| BTM, g $L^{-1}$ | 33 | 34 | 37 | 37 | 40 |
| 7β-HSDH, U $g_{BTM}^{-1}$ | 565 | 509 | 556 | 490 | 1360 |
| 3α-HSDH, U $g_{BTM}^{-1}$ | 111 | 135 | 74 | 70 | 135 |
| GDH, U $g_{BTM}^{-1}$ | 343 | 184 | 127 | 1900 | 1300 |

**b) Biotransformation des Ganzzellbiokatalysators *E. coli* BLLiu p7(A)T3TG-K**

Der im Rührkesselbioreaktor kultivierte neue Stamm *E. coli* BLLiu p7(A)T3TG-K wurde hinsichtlich der Ganzzellbiokatalyseleistung.

Für die Evaluation der Ganzzellbiokatalyseleistung wurden sowohl bei -20 °C gelagerte Zellen als auch bei Raumtemperatur und 4 °C gelagerte verwendet. Im Gegensatz zu vorherigen Stämmen reichte bei diesem Stamm eine Lagerdauer von 1 d bei Raumtemperatur nicht aus, um die volle Aktivität der Zellen zu erhalten. Deswegen wurde der Stamm nach 1 d Lagerung bei Raumtemperatur und 3 d Lagerung bei 4 °C erneut 3 d bei Raumtemperatur gelagert und anschließend für die Biotransformation verwendet.

Die Reaktionsbedingungen für die Ganzzellbiotransformationen waren: 70 mM DHCA, 350 mM Glucose, OD 2 Zellen, 50 μM NAD, 10 μM NADP, 1 mM $MgCl_2$, 50 mM KPi-Puffer (pH 7,0), 30 °C. Zusätzlich wurde bei den bei Raumtemperatur gelagerten Zellen ein Versuch mit der doppelten NAD-Konzentration von 100 μM durchgeführt. Der pH wurde halbstündlich manuell mit NaOH-Lösung (5 M) auf den Ausgangswert eingestellt. Die Verläufe der Biotransformationen sind in Figur 11 dargestellt.

Bei Vergleich der Biotransformationen bei standardmäßigen NAD-Konzentrationen (50 μM) waren die Reaktionen nach 4 h (bei -20 °C gelagerte Zellen) bzw. 4,5 h (bei RT/4 °C) gelagerte Zellen beendet. Die Dauer der Biotransformationen liegt damit im Rahmen bzw. leicht unterhalb der Zeiten, die mit anderen Biokatalysatorstämmen erzielt wurden (4,5 h-6 h). Erkennbar ist jedoch die ungleichmäßige Nebenproduktbildung. Bei beiden Ansätzen akkumuliert das Zwischenprodukt der 7β-HSDH (3,12-diketo-UDCA) stärker als das Zwischenprodukt der 3α-HSDH. Dies ist auf die deutlich erhöhte 7β-HSDH-Aktivität dieses Stammes im Vergleich zu anderen Ganzzellbiokatalysatorstämmen zurückzuführen. Wird die NAD-Konzentration - und somit die Kofaktorkonzentration für die 3α-HSDH-Umsetzung - erhöht, so resultiert das in einer Angleichung der Reaktionsgeschwindigkeiten beider HSDH, was in einer gleichmäßigen Bildung beider Zwischenprodukte zu erkennen ist. Zudem verringert sich die Dauer der Biotransformation von 4,5 h auf 4,0 h.

## C. 7β-HSDH-Mutanten

### *Beispiel C.1: Herstellung von 7β-HSDH Mutanten mit NADH Spezifität*

Hierin werden Enzymmutanten der 7β-HSDH aus *Collinsella aerofaciens* beschrieben, die mittels Protein Engineering hergestellt wurden, und die NADH anstelle von NADPH als Kofaktor akzeptieren.

Die erfindungsgemäßen 7β-HSDH-Mutanten unterscheiden sich gegenüber der publizierten Sequenz des nativen Enzyms in ihrer Aminosäuresequenz an den Positionen 39, 40, 41 und/oder 44, und von den bekannten 7β-HSDH-Mutanten an den Positionen 40, 41 und 44.

Die Mutante 7β-HSDH DF enthält gegenüber der Wildtypsequenz die Aminosäuresubstitutionen G39D R40F, die Mutante 7β-HSDH DFK enthält gegenüber der Wildtypsequenz die Aminosäuresubstitutionen G39D R40F R41K und die Mutante 7β-HSDH DFKG enthält gegenüber der Wildtypsequenz die Aminosäuresubstitutionen G39D R40F R41K K44G (vgl. Figur 8)).

### C.1.2 Herstellung der NADH-abhängigen Einfachmutante G39D

Die G39D-Mutante der 7β-HSDH wurde wie in der WO 2012/080504 beschrieben hergestellt. Das Protein wurde mit N-terminalem His-Tag exprimiert und über IMAC aufgereinigt. Ein erster Aktivitätsassay zeigte nur sehr geringe NADPH-Aktivität (0,040 ± 0,005 U $mg^{-1}$ bei 10 mM DHCA, 0,5 mM NADPH und pH 8,0). Dafür war eine deutliche NADH-Aktivität zu beobachten.

### C.1.3 Gerichtete Evolution der NADH-abhängigen 7β-HSDH

#### a) Herstellung des Wirtsstamms für die Mutantenbibliothek

Die weitere Mutagenese soll nach dem Prinzip der iterativen Sättigungsmutagenese erfolgen (Reetz et al., Mol. Biosyst., 5(2):115-22, 2009). Für die Herstellung einer Mutantenbibliothek wird hierzu ein E. coli-Wirtsstamm benötigt, der über eine hohe Transformationseffizienz verfügt und gleichzeitig gut für eine Durchmusterung mit Zelllysat geeignet ist. Da E. coli über eine genomisch codierte7β-HSDH verfügt, welche in der Durchmusterung eine Nebenreaktion katalysiert und somit Messdaten verfälschen kann, muss auf einen Wirtsstamm zurückgegriffen werden, bei dem dieses Gen ausgeschaltet ist. Die beiden vorhandenen Knockout-Stämme BL49 und BLLiu sind jedoch beides E. coli-BL21 (DE3)-Derivate, die über eine geringe Transformationseffizienz verfügen. Somit musste zunächst ein geeigneter Wirtsstamm hergestellt werden, indem die genomische 7α-HSDH ausgeschaltet wird.

Als Ausgangsstamm für den Knockout wurde E. coli NovaBlue(DE3) gewählt, einem K-12-Derivat mit hoher Transformationseffizienz. Gleichzeitig ermöglicht die DE3-Kassette eine Expression von Fremdprotein mit dem T7-Expressionssystem, wodurch sich dieser von E. coli DH5α unterscheidet. Die erfolgreiche Ausschaltung des Gens für die 7α-HSDH und das anschließende Ausschleusen des Knockout-Plasmids wurden durch Kultivierung auf entsprechenden Selektionsantibiotika, PCR und Sequenzierung bestätigt. Dieser Stamm trägt die Bezeichnung E. coli NB13.

#### b) Auswahl der zu mutierenden Positionen

Zu den konservierten Bereichen in NADPH-bindenden SDR (short chain dehydrogenase reductase) zählen vor allem basische Aminosäurereste in der Kofaktorbindetasche, welche die NADP(H)-Bindung durch Säure-Base-Interaktion mit der 2'-Phosphatgruppe an der Adenosinribose des NADP(H) stabilisieren (Carugo, O. & Argos, P., Proteins, 28(1):10-28, , 1997a + Proteins, 28(1): 29-40, 1997b; Woodyer et al., Biochemistry, 42(40):11604-14, 2003). Allen voran ist die zur Position 39 benachbarte Position 40 zu betrachten, an der sich bei NADPH-bindenden SDR in der Regel ein Arginin oder ein Lysin befindet (Bellamacina, C. R., FASEB J., 10(11):1257-69, 1996; Kallberg et al., Eur. J. Biochem., 269(18):4409-4417, 2002; Persson, B., Chem. Biol. Interact., 143-144:271-278, 2003).

Bei der 7β-HSDH befindet sich an der zur G39 benachbarten Position mit R40 ebenfalls eine basische Aminosäure. Zusätzlich wurden mit R41 und K44 zwei weitere basische Aminosäuren in unmittelbare Nähe zur Bindetasche identifiziert. Diese drei Positionen wurden als Zielpositionen für die iterative Sättigungsmutagenese festgelegt.

**c) Durchführung der gerichteten Evolution**

**Verwendete Primer:**

**7β-HSDH G39D R40F (DF).**

| | |
|---|---|
| 7beta mut G39D R40F fwd: | CGTCGTCATGGTCGACTTTCGCGAGG (SEQ ID NO: 14) |
| AntiMid rev: | CCGCCGCATCCATACCGCCAGTTGTTTACCC (SEQ ID NO: 15) |

**7β-HSDH G39D R40F R41K (DFK)**

| | |
|---|---|
| 7beta DF mut R41K fwd: | CGTCGTCATGGTCGACTTTAAAGAGGAGAAGCTG (SEQ ID NO:16) |
| AntiMid rev: | CCGCCGCATCCATACCGCCAGTTGTTTACCC (SEQ ID NO: 15) |

**7β-HSDH G39D R40F R41K K44G (DFKG)**

7beta DFK mut K44NDT fwd: GTCGACTTTAAAGAGGAGNDTCTGAACGTGCTC (SEQ ID NO:17)

Dieser Primer enthält ein degeneriertes Codon, so dass auch andere Aminosäure als G an Position 44 entstehen können.

AntiMid rev: CCGCCGCATCCATACCGCCAGTTGTTTACCC (SEQ ID NO: 15)

Die gerichtete Evolution wurde in drei Mutageneserunden durchgeführt, bei der jeweils eine Position mutiert wurde. Die Mutante jeder Runde, die bei der Durchmusterung die höchste NADH Aktivität zeigte, wurde zur Identifikation der Mutation sequenziert und diente bei der darauffolgenden Runde als Ausgangsmutante. Die Mutagenese erfolgte ausgehend von 7β-HSDH G39D (D) zunächst an Position R40. Die hierbei beste Mutante war 7β-HSDH G39D R40F (DF). Danach erfolgte die Mutagenese an der Position R41 mit 7β-HSDH G39D R40F R41K (DFK) als beste Mutante und zum Schluss an der Position K44 mit 7β-HSDH G39D R40F R41K K44G (DFKG) als beste Mutante.

***Beispiel C.2: Enzymkinetische Untersuchung der 7β-HSDH-Mutanten mit NADH Spezifität***

Die Bewertung der erstellten Mutanten erfolgt mittels enzymkinetischer Untersuchungen. Für die Untersuchung der DHCA-Kinetik wurden 0,5 mM NADH als Kofaktor eingesetzt (Figur 9), während für die Untersuchung der NADH-Kinetik 10 mM DHCA als Substrat eingesetzt wurde (Figur 10). Aus den Auftragungen der DHCA-Kinetiken lassen sich für die 7β-HSDH Mutanten die charakteristischen Kurvenverläufe der Michaelis-Menten-Kinetik erkennen, dabei wurde keine Substratinhibition beobachtet. Dementsprechend wurde das klassische Michaelis-Menten-Modell (Gleichung 1) für die Auswertung der kinetischen Parameter verwendet. Die NADH-Kinetiken zeigen hingegen einen linearen Verlauf und lassen keine Sättigung bei der Kofaktorkonzentration erkennen, deshalb konnten hierfür keine kinetischen Parameter bestimmt werden.

**Gleichung 1: Michaelis-Menten-Gleichung.**

$$EA_X = v_{\max} \cdot \frac{c_s}{K_m + c_s}$$

| | |
|---|---|
| $EA_X$: | spezifische Enzymaktivität, U $mg^{-1}$= μmol $min^{-1}$ $mg^{-1}$ |
| $v_{max}$: | maximale spezifische Enzymaktivität, U $mg^{-1}$= μmol $min^{-1}$ $mg^{-1}$ |
| $c_s$: | Substrat- bzw. Kofaktorkonzentration, mol $L^{-1}$ |
| $K_m$: | Halbsättigungskonzentration, mol $L^{-1}$ |

Die ermittelten kinetischen Parameter der neuen 7β-HSDH-Mutanten sind in **Tabelle 4** aufgeführt, zusätzlich enthält die Tabelle Vergleichswerte für die bereits berichteten Mutanten G39D und G39D R40I. Darin ist zu erkennen, dass die neuen Mutanten 7β-HSDH DFK und 7β-HSDH DFKG mit 5,91±0,23 U $mg^{-1}$ bzw. 5,72±0,19 U $mg^{-1}$ eine um 23 - 27 % höhere maximale spezifische Enzymaktivität zeigen als die bislang aktivste Mutante G39D R40I.

**Tabelle 4:** Enzymkinetische Parameter der neuen 7β-HSDH-Mutanten DF, DFK und DFKG mit NADH als Kofaktor. Als Referenz sind die zuvor hergestellten Mutanten G39D und G39D R40I angegeben.

| | $K_{m,\ DHCA}$, μM | $v_{max}$, U $mg^{-1}$ |
|---|---|---|
| **G39D** | 660 ±120 | 2,90±0,16 |
| **G39D R40I** | 920 ±170 | 4,64±0,27 |
| **DF** | 420 ±60 | 3,70±0,13 |
| **DFK** | 290 ± 50 | 5,91±0,23 |
| **DFKG** | 380 ± 50 | 5,72±0,19 |

***Beispiel C.3: Einstufige Biotransformation mit NADH-abhängigen 7β-HSDH-Mutanten***

Zum Vergleich der NADH-abhängigen 7β-HSDHs wurden einstufige Biotransformationen von 50 mM DHCA zu 3,12-Diketo-UDCA im 2 mL-Maßstab im Dreifachansatz durchgeführt. Dabei wurden aufgereinigte Enzyme eingesetzt und als Kofaktor lediglich NAD zugegeben. Zur Kofaktorregenerierung wurde eine GDH verwendet. Von den untersuchten 7β-HSDH-(Wildtyp (WT), Mutanten: D, DF, DFK und DKFG) wurden jeweils 0,2 mg $mL^{-1}$ Enzym eingesetzt. Die restlichen Reaktionsbedingungen waren: 10 U $mL^{-1}$ GDH, 0,5 mM NAD, 50 mM DHCA, 200 mM Glucose, 500 mM Kaliumphosphat, pH 8,0. Die Reaktionen wurden im Dreifachansatz in geschüttelten DeepWell-Platten bei 30 °C als Satzverfahren ohne pH-Kontrolle im stark gepufferten System durchgeführt.

Die Ergebnisse der Biotransformation sind in Figur 12 gezeigt. Mit sämtlichen NADH-abhängigen Mutanten konnte mehr 3,12-Diketo-UDCA gebildet werden als mit dem Wildtyp-Enzym. Mit der Mutante DFKG konnte nach 16 h ein vollständiger Umsatz von DHCA zu 3,12-Diketo-UDCA erzielt werden.

***Beispiel C.4 Zweistufige Biotransformation mit NADH-abhängigen 7β-HSDH-Mutanten***

Weiterhin wurden mit den NADH-abhängigen 7β-HSDH-Mutanten DFK und DFKG zweistufige Biotransformationen von 50 mM DHCA zu 12-Keto-UDCA im 2 mL-Maßstab im Dreifachansatz durchgeführt.

Dabei wurden aufgereinigte Enzyme eingesetzt und als Kofaktor lediglich NAD zugegeben. Neben der 7β-HSDH kamen eine 3α-HSDH aus *Comomonas* testosteroni und eine GDH zur Kofaktorregenerierung zum Einsatz. Von den untersuchten 7β-HSDH-Mutanten wurden jeweils 0,2 mg $mL^{-1}$ Enzym eingesetzt. Die restlichen Reaktionsbedingungen waren: 1 U $mL^{-1}$ 3α-HSDH, 10 U $mL^{-1}$ GDH, 0,5 mM NAD, 50 mM DHCA, 200 mM Glucose, 500 mM Kaliumphosphat, pH 8,0. Die Reaktionen wurden im Dreifachansatz in geschüttelten DeepWell-Platten bei 30 °C als Satzverfahren ohne pH-Kontrolle im stark gepufferten System durchgeführt.

Die Reaktionsverläufe der jeweiligen Ansätze sind in Figur 13 gezeigt. Mit beiden 7β-HSDH-Mutanten konnten ohne Zusatz von NADP vollständige Umsetzungen von DHCA zu 12-Keto-UDCA erzielt werden.

**Tabelle 5:** Zuordnung von SEQ ID NOs:

| SEQ ID NO: | Beschreibung | Typ |
|---|---|---|
| 1 | 7β-HSDH *(C. aerofaciens)* | NS |
| 2 | 7β-HSDH*(C. aerofaciens)* | AS |
| 3 | 3α-HSDH *(C. testosteroni)* | NS |
| 4 | 3α-HSDH *(C. testosteroni)* | AS |
| 5 | 7α-HSDH | NS |
| 6 | 7α-HSDH | AS |
| 7 | GDH, *(B. subtilis)* | NS |
| 8 | GDH (B. subtilis) | AS |
| 9 | 7β-HSDH G39D | AS |

(fortgesetzt)

| SEQ ID NO: | Beschreibung | Typ |
|---|---|---|
| 10 | 7β-HSDH G39D R401 | AS |
| 11 | 7β-HSDH G39D R40F | AS |
| 12 | 7β-HSDH G39D R40F R41K | AS |
| 13 | 7β-HSDH G39D R40F R41K K44G | AS |
| 14 | PCR Primer | NS |
| 15 | PCR Primer | NS |
| 16 | PCR Primer | NS |
| 17 | PCR Primer | NS |
| 18 | Plasmid p7(A)T3rG-A | NS |
| 19 | Plasmid p7(A)T3rG-K | NS |
| 20 | Plasmid p7(A)T3TG-A | NS |
| 21 | Plasmid p7(A)T3TG-K | NS |
| AS = Aminosäuresequenz<br>NS = Nukleinsäuresequenz | | |

Auf die Offenbarung der hierin erwähnten Druckschriften wird ausdrücklich Bezug genommen.

## Patentansprüche

**1.** 7β-Hydroxysteroiddehydrogenase (7β-HSDH), welche wenigstens die stereospezifische enzymatische Reduktion eines 7-Ketosteroids zum korrespondierenden 7-Hydroxysteroid katalysiert, wobei das Enzym je eine Mutation in den Positionen G39 und R40 von SEQ ID NO:2 oder in einer davon abgeleiteten Aminosäuresequenz mit wenigstens 80% Sequenzidentität zu SEQ ID NO:2 aufweist, wobei das Enzym die Doppelmutation G39D/R40F umfasst.

**2.** 7β-Hydroxysteroiddehydrogenase (7β-HSDH), welche wenigstens die stereospezifische enzymatische Reduktion eines 7-Ketosteroids zum korrespondierenden 7-Hydroxysteroid katalysiert, wobei das Enzym je eine Mutation in den Positionen G39, R40 und R41, sowie gegebenenfalls in der Position K44 von SEQ ID NO:2 oder in einer davon abgeleiteten Aminosäuresequenz mit wenigstens 80% Sequenzidentität zu SEQ ID NO:2 aufweist, wobei das Enzym umfasst:

a) die Dreifachmutation G39D/R40F/R41$X_1$, wobei $X_1$ für einen beliebigen anderen Aminosäurerest steht; oder
b) die Vierfachmutation G39D/R40F/R41$X_1$/K44$X_2$, wobei $X_1$ für einen beliebigen anderen Aminosäurerest und $X_2$ für einen beliebigen anderen Aminosäurerest steht.

**3.** 7β-HSDH nach Anspruch 2, umfassend die Dreifachmutation G39D/R40F/R41K.

**4.** 7β-HSDH nach Anspruch 2, umfassend die Vierfachmutation G39D/R40F/R41K/K44G

**5.** Nukleotidsequenz kodierend für eine 7β-HSDH nach einem der Ansprüche 1 bis 4.

**6.** Expressionskassette, umfassend unter der Kontrolle wenigstens einer regulativer Sequenz wenigstens eine Nukleotidsequenz nach Anspruch 5.

**7.** Expressionsvektor, umfassend wenigstens eine Expressionskassette nach Anspruch 6.

**8.** Rekombinanter Mikroorganismus, der wenigstens eine Nukleotidsequenz gemäß Anspruch 5 oder wenigstens eine Expressionskassette nach Anspruch 6 oder wenigstens einen Expressionsvektor nach Anspruch 7 trägt.

**9.** Rekombinanter Mikroorganismus nach Anspruch 8, der zusätzlich die kodierende Sequenz für wenigstens ein weiteres Enzym, ausgewählt unter Hydroxysteroiddehydrogenasen (HSDH) und zur Cofaktorregenerierung geeignete Dehydrogenasen, trägt.

**10.** Rekombinanter Mikroorganismus nach Anspruch 9, wobei
die weitere HSDH ausgewählt ist unter 3a-HSDHs; und
die Dehydrogenase ausgewählt ist unter NADH-regenerierenden Enzymen, wie NADH Dehydrogenasen, Alkoholdehydrogenasen (ADH), und NADH regenerierenden Formiatdehydrogenasen (FDH), sowie Glucosedehydrogenase (GDH), Glucose-6-Phosphat-Dehydrogenase (G-6-PDH), oder Phosphit-Dehydrogenasen (PtDH), sowie NADH regenerierenden Glucosedehydrogenasen (GDH).

**11.** Rekombinanter Mikroorganismus nach einem der Ansprüche 8 bis 10 welcher ein 7α-HSDH knock-out Stamm ist.

**12.** Verfahren zur Herstellung von Ursodesoxycholsäure (UDCS) der Formel (1)

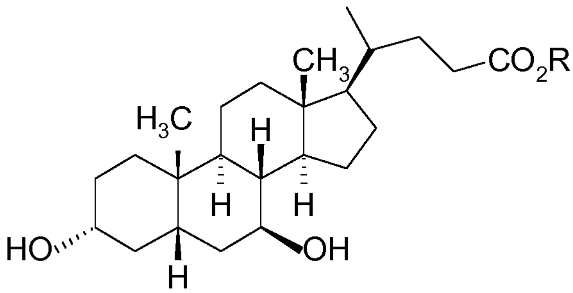

(1)

worin
R für Alkyl, H, ein Alkalimetallion oder $N(R^3)_4^+$steht, worin die Reste $R^3$ gleich oder verschieden sind und für H oder Alkyl stehen, oder die Gruppe $-CO_2R$ durch die Säureamid-Gruppe $-CONR^1R^2$, ersetzt ist, worin $R^1$ und $R^2$ unabhängig voneinander für einen Alkylrest stehen;
wobei man

a) gegebenenfalls eine Cholsäure (CS) der Formel (2)

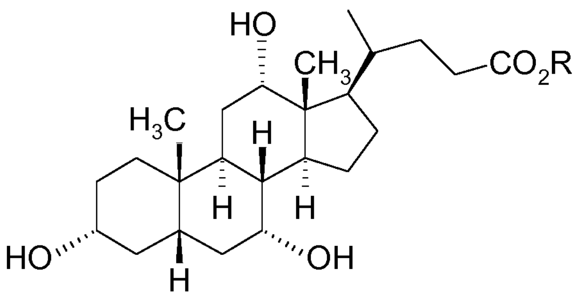

(2)

worin R die oben angegebenen Bedeutungen besitzt oder die Gruppe $-CO_2R$ durch die Säureamid-Gruppe

$-CONR^1R^2$, wie oben definiert ersetzt ist,
zur Dehydrocholsäure (DHCS) der Formel (3)

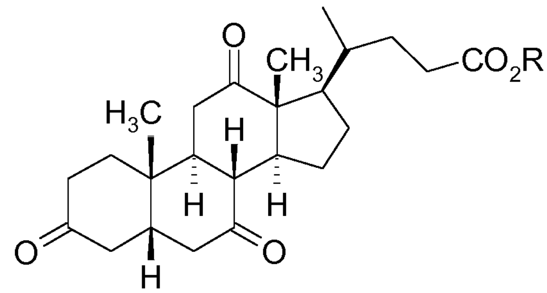

(3)

worin R die oben angegebenen Bedeutungen besitzt, oder die Gruppe $-CO_2R$ durch die Säureamid-Gruppe $-CONR^1R^2$, wie oben definiert ersetzt ist, oxidiert;

b) DHCS in Gegenwart wenigstens einer 7β-HSDH-Mutante gemäß der Definition in einem der Ansprüche 1 bis 4 und in Gegenwart wenigstens einer 3α-HSDH zur korrespondierenden 12-Keto-ursodesoxycholsäure (12-Keto UDCS) der Formel (5)

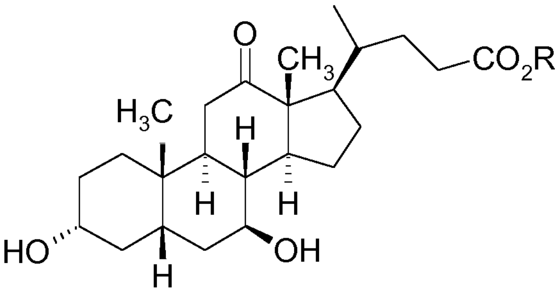

(5)

worin R die oben angegebenen Bedeutungen besitzt, oder die Gruppe $-CO_2R$ durch die Säureamid-Gruppe $-CONR^1R^2$, wie oben definiert ersetzt ist, reduziert und anschließend

c) 12-Keto-UDCS der Formel (5) chemisch zu UDCS reduziert; und

d) das Reaktionsprodukt gegebenenfalls weiter aufreinigt.

**13.** Verfahren zur Herstellung von UDCS der Formel (1)

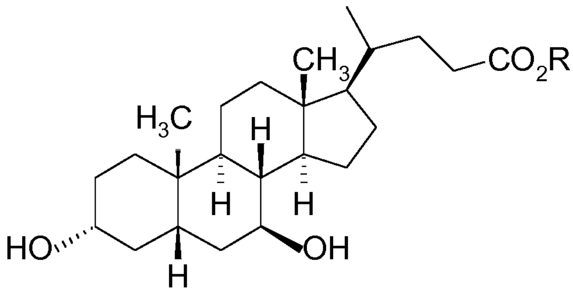

(1)

worin

R für Alkyl, $NR^1R^2$, H, ein Alkalimetallion oder $N(R^3)_4^+$steht, worin die Reste $R^3$ gleich oder verschieden sind und für H oder Alkyl stehen oder die Gruppe $-CO_2R$ durch die Säureamid-Gruppe $-CONR^1R^2$, wie oben definiert ersetzt ist wobei man

a) gegebenenfalls eine CS der Formel (2)

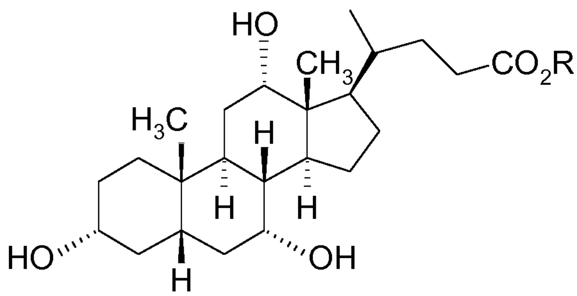

(2)

worin R die oben angegebenen Bedeutungen besitzt, oder die Gruppe $-CO_2R$ durch die Säureamid-Gruppe $-CONR^1R^2$, wie oben definiert ersetzt ist, zur DHCS der Formel (3)

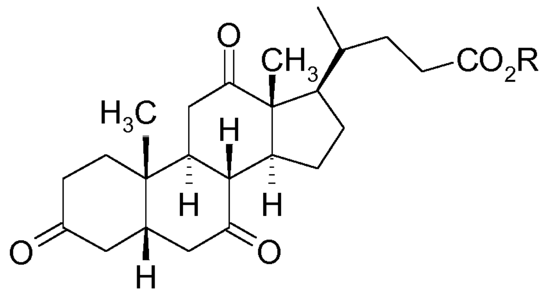

(3)

worin R die oben angegebenen Bedeutungen besitzt, oder die Gruppe $-CO_2R$ durch die Säureamid-Gruppe $-CONR^1R^2$wie oben definiert ersetzt ist, oxidiert;
b) DHCS in Gegenwart wenigstens einer 7ß-HSDH und in Gegenwart wenigstens einer 3α-HSDH zur korrespondierenden 12-Keto UDCS der Formel (5)

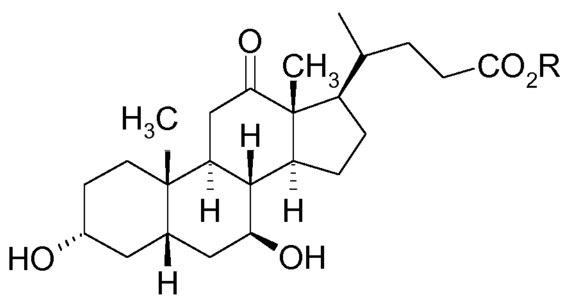

(5)

worin R die oben angegebenen Bedeutungen besitzt, oder die Gruppe $-CO_2R$ durch die Säureamid-Gruppe $-CONR^1R^2$wie oben definiert ersetzt ist, reduziert und anschließend
c) 12-Keto-UDCS der Formel (5) chemisch zu UDCS reduziert; und
d) das Reaktionsprodukt gegebenenfalls weiter aufreinigt;

wobei die Umsetzungen des Schritts b) in Gegenwart eines rekombinanten Mikroorganismus nach einem der Ansprüche 8 bis 11 erfolgen.

## Claims

1. 7β-Hydroxysteroid dehydrogenase (7β-HSDH) catalysing at least the stereospecific enzymatic reduction of a 7-ketosteroid to form the corresponding 7-hydroxysteroid, wherein the enzyme has a mutation in each case in positions G39 and R40 of SEQ ID NO:2 or in an amino acid sequence derived therefrom having at least 80% sequence identity to SEQ ID NO:2, wherein the enzyme comprises the double mutation G39D/R40F.

2. 7β-Hydroxysteroid dehydrogenase (7β-HSDH) catalysing at least the stereospecific enzymatic reduction of a 7-ketosteroid to form the corresponding 7-hydroxysteroid, wherein the enzyme has a mutation in each case in positions G39, R40 and R41 and optionally in position K44 of SEQ ID NO:2 or in an amino acid sequence derived therefrom having at least 80% sequence identity to SEQ ID NO:2, wherein the enzyme comprises:

a) the triple mutation G39D/R40F/R41$X_1$, where $X_1$ is any other amino acid residue; or
b) the quadruple mutation G39D/R40F/R41$X_1$/K44$X_2$, where $X_1$ is any other amino acid residue and $X_2$ is any other amino acid residue.

**3.** 7β-HSDH according to claim 2, comprising the triple mutation G39D/R40F/R41K.

**4.** 7β-HSDH according to claim 2, comprising the quadruple mutation G39D/R40F/R41K/K44G.

**5.** Nucleotide sequence encoding a 7β-HSDH according to any of claims 1 to 4.

**6.** Expression cassette comprising at least one nucleotide sequence according to claim 5 under the control of at least one regulatory sequence.

**7.** Expression vector comprising at least one expression cassette according to claim 6.

**8.** Recombinant microorganism bearing at least one nucleotide sequence according to claim 5 or at least one expression cassette according to claim 6 or at least one expression vector according to claim 7.

**9.** Recombinant microorganism according to claim 8, additionally bearing the coding sequence for at least one further enzyme selected from hydroxysteroid dehydrogenases (HSDHs) and dehydrogenases suitable for cofactor regeneration.

**10.** Recombinant microorganism according to claim 9, wherein
the further HSDH is selected from 3α-HSDHs; and
the dehydrogenase is selected from NADH-regenerating enzymes, such as NADH dehydrogenases, alcohol dehydrogenases (ADHs), and NADH-regenerating formate dehydrogenases (FDHs), and also glucose dehydrogenase (GDH), glucose-6-phosphate dehydrogenase (G-6-PDH), or phosphite dehydrogenases (PtDHs), and also NADH-regenerating glucose dehydrogenases (GDHs).

**11.** Recombinant microorganism according to any one of claims 8 to 10, which is a 7α-HSDH knock-out strain.

**12.** Process for preparing ursodeoxycholic acid (UDCA) of formula (1)

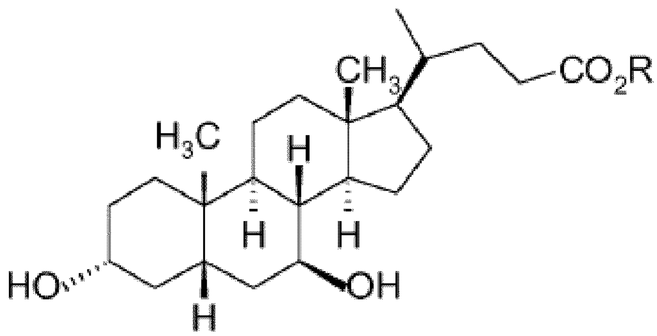

(1)

wherein
R is alkyl, H, an alkali metal ion or $N(R^3)_4^+$, wherein the residues $R^3$ are identical or different and are H or alkyl, or the group $-CO_2R$ has been replaced with the acid amide group $-CONR^1R^2$, wherein $R^1$ and $R^2$ independently of one another are an alkyl residue;
involving

a) optionally oxidizing a cholic acid (CA) of formula (2)

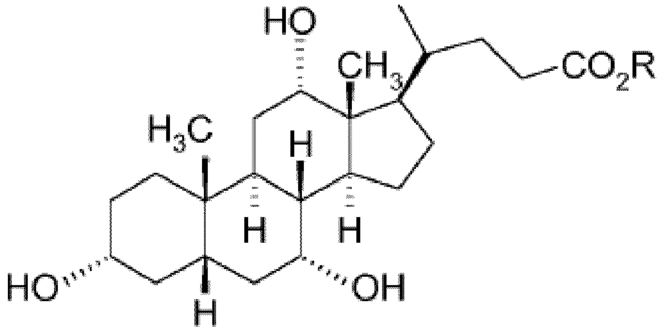

(2)

wherein R has the above-specified meanings or the group $-CO_2R$ has been replaced with the acid amide group $-CONR^1R^2$ as defined above,
to form dehydrocholic acid (DHCA) of formula (3)

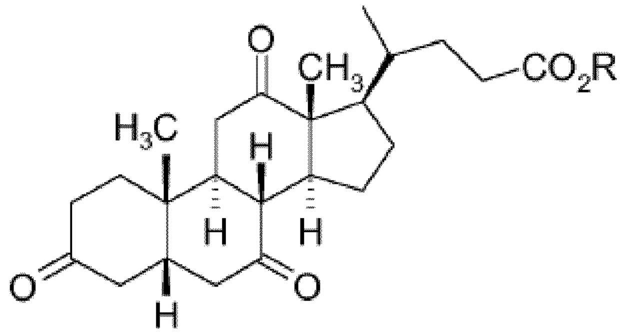

(3)

wherein R has the above-specified meanings or the group $-CO_2R$ has been replaced with the acid amide group $-CONR^1R^2$ as defined above;
b) reducing DHCA in the presence of at least one 7β-HSDH mutant as per the definition in any one of claims 1 to 4 and in the presence of at least one 3α-HSDH to form the corresponding 12-ketoursodeoxycholic acid (12-keto-UDCA) of formula (5)

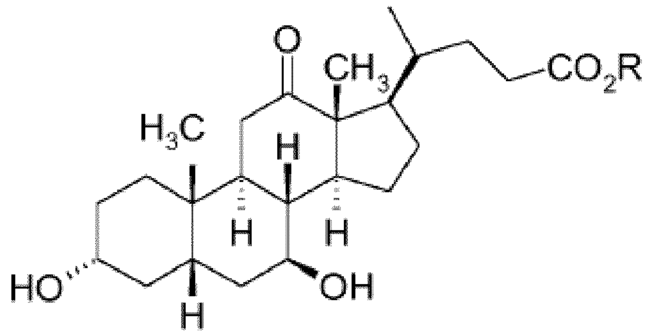

(5)

wherein R has the above-specified meanings or the group $-CO_2R$ has been replaced with the acid amide group $-CONR^1R^2$ as defined above, and subsequently
c) chemically reducing 12-keto-UDCA of formula (5) to form UDCA; and
d) optionally further purifying the reaction product.

**13.** Process for preparing UDCA of formula (1)

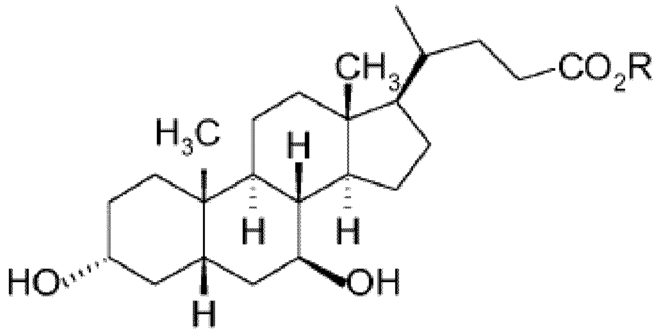

(1)

wherein
R is alkyl, $NR^1R^2$, H, an alkali metal ion or $N(R^3)_4^+$, wherein the residues $R^3$ are identical or different and are H or alkyl, or the group $-CO_2R$ has been replaced with the acid amide group $-CONR^1R^2$ as defined above;
involving

a) optionally oxidizing a CA of formula (2)

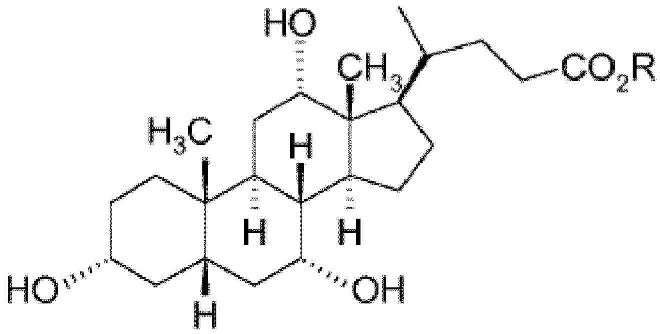

(2)

wherein R has the above-specified meanings or the group $-CO_2R$ has been replaced with the acid amide group $-CONR^1R^2$ as defined above, to form DHCA of formula (3)

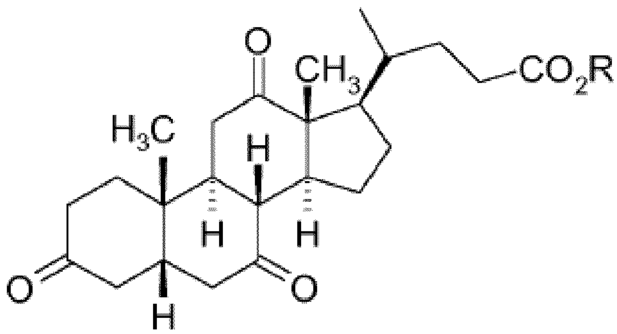

(3)

wherein R has the above-specified meanings or the group $-CO_2R$ has been replaced with the acid amide group $-CONR^1R^2$ as defined above;
b) reducing DHCA in the presence of at least one 7β-HSDH and in the presence of at least one 3α-HSDH to form the corresponding 12-keto-UDCA of formula (5)

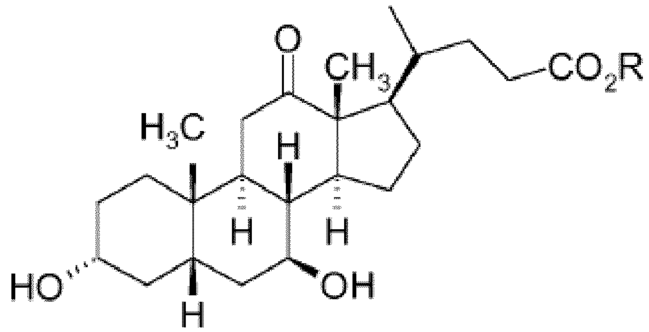

(5)

wherein R has the above-specified meanings or the group $-CO_2R$ has been replaced with the acid amide group $-CONR^1R^2$ as defined above, and subsequently

c) chemically reducing 12-keto-UDCA of formula (5) to form UDCA; and

d) optionally further purifying the reaction product;

wherein the reactions of step b) are carried out in the presence of a recombinant microorganism according to any one of claims 8 to 11.

**Revendications**

1. 7β-hydroxystéroïde-déshydrogénase (7β-HSDH), qui catalyse au moins la réduction enzymatique stéréospécifique d'un 7-cétostéroïde en le 7-hydroxystéroïde correspondant, l'enzyme présentant une mutation aux positions G39 et R40 de SEQ ID NO : 2 ou dans une séquence d'acides aminés dérivée de celle-ci présentant au moins 80 % d'identité de séquence avec SEQ ID NO: 2, l'enzyme comprenant la mutation double G39D/R40F.

2. 7β-hydroxystéroïde-déshydrogénase (7β-HSDH), qui catalyse au moins la réduction enzymatique stéréospécifique d'un 7-cétostéroïde en le 7-hydroxystéroïde correspondant, l'enzyme présentant une mutation aux positions G39, R40 et R41, ainsi qu'éventuellement à la position K44 de SEQ ID NO: 2 ou dans une séquence d'acides aminés dérivée de celle-ci présentant au moins 80 % d'identité de séquence avec SEQ ID NO: 2, l'enzyme comprenant:

   a) la mutation triple G39D/R40F/R41$X_1$, $X_1$ représentant un autre radical acide aminé quelconque; ou
   b) la mutation quadruple G39D/R40F/R41$X_1$/K44$X_2$, $X_1$ représentant un autre radical acide aminé quelconque et $X_2$ représentant un autre radical acide aminé quelconque.

3. 7β-HSDH selon la revendication 2, comprenant la mutation triple G39D/R40F/R41K.

4. 7β-HSDH selon la revendication 2, comprenant la mutation quadruple G39D/R40F/R41K/K44G.

5. Séquence nucléotidique codant pour une 7β-HSDH selon l'une quelconque des revendications 1 à 4.

6. Cassette d'expression, comprenant au moins une séquence nucléotidique selon la revendication 5 sous le contrôle d'au moins une séquence de régulation.

7. Vecteur d'expression, comprenant au moins une cassette d'expression selon la revendication 6.

8. Microorganisme recombinant, qui comprend au moins une séquence nucléotidique selon la revendication 5 ou au moins une cassette d'expression selon la revendication 6 ou au moins un vecteur d'expression selon la revendication 7.

9. Microorganisme recombinant selon la revendication 8, qui porte en outre la séquence codante pour au moins une enzyme supplémentaire, choisie parmi les hydroxystéroïde-déshydrogénases (HSDH) et les déshydrogénases appropriées pour la régénération du cofacteur.

10. Microorganisme recombinant selon la revendication 8, dans lequel l'HSDH supplémentaire est choisie parmi les 3α-HSDH; et

la déshydrogénase est choisie parmi les enzymes régénérant NADH, telles que les NADH-déshydrogénases, les alcool-déshydrogénases (ADH), et les formiate-déshydrogénases régénérant NADH (FDH), ainsi que la glucose-déshydrogénase (GDH), la glucose-6-phosphate-déshydrogénase (G-6-PDH) ou les phosphite-déshydrogénases (PtDH), ainsi que les glucose-déshydrogénases régénérant NADH (GDH).

**11.** Microorganisme recombinant selon l'une quelconque des revendications 8 à 10, qui est une souche de 7$\alpha$-HSDH knock-out.

**12.** Procédé de fabrication d'acide ursodésoxycholique (UDCS) de la formule (1) :

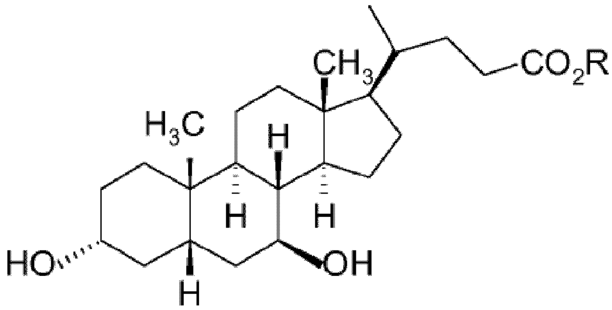

(1)

dans laquelle
R représente alkyle, H, un ion de métal alcalin ou $N(R^3)_4^+$, les résidus $R^3$ étant identiques ou différents, et représentant H ou alkyle, ou le groupe $-CO_2R$ est remplacé par le groupe amide d'acide $-CONR^1R^2$, $R^1$ et $R^2$ représentant indépendamment l'un de l'autre un résidu alkyle;
selon lequel

a) éventuellement un acide cholique (CS) de la formule (2):

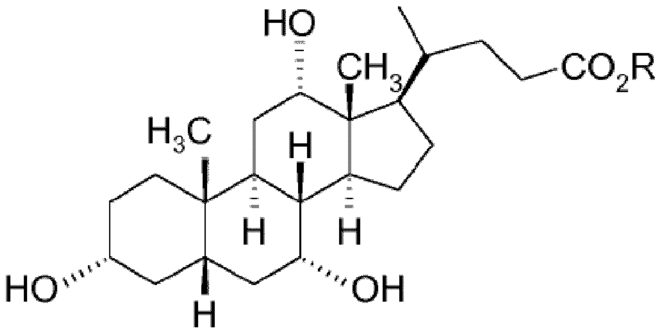

(2)

dans laquelle R a les significations indiquées précédemment, ou le groupe $-CO_2R$ est remplacé par le groupe amide d'acide $-CONR^1R^2$, tel que défini précédemment,
est oxydé en l'acide déshydrocholique (DHCS) de la formule (3):

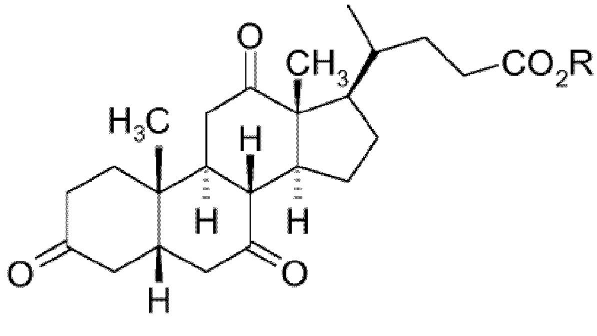

(3)

dans laquelle R a les significations indiquées précédemment, ou le groupe $-CO_2R$ est remplacé par le groupe

amide d'acide $-CONR^1R^2$, tel que défini précédemment ;

b) le DHCS est réduit en présence d'au moins un mutant de 7β-HSDH selon la définition dans l'une quelconque des revendications 1 à 4 et en présence d'au moins un 3α-HSDH pour former l'acide 12-céto-ursodésoxycholique correspondant (12-céto-UDCS) de la formule (5):

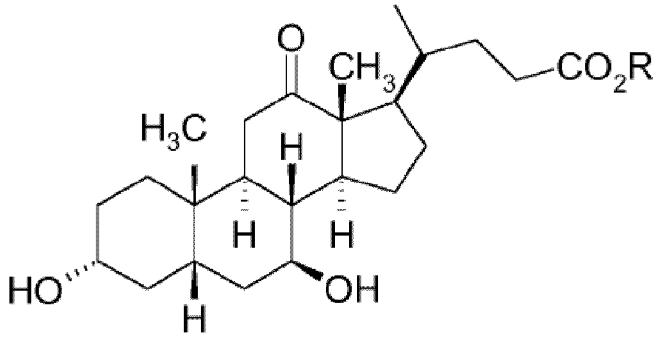

(5)

dans laquelle R a les significations indiquées précédemment, ou le groupe $-CO_2R$ est remplacé par le groupe amide d'acide $-CONR^1R^2$, tel que défini précédemment; puis

c) le 12-céto-UDCS de la formule (5) est réduit chimiquement en UDCS; et

d) le produit de réaction est éventuellement davantage purifié.

**13.** Procédé de fabrication d'UDCS de la formule (1) :

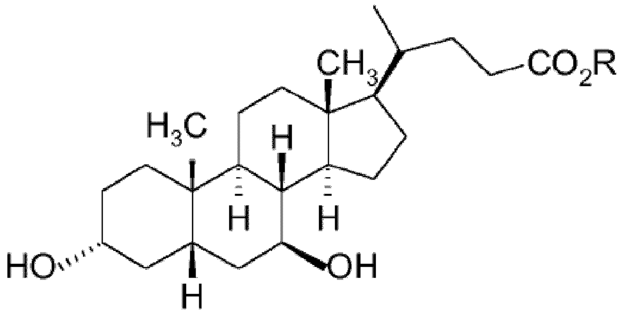

(1)

dans laquelle

R représente alkyle, $NR^1R^2$, H, un ion de métal alcalin ou $N(R^3)_4^+$, les résidus $R^3$ étant identiques ou différents, et représentant H ou alkyle, ou le groupe $-CO_2R$ est remplacé par le groupe amide d'acide $-CONR^1R^2$, tel que défini précédemment,

selon lequel

a) éventuellement un CS de la formule (2):

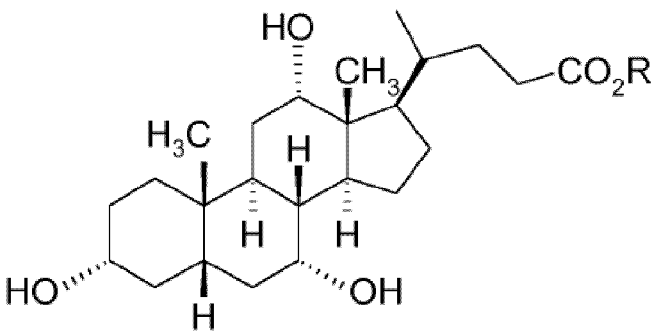

(2)

dans laquelle R a les significations indiquées précédemment, ou le groupe $-CO_2R$ est remplacé par le groupe amide d'acide $-CONR^1R^2$, tel que défini précédemment, est oxydé en DHCS de la formule (3):

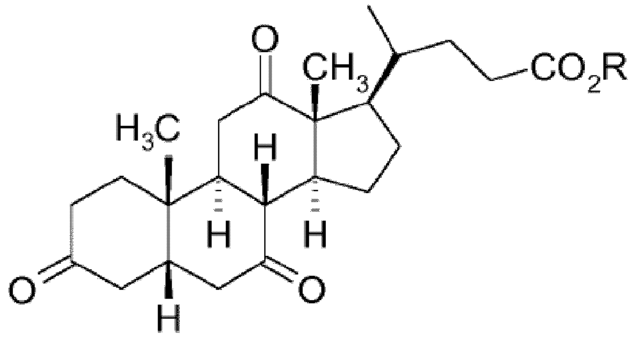

(3)

dans laquelle R a les significations indiquées précédemment, ou le groupe $-CO_2R$ est remplacé par le groupe amide d'acide $-CONR^1R^2$, tel que défini précédemment ;

b) le DHCS est réduit en présence d'au moins une 7β-HSDH et en présence d'au moins un 3α-HSDH pour former le 12-céto-UDCS correspondant de la formule (5):

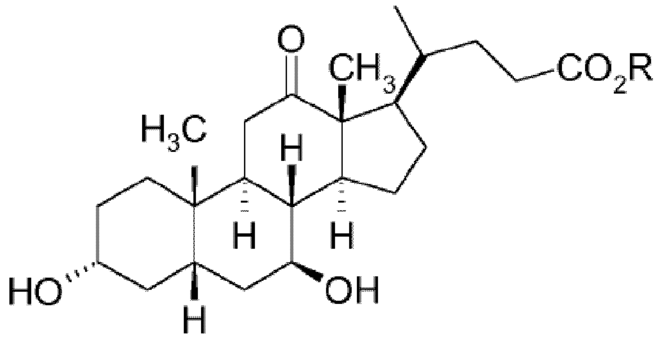

(5)

dans laquelle R a les significations indiquées précédemment, ou le groupe $-CO_2R$ est remplacé par le groupe amide d'acide $-CONR^1R^2$, tel que défini précédemment; puis

c) le 12-céto-UDCS de la formule (5) est réduit chimiquement en UDCS; et

d) le produit de réaction est éventuellement davantage purifié;

les réactions de l'étape b) ayant lieu en présence d'un microorganisme recombinant selon l'une quelconque des revendications 8 à 11.

7ß-HSDH aus *Collinsella aerofaciens*

Accession: ZP_01773061

```
  1 mnlrekygew glilgategv gkafcekiaa ggmnvvmvgr reeklnvlag eiretygvet
 61 kvvradfsqp gaaetvfaat egldmgfmsy vaclhsfgki qdtpwekhea minvnvvtfl
121 kcfhhymrif aaqdrgavin vssmtgisss pwngqygagk afilkmteav acecegtgvd
181 vevitlgttl tpsllsnlpg gpqgeavmki altpeecvde afeklgkels viagqrnkds
241 vhdwkanhte deyirymgsf yrd
```

**Fig. 1a**

Nukleinsäuresequenz codierend für 7ß-HSDH *aus Collinsella aerofaciens*

Accession NO: NZ_AAVN02000010, Region: 52005..52796

```
  1 atgaacctga gggagaagta cggtgagtgg ggcctgatcc tgggcgcgac cgagggcgtc
 61 ggcaaggcgt tctgcgagaa gatcgccgcc ggcggcatga acgtcgtcat ggtcggccgt
121 cgcgaggaga agctgaacgt gctcgcaggc gagatccgcg agacctacgg cgtggagacc
181 aaggtcgtgc gcgccgactt tagccagccc ggcgctgccg agaccgtctt cgccgcgacc
241 gagggcctgg acatgggctt catgagctac gtggcctgcc tgcacagctt cggtaagatc
301 caggacaccc cctgggagaa gcacgaggcc atgatcaacg tcaacgtcgt gaccttcctc
361 aagtgcttcc accactacat gcggatcttt gccgcccagg accgcggcgc cgtgatcaac
421 gtctcgtcga tgaccggcat cagctccagc ccctggaacg gccagtacgg cgcgggcaag
481 gccttcatcc tcaagatgac cgaggccgtg gcctgcgagt gcgagggcac cggcgtcgac
541 gtcgaggtca tcaccctcgg caccaccctc accccccagcc tgctgtccaa cctccccggc
601 ggcccgcagg gcgaggccgt catgaagatc gccctcaccc ccgaggagtg cgttgacgag
661 gcctttgaga agctgggtaa ggagctctcc gtcatcgccg gccagcgcaa caaggactcc
721 gtccacgact ggaaggcaaa ccacaccgag gacgagtaca tccgctacat ggggtcgttc
781 taccgcgact ag
```

**Fig.1b**

3α-HSDH

Quelle: *Comamonas testosteroni* ATCC 11996

```
  1 msiivisgca tgigaatrkv leaaghqivg idirdaevia dlstaegrkq aiadvlakcs
 61 kgmdglvlca glgpqtkvlg nvvsvnyfga telmdaflpa lkkghqpaav vissvasahl
121 afdknplala leageeakar aivehageqg gnlayagskn altvavrkra aawgeagvrl
181 ntiapgatet pllqaglqdp rygesiakfv ppmgrraeps emasviaflm spaasyvhga
241 qividggida vmrptqf
```

**Fig.1c**

Nukleinsäuresequenz codierend für 3α-HSDH

Quelle: *Comamonas testosteroni* ATCC 11996

Accession: AF092031, Region: 158..931

```
  1 atgtccatca tcgtgataag cggctgcgcc accggcattg gtgcggctac gcgcaaggtc
 61 ctggaggcgg ccggtcacca gatcgtaggc atcgatatac gcgatgcgga agtgattgcc
121 gatctctcga cggccgaagg tcgaaagcag gcgattgccg atgtactggc gaagtgcagc
181 aagggcatgg acggcctggt gctgtgcgcc ggcctgggac cgcagaccaa ggtgcttggc
241 aatgtggttt cggtcaatta ttttggcgcg accgagctga tggatgcctt tttgccagcg
301 ctgaaaaaag gccatcagcc cgcagccgtc gtcatctcgt ccgtggcttc cgcgcatctg
361 gcttttgaca agaacccact ggcgctggca ctggaagccg gcgaggaagc caaggcccgc
421 gccattgtcg aacatgcggg agagcagggc ggaaatctgg cctatgcggg cagcaagaat
481 gctttgacgg tggctgtgcg caaacgcgcc gccgcctggg gcgaggctgg cgtgcgcctg
541 aacaccatcg cccccggtgc aaccgagact cccttgctgc aggcgggcct gcaggacccg
601 cgctatggcg aatccattgc caagttcgtt cctcccatgg gccgccgtgc cgagccgtcc
661 gagatggcgt cggtcatcgc ctttttgatg agcccggccg caagctatgt gcatggcgcg
721 cagatcgtca ttgatggcgg cattgatgcg gtgatgcgcc cgacacagtt ctga
```

**Fig.1d**

Wildtyp:

MNLREKYGEWGLILGATEGVGKAFCEKIAAGGMNVVMVGRREEKLNVLAGEIR
ETYGVETKVVRADFSQPGAAETVFAATEGLDMGFMSYVACLHSFGKIQDTPWE
KHEAMINVNVVTFLKCFHHYMRIFAAQDRGAVINVSSMTGISSSPWNGQYGAG
KAFILKMTEAVACECEGTGVDVEVITLGTTLTPSLLSNLPGGPQGEAVMKIAL
TPEECVDEAFEKLGKELSVIAGQRNKDSVHDWKANHTEDEYIRYMGSFYRD*

G39D:

MNLREKYGEWGLILGATEGVGKAFCEKIAAGGMNVVMV**D**RREEKLNVLAGEIR
ETYGVETKVVRADFSQPGAAETVFAATEGLDMGFMSYVACLHSFGKIQDTPWE
KHEAMINVNVVTFLKCFHHYMRIFAAQDRGAVINVSSMTGISSSPWNGQYGAG
KAFILKMTEAVACECEGTGVDVEVITLGTTLTPSLLSNLPGGPQGEAVMKIAL
TPEECVDEAFEKLGKELSVIAGQRNKDSVHDWKANHTEDEYIRYMGSFYRD*

G39D R40F:

MNLREKYGEWGLILGATEGVGKAFCEKIAAGGMNVVMV**DF**REEKLNVLAGEIR
ETYGVETKVVRADFSQPGAAETVFAATEGLDMGFMSYVACLHSFGKIQDTPWE
KHEAMINVNVVTFLKCFHHYMRIFAAQDRGAVINVSSMTGISSSPWNGQYGAG
KAFILKMTEAVACECEGTGVDVEVITLGTTLTPSLLSNLPGGPQGEAVMKIAL
TPEECVDEAFEKLGKELSVIAGQRNKDSVHDWKANHTEDEYIRYMGSFYRD*

G39D R40F R41K:

MNLREKYGEWGLILGATEGVGKAFCEKIAAGGMNVVMV**DFK**EEKLNVLAGEIR
ETYGVETKVVRADFSQPGAAETVFAATEGLDMGFMSYVACLHSFGKIQDTPWE
KHEAMINVNVVTFLKCFHHYMRIFAAQDRGAVINVSSMTGISSSPWNGQYGAG
KAFILKMTEAVACECEGTGVDVEVITLGTTLTPSLLSNLPGGPQGEAVMKIAL
TPEECVDEAFEKLGKELSVIAGQRNKDSVHDWKANHTEDEYIRYMGSFYRD*

G39D R40F R41K K44G:

MNLREKYGEWGLILGATEGVGKAFCEKIAAGGMNVVMV**DFK**EE**G**LNVLAGEIR
ETYGVETKVVRADFSQPGAAETVFAATEGLDMGFMSYVACLHSFGKIQDTPWE
KHEAMINVNVVTFLKCFHHYMRIFAAQDRGAVINVSSMTGISSSPWNGQYGAG
KAFILKMTEAVACECEGTGVDVEVITLGTTLTPSLLSNLPGGPQGEAVMKIAL
TPEECVDEAFEKLGKELSVIAGQRNKDSVHDWKANHTEDEYIRYMGSFYRD*

**Fig.1e**

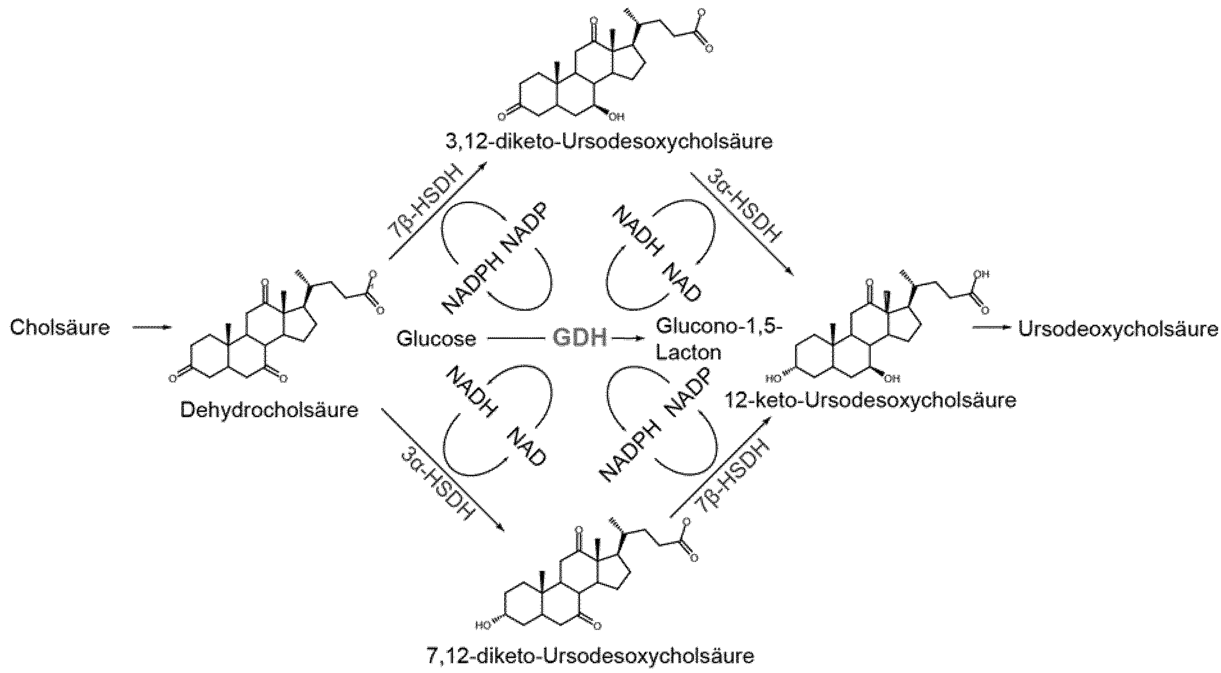


Cholsäure

Dehydrocholsäure

3,12-diketo-Ursodesoxycholsäure

12-keto-Ursodesoxycholsäure

Ursodeoxycholsäure

7,12-diketo-Ursodesoxycholsäure

**Fig. 2**

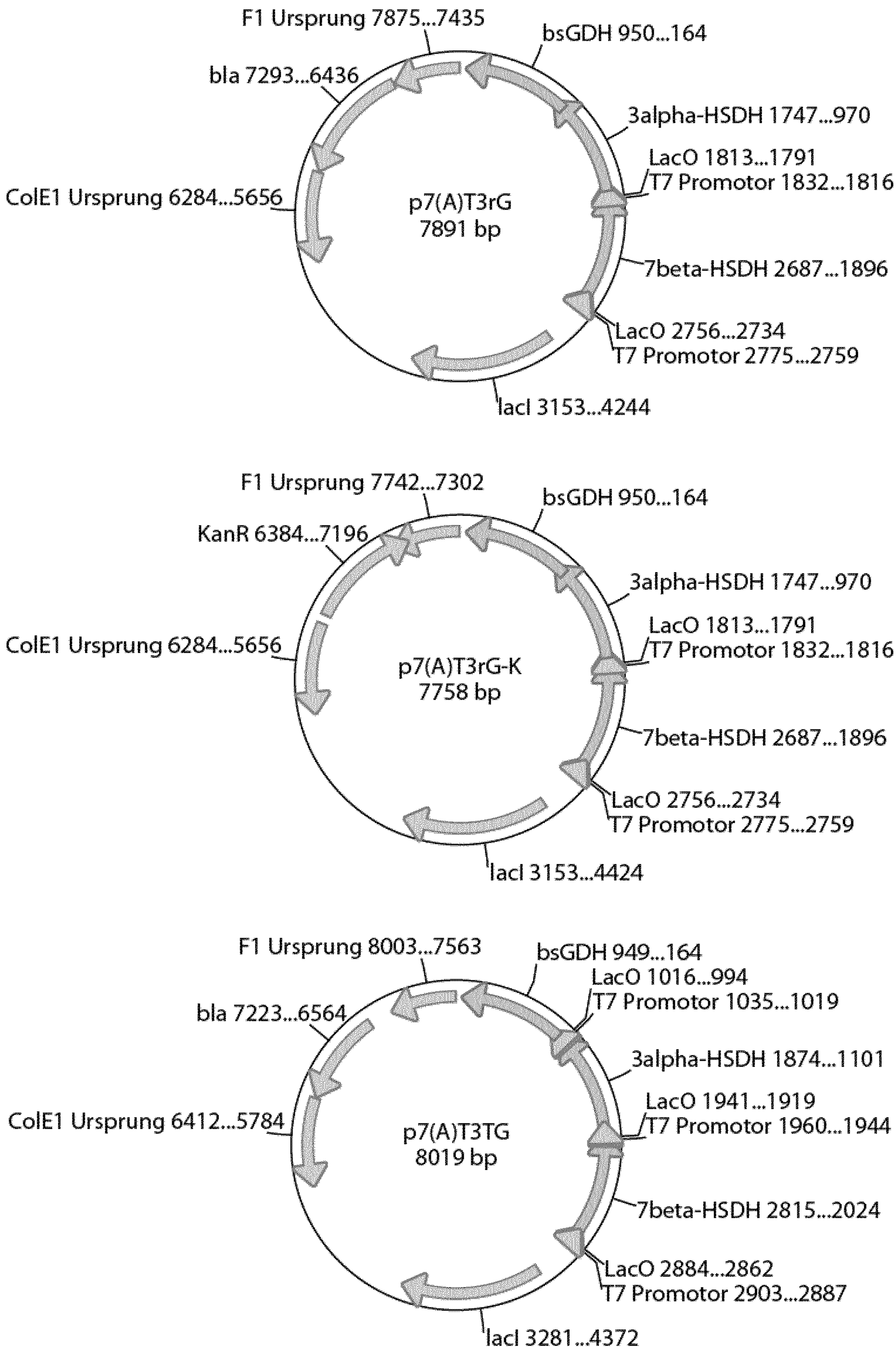
F1 Ursprung 7875...7435
bsGDH 950...164
bla 7293...6436
3alpha-HSDH 1747...970
LacO 1813...1791
T7 Promotor 1832...1816
ColE1 Ursprung 6284...5656
p7(A)T3rG
7891 bp
7beta-HSDH 2687...1896
LacO 2756...2734
T7 Promotor 2775...2759
lacI 3153...4244
F1 Ursprung 7742...7302
bsGDH 950...164
KanR 6384...7196
3alpha-HSDH 1747...970
LacO 1813...1791
T7 Promotor 1832...1816
ColE1 Ursprung 6284...5656
p7(A)T3rG-K
7758 bp
7beta-HSDH 2687...1896
LacO 2756...2734
T7 Promotor 2775...2759
lacI 3153...4424
F1 Ursprung 8003...7563
bsGDH 949...164
LacO 1016...994
T7 Promotor 1035...1019
bla 7223...6564
3alpha-HSDH 1874...1101
LacO 1941...1919
T7 Promotor 1960...1944
ColE1 Ursprung 6412...5784
p7(A)T3TG
8019 bp
7beta-HSDH 2815...2024
LacO 2884...2862
T7 Promotor 2903...2887
lacI 3281...4372

**Fig.3**

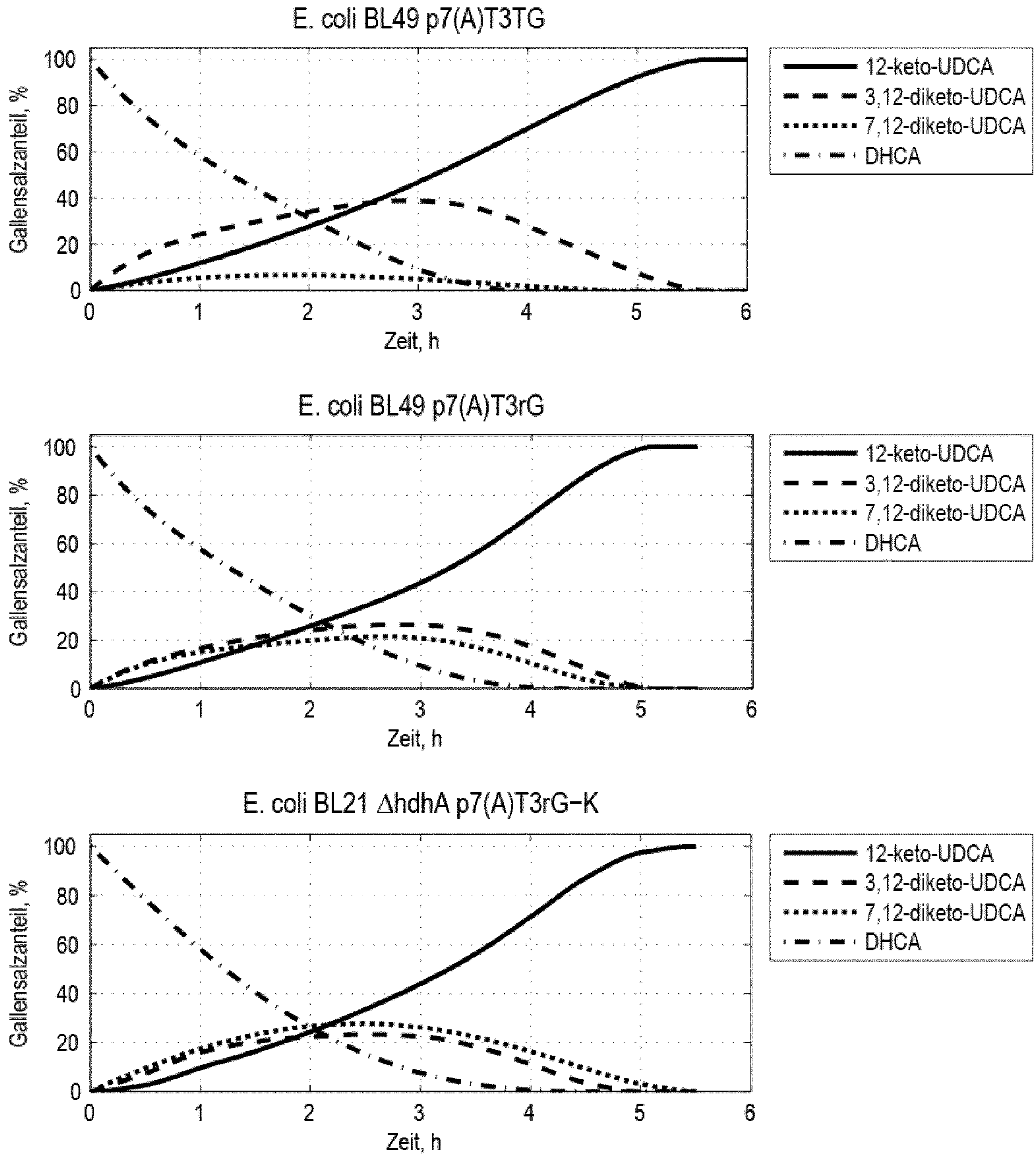
E. coli BL49 p7(A)T3TG
Gallensalzanteil, %
Zeit, h
12-keto-UDCA
3,12-diketo-UDCA
7,12-diketo-UDCA
DHCA
E. coli BL49 p7(A)T3rG
Gallensalzanteil, %
Zeit, h
12-keto-UDCA
3,12-diketo-UDCA
7,12-diketo-UDCA
DHCA
E. coli BL21 ΔhdhA p7(A)T3rG−K
Gallensalzanteil, %
Zeit, h
12-keto-UDCA
3,12-diketo-UDCA
7,12-diketo-UDCA
DHCA

**Fig. 4**

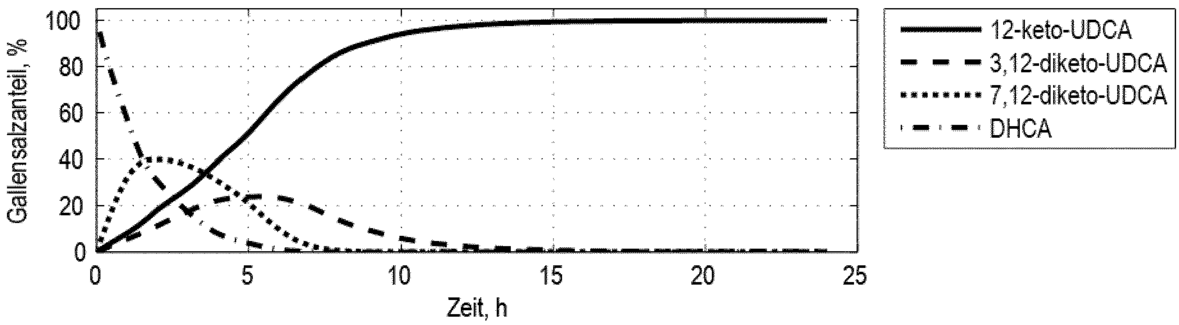


Fig. 5

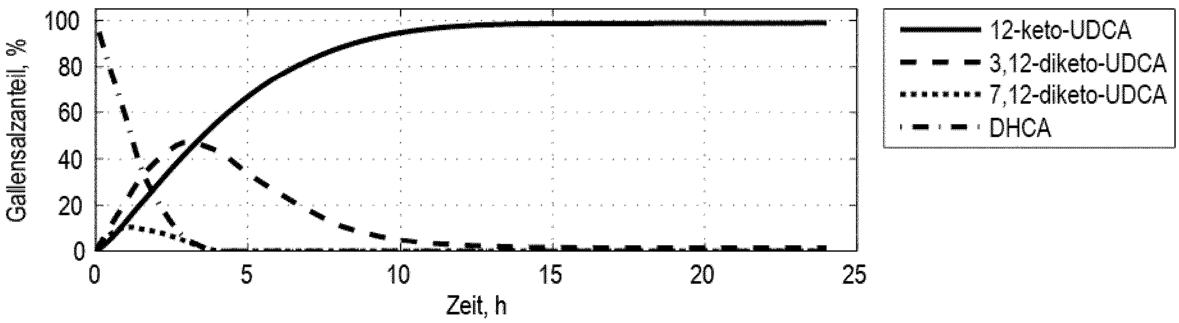


Fig. 6

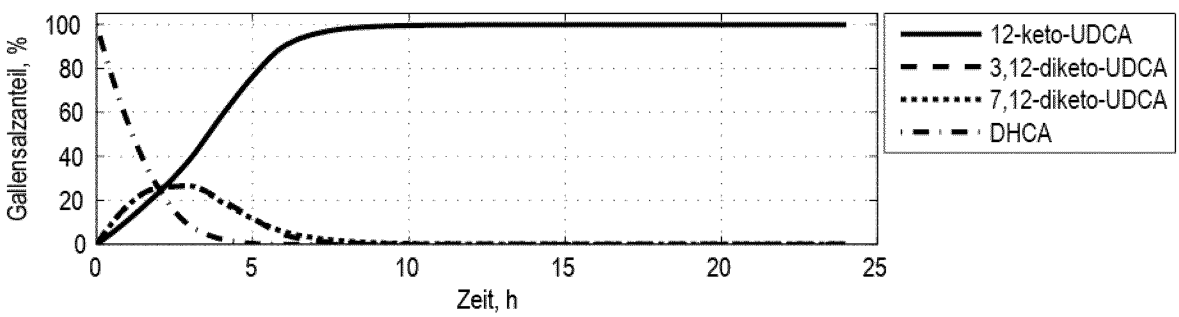


Fig. 7

```
7beta-HSDH Wildtyp        MNLREKYGEW GLILGATEGV GKAFCEKIAA GGMNVVMVGR REEKLNVLAG
7beta-HSDH G39D           MNLREKYGEW GLILGATEGV GKAFCEKIAA GGMNVVMVDR REEKLNVLAG
7beta-HSDH G39D R40I      MNLREKYGEW GLILGATEGV GKAFCEKIAA GGMNVVMVDI REEKLNVLAG
7beta-HSDH DF             MNLREKYGEW GLILGATEGV GKAFCEKIAA GGMNVVMVDF REEKLNVLAG
7beta-HSDH DFK            MNLREKYGEW GLILGATEGV GKAFCEKIAA GGMNVVMVDF KEEKLNVLAG
7beta-HSDH DFKG           MNLREKYGEW GLILGATEGV GKAFCEKIAA GGMNVVMVDF KEEGLNVLAG


7beta-HSDH Wildtyp        EIRETYGVET KVVRADFSQP GAAETVFAAT EGLDMGFMSY VACLHSFGKI
7beta-HSDH G39D           EIRETYGVET KVVRADFSQP GAAETVFAAT EGLDMGFMSY VACLHSFGKI
7beta-HSDH G39D R40I      EIRETYGVET KVVRADFSQP GAAETVFAAT EGLDMGFMSY VACLHSFGKI
7beta-HSDH DF             EIRETYGVET KVVRADFSQP GAAETVFAAT EGLDMGFMSY VACLHSFGKI
7beta-HSDH DFK            EIRETYGVET KVVRADFSQP GAAETVFAAT EGLDMGFMSY VACLHSFGKI
7beta-HSDH DFKG           EIRETYGVET KVVRADFSQP GAAETVFAAT EGLDMGFMSY VACLHSFGKI

7beta-HSDH Wildtyp        QDTPWEKHEA MINVNVVTFL KCFHHYMRIF AAQDRGAVIN VSSMTGISSS
7beta-HSDH G39D           QDTPWEKHEA MINVNVVTFL KCFHHYMRIF AAQDRGAVIN VSSMTGISSS
7beta-HSDH G39D R40I      QDTPWEKHEA MINVNVVTFL KCFHHYMRIF AAQDRGAVIN VSSMTGISSS
7beta-HSDH DF             QDTPWEKHEA MINVNVVTFL KCFHHYMRIF AAQDRGAVIN VSSMTGISSS
7beta-HSDH DFK            QDTPWEKHEA MINVNVVTFL KCFHHYMRIF AAQDRGAVIN VSSMTGISSS
7beta-HSDH DFKG           QDTPWEKHEA MINVNVVTFL KCFHHYMRIF AAQDRGAVIN VSSMTGISSS

7beta-HSDH Wildtyp        PWNGQYGAGK AFILKMTEAV ACECEGTGVD VEVITLGTTL TPSLLSNLPG
7beta-HSDH G39D           PWNGQYGAGK AFILKMTEAV ACECEGTGVD VEVITLGTTL TPSLLSNLPG
7beta-HSDH G39D R40I      PWNGQYGAGK AFILKMTEAV ACECEGTGVD VEVITLGTTL TPSLLSNLPG
7beta-HSDH DF             PWNGQYGAGK AFILKMTEAV ACECEGTGVD VEVITLGTTL TPSLLSNLPG
7beta-HSDH DFK            PWNGQYGAGK AFILKMTEAV ACECEGTGVD VEVITLGTTL TPSLLSNLPG
7beta-HSDH DFK            PWNGQYGAGK AFILKMTEAV ACECEGTGVD VEVITLGTTL TPSLLSNLPG

7beta-HSDH Wildtyp        GPQGEAVMKI ALTPEECVDE AFEKLGKELS VIAGQRNKDS VHDWKANHTE
7beta-HSDH G39D           GPQGEAVMKI ALTPEECVDE AFEKLGKELS VIAGQRNKDS VHDWKANHTE
7beta-HSDH G39D R40I      GPQGEAVMKI ALTPEECVDE AFEKLGKELS VIAGQRNKDS VHDWKANHTE
7beta-HSDH DF             GPQGEAVMKI ALTPEECVDE AFEKLGKELS VIAGQRNKDS VHDWKANHTE
7beta-HSDH DFK            GPQGEAVMKI ALTPEECVDE AFEKLGKELS VIAGQRNKDS VHDWKANHTE
7beta-HSDH DFKG           GPQGEAVMKI ALTPEECVDE AFEKLGKELS VIAGQRNKDS VHDWKANHTE

7beta-HSDH Wildtyp        DEYIRYMGSF YRD
7beta-HSDH G39D           DEYIRYMGSF YRD
7beta-HSDH G39D R40I      DEYIRYMGSF YRD
7beta-HSDH DF             DEYIRYMGSF YRD
7beta-HSDH DFK            DEYIRYMGSF YRD
7beta-HSDH DFKG           DEYIRYMGSF YRD
```

**Fig. 8**

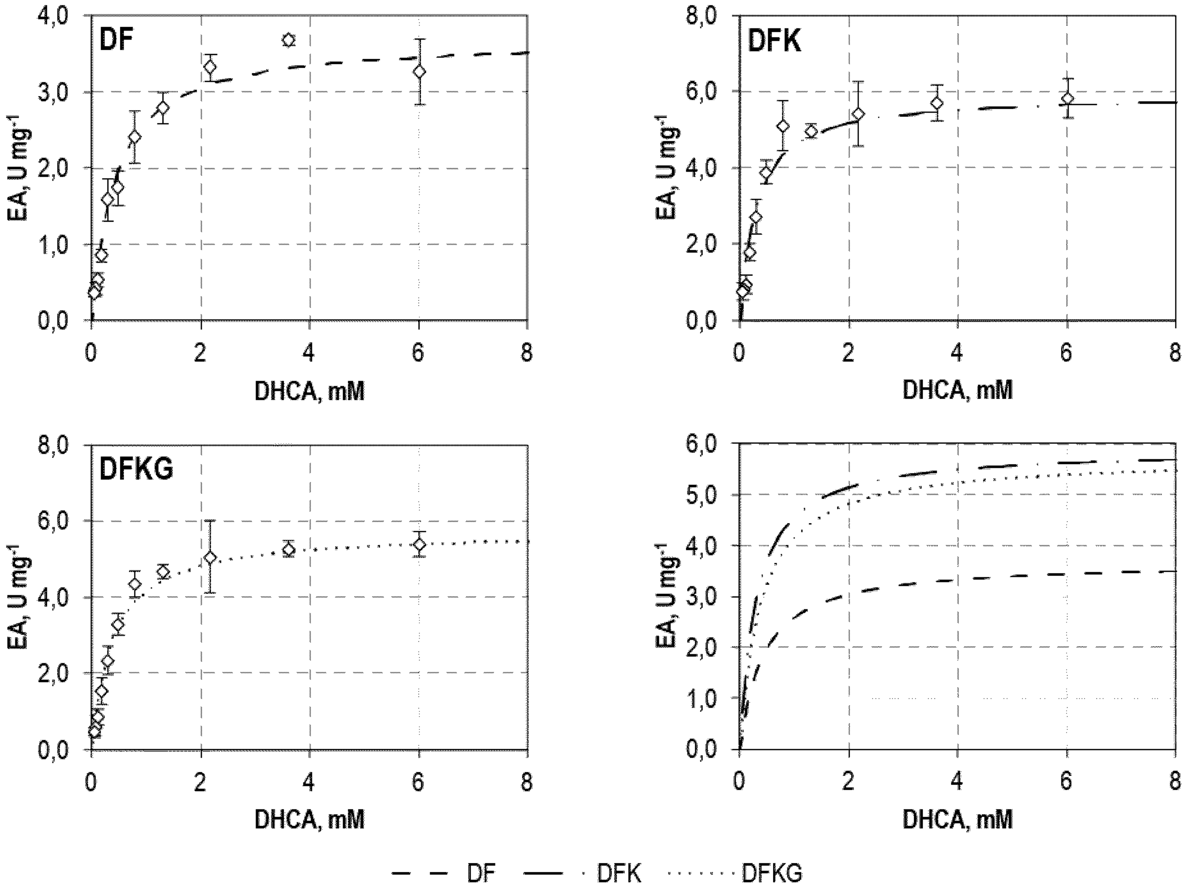


Fig. 9

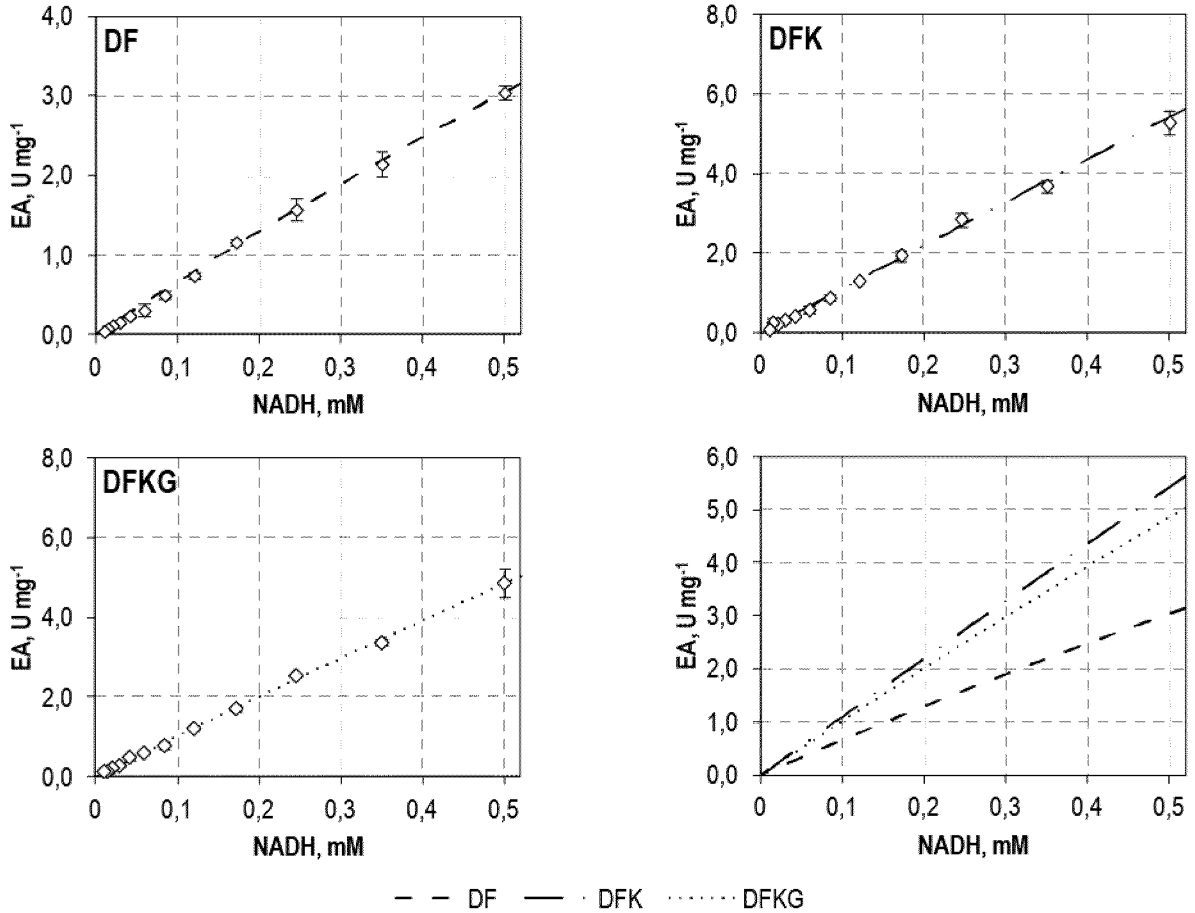
DF
DFK
DFKG
EA, U mg-1
NADH, mM
4,0
3,0
2,0
1,0
0,0
8,0
6,0
5,0
0
0,1
0,2
0,3
0,4
0,5
DF
DFK
DFKG

Fig. 10

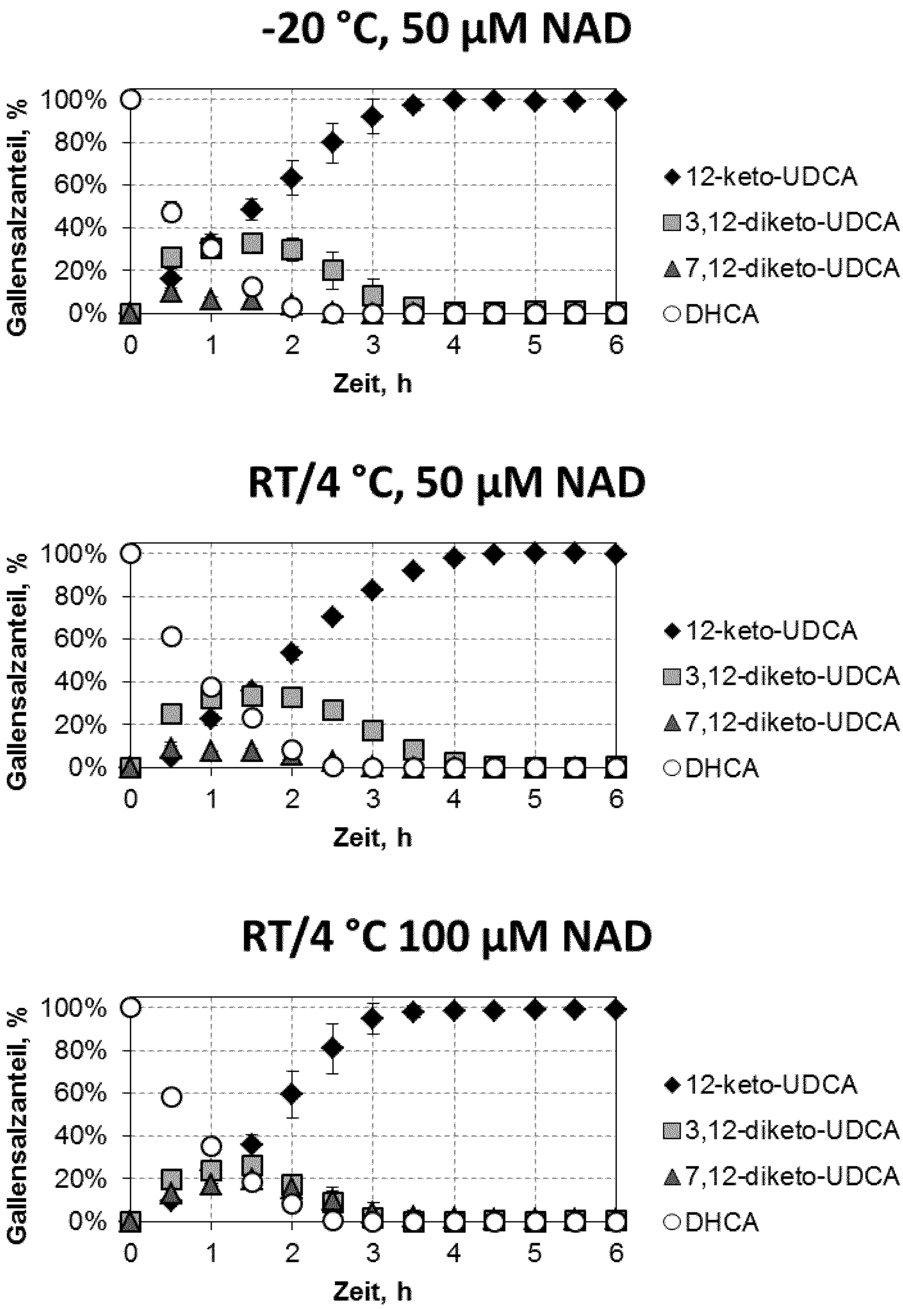
-20 °C, 50 µM NAD
Gallensalzanteil, %
100%
80%
60%
40%
20%
0%
0
1
2
3
4
5
6
Zeit, h
12-keto-UDCA
3,12-diketo-UDCA
7,12-diketo-UDCA
DHCA
RT/4 °C, 50 µM NAD
Gallensalzanteil, %
Zeit, h
12-keto-UDCA
3,12-diketo-UDCA
7,12-diketo-UDCA
DHCA
RT/4 °C 100 µM NAD
Gallensalzanteil, %
Zeit, h
12-keto-UDCA
3,12-diketo-UDCA
7,12-diketo-UDCA
DHCA

Fig. 11

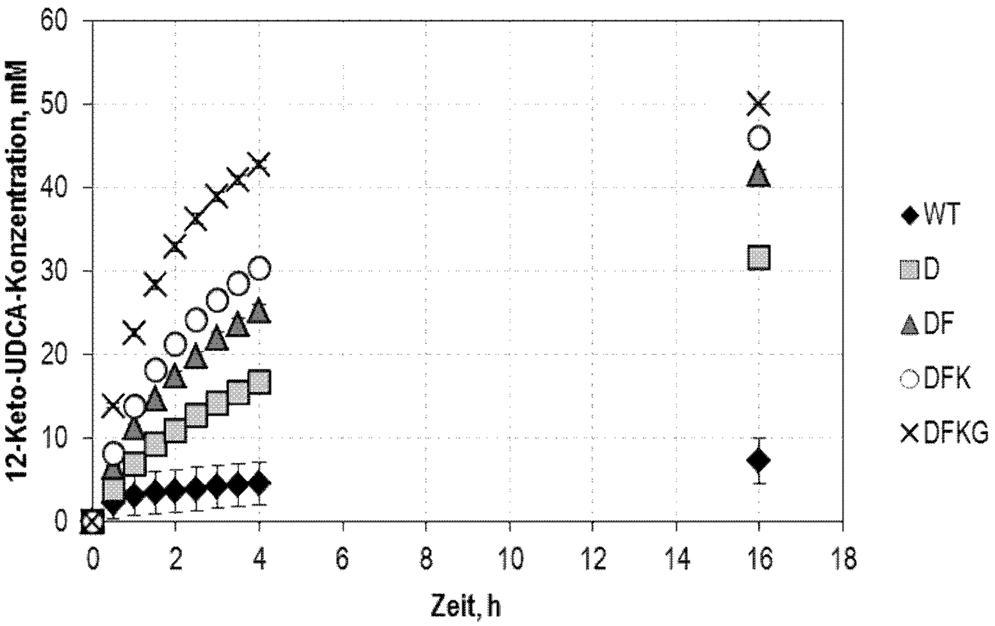
60
50
40
30
20
10
0
12-Keto-UDCA-Konzentration, mM
0
2
4
6
8
10
12
14
16
18
Zeit, h
WT
D
DF
DFK
DFKG

Fig. 12

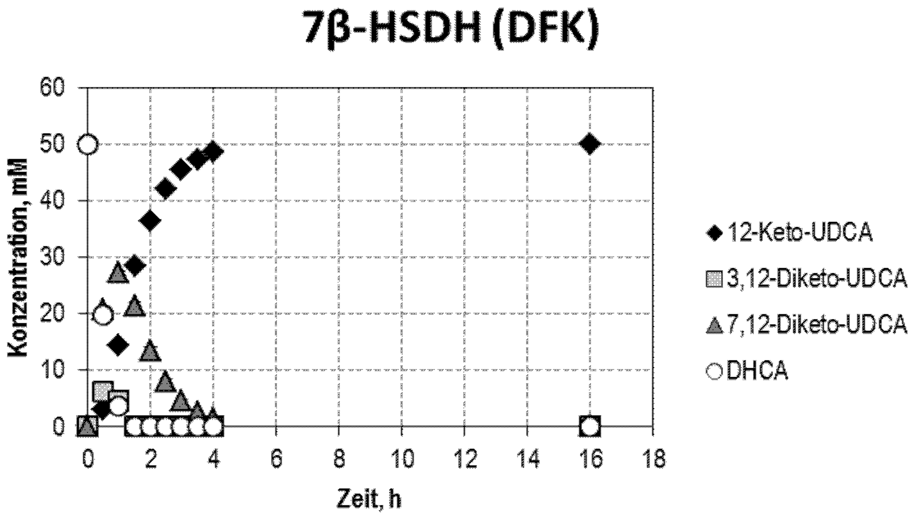
7β-HSDH (DFK)
Konzentration, mM
Zeit, h
12-Keto-UDCA
3,12-Diketo-UDCA
7,12-Diketo-UDCA
DHCA

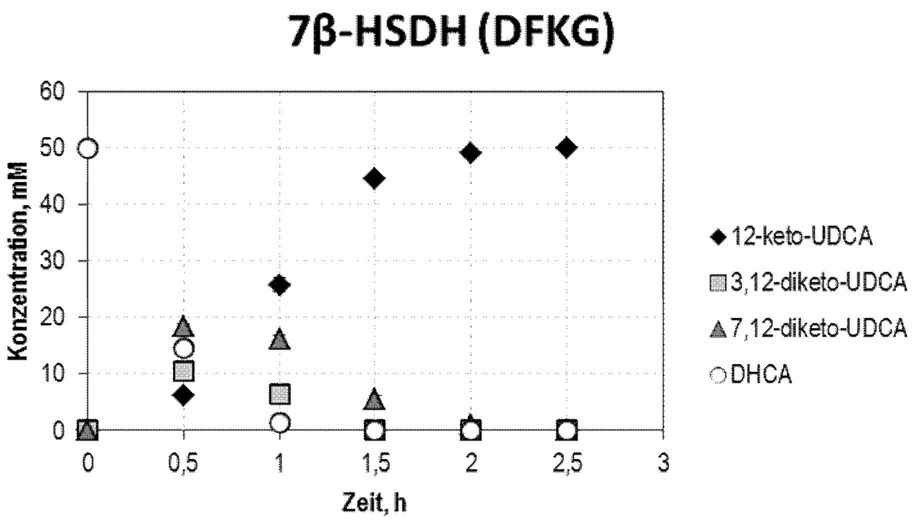
7β-HSDH (DFKG)
Konzentration, mM
Zeit, h
12-keto-UDCA
3,12-diketo-UDCA
7,12-diketo-UDCA
DHCA

Fig. 13

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2009002190 W
- WO 2009118176 A
- WO 2011064404 A
- WO 2012080504 A
- EP 2010068576 W
- EP 1149849 A
- EP 1069183 A
- DE OS100193773 A
- WO 2011147957 A


### In der Beschreibung aufgeführte Nicht-Patentliteratur


- **MONTI, D. et al.** One-Pot Multienzymatic Synthesis of 12-Ketoursodeoxycholic Acid: Subtle Cofactor Specificities Rule the Reaction Equilibria of Five Biocatalysts Working in a Row. *Advanced Synthesis & Catalysis,* 2009
- **ZHU, D. et al.** Enzymatic enantioselective reduction of -ketoesters by a thermostable 7 -hydroxysteroid dehydrogenase from Bacteroides fragilis. *Tetrahedron,* 2006, vol. 62 (18), 4535-4539
- **MACDONALD, I.A. ; P.D. ROACH.** Bile induction of 7 alpha- and 7 beta-hydroxysteroid dehydrogenases in Clostridium absonum. *Biochim Biophys Acta,* 1981, vol. 665 (2), 262-9
- **HIRANO, S. ; N. MASUDA.** Characterization of NADP-dependent 7 beta-hydroxysteroid dehydrogenases from Peptostreptococcus productus and Eubacterium aerofaciens. *Appl Environ Microbiol,* 1982, vol. 43 (5), 1057-63
- **S HIRANO ; N MASUDA.** Characterization of NADP-dependent 7 beta-hydroxysteroid dehydrogenases from Peptostreptococcus productus and Eubacterium aerofaciens. *Appl Environ Microbiol.,* 1982
- **J E PAWLOWSKI ; M HUIZINGA ; T M PENNING.** Cloning and sequencing of the cDNA for rat liver 3 alphahydroxysteroid/dihydrodiol dehydrogenase. *The Journal of Biological Chemistry,* 15. Mai 1991, vol. 266, 8820-8825
- **R.K. SCOPES.** Protein Purification. Springer Verlag, 1982
- Algorithmus von Pearson und Lipman. *Proc. Natl. Acad, Sci. (USA),* 1988, vol. 85 (8), 2444-2448
- **NARANG, S.A.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- **HIGGINS DG ; SHARP PM.** Fast and sensitive multiple sequence alignments on a microcomputer. *Comput Appl. Biosci.,* April 1989, vol. 5 (2), 151-1
- **CHENNA ; RAMU ; SUGAWARA ; HIDEAKI ; KOIKE ; TADASHI ; LOPEZ ; RODRIGO ; GIBSON ; TOBY J.** Multiple sequence alignment with the Clustal series of programs. *Nucleic Acids Res,* 2003, vol. 31 (13), 3497-500, http://www.ebi.ac.uk/Tools/clustalw/index.html#
- **VOET ; VOET.** Phosphoamiditmethode. Wiley Press, 896-897
- **SAMBROOK et al.** Molecular Cloning: A laboratory manual. Cold Spring Harbor Laboratory Press, 1989
- **SAMBROOK, J. ; FRITSCH, E.F. ; MANIATIS, T.** Molecular Cloning: A Laboratory Manual. Cold Spring Harbor Laboratory Press, 1989
- **SAMBROOK et al.** Molecular Cloning. Cold Spring Harbor Laboratory, 1989
- Current Protocols in Molecular Biology. John Wiley & Sons, 1985
- Nucleic Acids Hybridization: A Practical Approach. Oxford University Press, 1985
- Essential Molecular Biology: A Practical Approach. Oxford University Press, 1991
- **SAMBROOK, J. ; FRITSCH, E.F. ; MANIATIS, T.** Molecular Cloning (A Laboratory Manual). Cold Spring Harbor Laboratory Press, 1989, 9.31-9.57
- Current Protocols in Molecular Biology. John Wiley & Sons, 1989, 6.3.1-6.3.6
- **SAMBROOK ; RUSSELL.** Molecular Cloning. Cold Spring Harbor Laboratory Press, 2001
- In vitro mutagenesis protocols. Humana Press, 1996
- **KEGLER-EBO DM ; DOCKTOR CM ; DIMAIO D.** *Nucleic Acids Res,* 1994, vol. 22, 1593

- **BARETTINO D ; FEIGENBUTZ M ; VALCÄREL R ; STUNNENBERG HG.** *Nucleic Acids Res,* 1994, vol. 22, 541
- **BARIK S.** *Mol Biotechnol,* 1995, vol. 3, 1
- **ECKERT KA ; KUNKEL TA.** *Nucleic Acids Res,* 1990, vol. 18, 3739
- An efficient random mutagenesis technique u-sing an E.coli mutator strain. **GREENER A ; CALLAHAN M ; JERPSETH B.** In vitro mutagenesis protocols. Humana Press, 1996
- **STEMMER WPC.** *Nature,* 1994, vol. 370, 389
- **STEMMER WPC.** *Proc Natl Acad Sci USA,* 1994, vol. 91, 10747
- **REETZ MT ; JAEGER K-E.** *Topics Curr Chem,* 1999, vol. 200, 31
- Methods for optimizing industrial enzymes by directed evolution. **ZHAO H ; MOORE JC ; VOLKOV AA ; ARNOLD FH.** Manual of industrial microbiology and biotechnology. American Society for Microbiology, 1999
- **GOEDDE.** Gene Expression Technology: Methods in Enzymology. Academic Press, 1990, vol. 185
- Buch Cloning Vectors. Elsevier, 1985
- **T. MANIATIS ; E.F. FRITSCH ; J. SAMBROOK.** Molecular Cloning: A Laboratory Manual. Cold Spring Harbor Laboratory, 1989
- **T.J. SILHAVY ; M.L. BERMAN ; L.W. ENQUIST.** Experiments with Gene Fusions. Cold Spring Harbor Laboratory, 1984
- **AUSUBEL, F.M. et al.** Current Protocols in Molecular Biology. Greene Publishing Assoc, 1987
- Cloning Vectors. Elsevier, 1985
- Current Protocols in Molecular Biology. Wiley Interscience, 1997
- **SAMBROOK et al.** Molecular Cloning: A Laboratory Manual. Cold Spring Harbor Laboratory, 1989
- **TERPE, K.** *Appl. Microbiol. Biotechnol.,* 2006, vol. 72, 211-222
- Bioreaktoren und periphere Einrichtungen. Vieweg Verlag, 1994
- Handbuch ''Manual of Methods für General Bacteriology. American Society für Bacteriology, 1981
- Information über die Medienoptimierung ist erhältlich aus dem Lehrbuch. Applied Microbiol. Physiology, A Practical Approach. IRL Press, 1997, 53-73
- **HARLOW, E. ; LANE, D.** Antibodies: A Laboratory Manual. Cold Spring Harbor, 1988
- Immobilization of Enzymes. **J. LALONDE ; A. MARGOLIN.** Enzyme Catalysis in Organic Synthesis. Wiley-VCH, 2002, vol. III, 991-1032
- Current Protocols in Molecular Biology. John Wiley &Sons, 1993
- Gene Transfer and Expression. **KRIEGLER.** A Laboratory Manual. Stockton Press, 1990
- **WILMS et al.** *Material und gefüllten 1 L-Enghals-Erlenmeyerkolben überführt,* 2001
- **SAIKI R. K. et al.** *Science,* 1988, vol. 239, 487-491
- **SANCHIS et al.** *Appl. Microbiol. Biotechnol.,* 2008, vol. 81 (2), 387-97
- **LIU, H. ; NAISMITH, J. H.** *BMC Biotechnol.,* 2008, vol. 8, 91
- **BRAUN, M.** *PhD thesis, Technische Universität München,* 2011
- **LAEMMLI, U. K.** *Nature,* 1970, vol. 227 (5259), 680-5
- **FLING, S. P. ; GREGERSON, D. S.** *Anal. Biochem,* 1986, vol. 155 (1), 83-8
- **SULLER, M. T. ; LLOYD D.** *Cytometry,* 1999, vol. 35 (3), 235-41
- **LANGEMANN et al.** *Bioeng. Bugs,* 2010, vol. 1 (5), 326-36
- **REETZ et al.** *Mol. Biosyst.,* 2009, vol. 5 (2), 115-22
- **CARUGO, O. ; ARGOS, P.** *Proteins,* 1997, vol. 28 (1), 10-28
- *Proteins,* 1997, vol. 28 (1), 29-40
- **WOODYER et al.** *Biochemistry,* 2003, vol. 42 (40), 11604-14
- **BELLAMACINA, C. R.** *FASEB J.,* 1996, vol. 10 (11), 1257-69
- **KALLBERG et al.** *Eur. J. Biochem,* 2002, vol. 269 (18), 4409-4417
- **PERSSON, B.** *Chem. Biol. Interact.,* 2003, vol. 143-144, 271-278

SEQUENCE LISTING

<110> PharmaZell GmbH

<120> Neuartige Ganzzellreduktion von Dehydrocholsäure und Neuartige 7ß-Hydroxysteroiddehydrogenase-Mutanten und deren Verwendung

<130> M/54141

<160> 21

<170> PatentIn version 3.5

<210> 1
<211> 792
<212> DNA
<213> Collinsella aerofaciens

<220>
<221> CDS
<222> (1)..(792)

<400> 1

```
atg aac ctg agg gag aag tac ggt gag tgg ggc ctg atc ctg ggc gcg        48
Met Asn Leu Arg Glu Lys Tyr Gly Glu Trp Gly Leu Ile Leu Gly Ala
1               5                   10                  15

acc gag ggc gtc ggc aag gcg ttc tgc gag aag atc gcc gcc ggc ggc        96
Thr Glu Gly Val Gly Lys Ala Phe Cys Glu Lys Ile Ala Ala Gly Gly
            20                  25                  30

atg aac gtc gtc atg gtc ggc cgt cgc gag gag aag ctg aac gtg ctc       144
Met Asn Val Val Met Val Gly Arg Arg Glu Glu Lys Leu Asn Val Leu
        35                  40                  45

gca ggc gag atc cgc gag acc tac ggc gtg gag acc aag gtc gtg cgc       192
Ala Gly Glu Ile Arg Glu Thr Tyr Gly Val Glu Thr Lys Val Val Arg
    50                  55                  60

gcc gac ttt agc cag ccc ggc gct gcc gag acc gtc ttc gcc gcg acc       240
Ala Asp Phe Ser Gln Pro Gly Ala Ala Glu Thr Val Phe Ala Ala Thr
65                  70                  75                  80

gag ggc ctg gac atg ggc ttc atg agc tac gtg gcc tgc ctg cac agc       288
Glu Gly Leu Asp Met Gly Phe Met Ser Tyr Val Ala Cys Leu His Ser
                85                  90                  95

ttc ggt aag atc cag gac acc ccc tgg gag aag cac gag gcc atg atc       336
Phe Gly Lys Ile Gln Asp Thr Pro Trp Glu Lys His Glu Ala Met Ile
            100                 105                 110

aac gtc aac gtc gtg acc ttc ctc aag tgc ttc cac cac tac atg cgg       384
Asn Val Asn Val Val Thr Phe Leu Lys Cys Phe His His Tyr Met Arg
        115                 120                 125

atc ttt gcc gcc cag gac cgc ggc gcc gtg atc aac gtc tcg tcg atg       432
Ile Phe Ala Ala Gln Asp Arg Gly Ala Val Ile Asn Val Ser Ser Met
    130                 135                 140

acc ggc atc agc tcc agc ccc tgg aac ggc cag tac ggc gcg ggc aag       480
Thr Gly Ile Ser Ser Ser Pro Trp Asn Gly Gln Tyr Gly Ala Gly Lys
145                 150                 155                 160
```

```
gcc ttc atc ctc aag atg acc gag gcc gtg gcc tgc gag tgc gag ggc        528
Ala Phe Ile Leu Lys Met Thr Glu Ala Val Ala Cys Glu Cys Glu Gly
                165                 170                 175

acc ggc gtc gac gtc gag gtc atc acc ctc ggc acc acc cta acc ccc        576
Thr Gly Val Asp Val Glu Val Ile Thr Leu Gly Thr Thr Leu Thr Pro
            180                 185                 190

agc ctg ctg tcc aac ctc ccc ggc ggc ccg cag ggc gag gcc gtc atg        624
Ser Leu Leu Ser Asn Leu Pro Gly Gly Pro Gln Gly Glu Ala Val Met
        195                 200                 205

aag atc gcc ctc acc ccc gag gag tgc gtt gac gag gcc ttt gag aag        672
Lys Ile Ala Leu Thr Pro Glu Glu Cys Val Asp Glu Ala Phe Glu Lys
    210                 215                 220

ctg ggt aag gag ctc tcc gtc atc gcc ggc cag cgc aac aag gac tcc        720
Leu Gly Lys Glu Leu Ser Val Ile Ala Gly Gln Arg Asn Lys Asp Ser
225                 230                 235                 240

gtc cac gac tgg aag gca aac cac acc gag gac gag tac atc cgc tac        768
Val His Asp Trp Lys Ala Asn His Thr Glu Asp Glu Tyr Ile Arg Tyr
                245                 250                 255

atg ggg tcg ttc tac cgc gac tag                                        792
Met Gly Ser Phe Tyr Arg Asp
            260
```

<210> 2
<211> 263
<212> PRT
<213> Collinsella aerofaciens

<400> 2

```
Met Asn Leu Arg Glu Lys Tyr Gly Glu Trp Gly Leu Ile Leu Gly Ala
1               5                   10                  15

Thr Glu Gly Val Gly Lys Ala Phe Cys Glu Lys Ile Ala Ala Gly Gly
            20                  25                  30

Met Asn Val Val Met Val Gly Arg Arg Glu Glu Lys Leu Asn Val Leu
        35                  40                  45

Ala Gly Glu Ile Arg Glu Thr Tyr Gly Val Glu Thr Lys Val Val Arg
    50                  55                  60

Ala Asp Phe Ser Gln Pro Gly Ala Ala Glu Thr Val Phe Ala Ala Thr
65                  70                  75                  80

Glu Gly Leu Asp Met Gly Phe Met Ser Tyr Val Ala Cys Leu His Ser
                85                  90                  95

Phe Gly Lys Ile Gln Asp Thr Pro Trp Glu Lys His Glu Ala Met Ile
            100                 105                 110
```

```
Asn Val Asn Val Val Thr Phe Leu Lys Cys Phe His His Tyr Met Arg
        115                 120                 125

Ile Phe Ala Ala Gln Asp Arg Gly Ala Val Ile Asn Val Ser Ser Met
    130                 135                 140

Thr Gly Ile Ser Ser Ser Pro Trp Asn Gly Gln Tyr Gly Ala Gly Lys
145                 150                 155                 160

Ala Phe Ile Leu Lys Met Thr Glu Ala Val Ala Cys Glu Cys Glu Gly
                165                 170                 175

Thr Gly Val Asp Val Glu Val Ile Thr Leu Gly Thr Thr Leu Thr Pro
            180                 185                 190

Ser Leu Leu Ser Asn Leu Pro Gly Gly Pro Gln Gly Glu Ala Val Met
        195                 200                 205

Lys Ile Ala Leu Thr Pro Glu Glu Cys Val Asp Glu Ala Phe Glu Lys
    210                 215                 220

Leu Gly Lys Glu Leu Ser Val Ile Ala Gly Gln Arg Asn Lys Asp Ser
225                 230                 235                 240

Val His Asp Trp Lys Ala Asn His Thr Glu Asp Glu Tyr Ile Arg Tyr
                245                 250                 255

Met Gly Ser Phe Tyr Arg Asp
            260
```

<210> 3
<211> 774
<212> DNA
<213> Comamonas testosteroni

<220>
<221> CDS
<222> (1)..(774)

<400> 3

```
atg tcc atc atc gtg ata agc ggc tgc gcc acc ggc att ggt gcc gct       48
Met Ser Ile Ile Val Ile Ser Gly Cys Ala Thr Gly Ile Gly Ala Ala
1               5                   10                  15

acg cgc aag gtc ctg gag gcg gcc ggt cac cag atc gta ggc atc gat       96
Thr Arg Lys Val Leu Glu Ala Ala Gly His Gln Ile Val Gly Ile Asp
            20                  25                  30

ata cgc gat gcg gaa gtg att gcc gat ctc tcg acg gcc gaa ggt cga      144
Ile Arg Asp Ala Glu Val Ile Ala Asp Leu Ser Thr Ala Glu Gly Arg
```

```
        35                  40                  45

aag cag gcg att gcc gat gta ctg gcg aag tgc agc aag ggc atg gac      192
Lys Gln Ala Ile Ala Asp Val Leu Ala Lys Cys Ser Lys Gly Met Asp
    50                  55                  60

ggc ctg gtg ctg tgc gcc ggc ctg gga ccg cag acc aag gtg ctt ggc      240
Gly Leu Val Leu Cys Ala Gly Leu Gly Pro Gln Thr Lys Val Leu Gly
65                  70                  75                  80

aat gtg gtt tcg gtc aat tat ttt ggc gcg acc gag ctg atg gat gcc      288
Asn Val Val Ser Val Asn Tyr Phe Gly Ala Thr Glu Leu Met Asp Ala
                85                  90                  95

ttt ttg cca gcg ctg aaa aaa ggc cat cag ccc gca gcc gtc gtc atc      336
Phe Leu Pro Ala Leu Lys Lys Gly His Gln Pro Ala Ala Val Val Ile
            100                 105                 110

tcg tcc gtg gct tcc gcg cat ctg gct ttt gac aag aac cca ctg gcg      384
Ser Ser Val Ala Ser Ala His Leu Ala Phe Asp Lys Asn Pro Leu Ala
        115                 120                 125

ctg gca ctg gaa gcc ggc gag gaa gcc aag gcc cgc gcc att gtc gaa      432
Leu Ala Leu Glu Ala Gly Glu Glu Ala Lys Ala Arg Ala Ile Val Glu
    130                 135                 140

cat gcg gga gag cag ggc gga aat ctg gcc tat gcg ggc agc aag aat      480
His Ala Gly Glu Gln Gly Gly Asn Leu Ala Tyr Ala Gly Ser Lys Asn
145                 150                 155                 160

gct ttg acg gtg gct gtg cgc aaa cgc gcc gcc gcc tgg ggc gag gct      528
Ala Leu Thr Val Ala Val Arg Lys Arg Ala Ala Ala Trp Gly Glu Ala
                165                 170                 175

ggc gtg cgc ctg aac acc atc gcc ccc ggt gca acc gag act ccc ttg      576
Gly Val Arg Leu Asn Thr Ile Ala Pro Gly Ala Thr Glu Thr Pro Leu
            180                 185                 190

ctg cag gcg ggc ctg cag gac ccg cgc tat ggc gaa tcc att gcc aag      624
Leu Gln Ala Gly Leu Gln Asp Pro Arg Tyr Gly Glu Ser Ile Ala Lys
        195                 200                 205

ttc gtt cct ccc atg ggc cgc cgt gcc gag ccg tcc gag atg gcg tcg      672
Phe Val Pro Pro Met Gly Arg Arg Ala Glu Pro Ser Glu Met Ala Ser
    210                 215                 220

gtc atc gcc ttt ttg atg agc ccg gcc gca agc tat gtg cat ggc gcg      720
Val Ile Ala Phe Leu Met Ser Pro Ala Ala Ser Tyr Val His Gly Ala
225                 230                 235                 240

cag atc gtc att gat ggc ggc att gat gcg gtg atg cgc ccg aca cag      768
Gln Ile Val Ile Asp Gly Gly Ile Asp Ala Val Met Arg Pro Thr Gln
                245                 250                 255

ttc tga                                                              774
Phe
```

<210> 4
<211> 257
<212> PRT
<213> Comamonas testosteroni

<400> 4

Met Ser Ile Ile Val Ile Ser Gly Cys Ala Thr Gly Ile Gly Ala Ala
1 5 10 15

Thr Arg Lys Val Leu Glu Ala Ala Gly His Gln Ile Val Gly Ile Asp
20 25 30

Ile Arg Asp Ala Glu Val Ile Ala Asp Leu Ser Thr Ala Glu Gly Arg
35 40 45

Lys Gln Ala Ile Ala Asp Val Leu Ala Lys Cys Ser Lys Gly Met Asp
50 55 60

Gly Leu Val Leu Cys Ala Gly Leu Gly Pro Gln Thr Lys Val Leu Gly
65 70 75 80

Asn Val Val Ser Val Asn Tyr Phe Gly Ala Thr Glu Leu Met Asp Ala
85 90 95

Phe Leu Pro Ala Leu Lys Lys Gly His Gln Pro Ala Ala Val Val Ile
100 105 110

Ser Ser Val Ala Ser Ala His Leu Ala Phe Asp Lys Asn Pro Leu Ala
115 120 125

Leu Ala Leu Glu Ala Gly Glu Glu Ala Lys Ala Arg Ala Ile Val Glu
130 135 140

His Ala Gly Glu Gln Gly Gly Asn Leu Ala Tyr Ala Gly Ser Lys Asn
145 150 155 160

Ala Leu Thr Val Ala Val Arg Lys Arg Ala Ala Ala Trp Gly Glu Ala
165 170 175

Gly Val Arg Leu Asn Thr Ile Ala Pro Gly Ala Thr Glu Thr Pro Leu
180 185 190

Leu Gln Ala Gly Leu Gln Asp Pro Arg Tyr Gly Glu Ser Ile Ala Lys
195 200 205

Phe Val Pro Pro Met Gly Arg Arg Ala Glu Pro Ser Glu Met Ala Ser
210 215 220

Val Ile Ala Phe Leu Met Ser Pro Ala Ala Ser Tyr Val His Gly Ala
225 230 235 240

```
Gln Ile Val Ile Asp Gly Gly Ile Asp Ala Val Met Arg Pro Thr Gln
                245                 250                 255

Phe
```

<210> 5
<211> 768
<212> DNA
<213> Escherichia coli

<220>
<221> CDS
<222> (1)..(768)

<400> 5

```
gtg ttt aat tct gac aac ctg aga ctc gac gga aaa tgc gcc atc atc      48
Val Phe Asn Ser Asp Asn Leu Arg Leu Asp Gly Lys Cys Ala Ile Ile
1               5                   10                  15

aca ggt gcg ggt gca ggt att ggt aaa gaa atc gcc att aca ttc gcg      96
Thr Gly Ala Gly Ala Gly Ile Gly Lys Glu Ile Ala Ile Thr Phe Ala
            20                  25                  30

aca gct ggc gca tct gtg gtg gtc agt gat att aac gcc gac gca gct     144
Thr Ala Gly Ala Ser Val Val Val Ser Asp Ile Asn Ala Asp Ala Ala
        35                  40                  45

aac cat gtt gta gac gaa att caa caa ctg ggt ggt cag gca ttt gcc     192
Asn His Val Val Asp Glu Ile Gln Gln Leu Gly Gly Gln Ala Phe Ala
    50                  55                  60

tgc cgt tgt gat att act tcc gaa cag gaa ctc tct gca ctg gca gac     240
Cys Arg Cys Asp Ile Thr Ser Glu Gln Glu Leu Ser Ala Leu Ala Asp
65                  70                  75                  80

ttt gct atc agt aag ctg ggt aaa gtt gat att ctg gtt aac aac gcc     288
Phe Ala Ile Ser Lys Leu Gly Lys Val Asp Ile Leu Val Asn Asn Ala
                85                  90                  95

ggt ggc ggt gga cct aaa ccg ttt gat atg cca atg gcg gat ttt cgc     336
Gly Gly Gly Gly Pro Lys Pro Phe Asp Met Pro Met Ala Asp Phe Arg
            100                 105                 110

cgt gct tat gaa ctg aat gtg ttt tct ttt ttc cat ctg tca caa ctt     384
Arg Ala Tyr Glu Leu Asn Val Phe Ser Phe Phe His Leu Ser Gln Leu
        115                 120                 125

gtt gcg cca gaa atg gaa aaa aat ggc ggt ggc gtt att ctg acc atc     432
Val Ala Pro Glu Met Glu Lys Asn Gly Gly Gly Val Ile Leu Thr Ile
    130                 135                 140

act tct atg gcg gca gaa aat aaa aat ata aac atg act tcc tat gca     480
Thr Ser Met Ala Ala Glu Asn Lys Asn Ile Asn Met Thr Ser Tyr Ala
145                 150                 155                 160

tca tct aaa gct gcg gcc agt cat ctg gtc aga aat atg gcg ttt gac     528
Ser Ser Lys Ala Ala Ala Ser His Leu Val Arg Asn Met Ala Phe Asp
                165                 170                 175
```

```
cta ggt gaa aaa aat att cgg gta aat ggc att gcg ccg ggg gca ata         576
Leu Gly Glu Lys Asn Ile Arg Val Asn Gly Ile Ala Pro Gly Ala Ile
            180                 185                 190

tta acc gat gcc ctg aaa tcc gtt att aca cca gaa att gaa caa aaa         624
Leu Thr Asp Ala Leu Lys Ser Val Ile Thr Pro Glu Ile Glu Gln Lys
        195                 200                 205

atg tta cag cac acg ccg atc aga cgt ctg ggc caa ccg caa gat att         672
Met Leu Gln His Thr Pro Ile Arg Arg Leu Gly Gln Pro Gln Asp Ile
    210                 215                 220

gct aac gca gcg ctg ttc ctt tgc tcg cct gct gcg agc tgg gta agc         720
Ala Asn Ala Ala Leu Phe Leu Cys Ser Pro Ala Ala Ser Trp Val Ser
225                 230                 235                 240

gga caa att ctc acc gtc tcc ggt ggt ggg gta cag gag ctc aat taa         768
Gly Gln Ile Leu Thr Val Ser Gly Gly Gly Val Gln Glu Leu Asn
                245                 250                 255
```

<210> 6
<211> 255
<212> PRT
<213> Escherichia coli

<400> 6

```
Val Phe Asn Ser Asp Asn Leu Arg Leu Asp Gly Lys Cys Ala Ile Ile
1               5                   10                  15

Thr Gly Ala Gly Ala Gly Ile Gly Lys Glu Ile Ala Ile Thr Phe Ala
            20                  25                  30

Thr Ala Gly Ala Ser Val Val Val Ser Asp Ile Asn Ala Asp Ala Ala
        35                  40                  45

Asn His Val Val Asp Glu Ile Gln Gln Leu Gly Gly Gln Ala Phe Ala
    50                  55                  60

Cys Arg Cys Asp Ile Thr Ser Glu Gln Glu Leu Ser Ala Leu Ala Asp
65                  70                  75                  80

Phe Ala Ile Ser Lys Leu Gly Lys Val Asp Ile Leu Val Asn Asn Ala
                85                  90                  95

Gly Gly Gly Gly Pro Lys Pro Phe Asp Met Pro Met Ala Asp Phe Arg
            100                 105                 110

Arg Ala Tyr Glu Leu Asn Val Phe Ser Phe Phe His Leu Ser Gln Leu
        115                 120                 125

Val Ala Pro Glu Met Glu Lys Asn Gly Gly Gly Val Ile Leu Thr Ile
    130                 135                 140
```

Thr Ser Met Ala Ala Glu Asn Lys Asn Ile Asn Met Thr Ser Tyr Ala
145 150 155 160

Ser Ser Lys Ala Ala Ala Ser His Leu Val Arg Asn Met Ala Phe Asp
165 170 175

Leu Gly Glu Lys Asn Ile Arg Val Asn Gly Ile Ala Pro Gly Ala Ile
180 185 190

Leu Thr Asp Ala Leu Lys Ser Val Ile Thr Pro Glu Ile Glu Gln Lys
195 200 205

Met Leu Gln His Thr Pro Ile Arg Arg Leu Gly Gln Pro Gln Asp Ile
210 215 220

Ala Asn Ala Ala Leu Phe Leu Cys Ser Pro Ala Ala Ser Trp Val Ser
225 230 235 240

Gly Gln Ile Leu Thr Val Ser Gly Gly Gly Val Gln Glu Leu Asn
245 250 255

<210> 7
<211> 786
<212> DNA
<213> Bacillus subtilis

<400> 7

```
atgtatccgg atttaaaagg aaaagtcgtc gctattacag gagctgcttc agggctcgga      60
aaggcgatgg ccattcgctt cggcaaggag caggcaaaag tggttatcaa ctattatagt     120
aataaacaag atccgaacga ggtaaaagaa gaggtcatca aggcgggcgg tgaagctgtt     180
gtcgtccaag gagatgtcac gaaagaggaa gatgtaaaaa atatcgtgca aacggcaatt     240
aaggagttcg gcacactcga tattatgatt aataatgccg gtcttgaaaa tcctgtgcca     300
tctcacgaaa tgccgctcaa ggattgggat aaagtcatcg gcacgaactt aacgggtgcc     360
tttttaggaa gccgtgaagc gattaaatat ttcgtagaaa acgatatcaa gggaaatgtc     420
attaacatgt ccagtgtgca cgaagtgatt ccttggccgt tatttgtcca ctatgcggca     480
agtaaaggcg ggataaagct gatgacagaa acattagcgt tggaatacgc gccgaagggc     540
attcgcgtca ataatattgg gccaggtgcg atcaacacgc caatcaatgc tgaaaaattc     600
gctgacccta aacagaaagc tgatgtagaa agcatgattc caatgggata tatcggcgaa     660
ccggaggaga tcgccgcagt agcagcctgg cttgcttcga aggaagccag ctacgtcaca     720
ggcatcacgt tattcgcgga cggcggtatg acacaatatc cttcattcca ggcaggccgc     780
ggttaa                                                                786
```

<210> 8
<211> 261
<212> PRT
<213> Bacillus subtilis

<400> 8

```
Met Tyr Pro Asp Leu Lys Gly Lys Val Val Ala Ile Thr Gly Ala Ala
1               5                   10                  15

Ser Gly Leu Gly Lys Ala Met Ala Ile Arg Phe Gly Lys Glu Gln Ala
            20                  25                  30

Lys Val Val Ile Asn Tyr Tyr Ser Asn Lys Gln Asp Pro Asn Glu Val
        35                  40                  45

Lys Glu Glu Val Ile Lys Ala Gly Gly Glu Ala Val Val Val Gln Gly
    50                  55                  60

Asp Val Thr Lys Glu Glu Asp Val Lys Asn Ile Val Gln Thr Ala Ile
65                  70                  75                  80

Lys Glu Phe Gly Thr Leu Asp Ile Met Ile Asn Asn Ala Gly Leu Glu
                85                  90                  95

Asn Pro Val Pro Ser His Glu Met Pro Leu Lys Asp Trp Asp Lys Val
            100                 105                 110

Ile Gly Thr Asn Leu Thr Gly Ala Phe Leu Gly Ser Arg Glu Ala Ile
        115                 120                 125

Lys Tyr Phe Val Glu Asn Asp Ile Lys Gly Asn Val Ile Asn Met Ser
    130                 135                 140

Ser Val His Glu Val Ile Pro Trp Pro Leu Phe Val His Tyr Ala Ala
145                 150                 155                 160

Ser Lys Gly Gly Ile Lys Leu Met Thr Glu Thr Leu Ala Leu Glu Tyr
                165                 170                 175

Ala Pro Lys Gly Ile Arg Val Asn Asn Ile Gly Pro Gly Ala Ile Asn
            180                 185                 190

Thr Pro Ile Asn Ala Glu Lys Phe Ala Asp Pro Lys Gln Lys Ala Asp
        195                 200                 205

Val Glu Ser Met Ile Pro Met Gly Tyr Ile Gly Glu Pro Glu Glu Ile
    210                 215                 220
```

```
Ala Ala Val Ala Ala Trp Leu Ala Ser Lys Glu Ala Ser Tyr Val Thr
225                 230                 235                 240

Gly Ile Thr Leu Phe Ala Asp Gly Gly Met Thr Gln Tyr Pro Ser Phe
                245                 250                 255

Gln Ala Gly Arg Gly
            260
```

<210> 9
<211> 263
<212> PRT
<213> Artificial Sequence

<220>
<223> abgeleitet aus Collinsella aerofaciens (G39D)

<400> 9

```
Met Asn Leu Arg Glu Lys Tyr Gly Glu Trp Gly Leu Ile Leu Gly Ala
1               5                   10                  15

Thr Glu Gly Val Gly Lys Ala Phe Cys Glu Lys Ile Ala Ala Gly Gly
            20                  25                  30

Met Asn Val Val Met Val Asp Arg Arg Glu Glu Lys Leu Asn Val Leu
        35                  40                  45

Ala Gly Glu Ile Arg Glu Thr Tyr Gly Val Glu Thr Lys Val Val Arg
    50                  55                  60

Ala Asp Phe Ser Gln Pro Gly Ala Ala Glu Thr Val Phe Ala Ala Thr
65                  70                  75                  80

Glu Gly Leu Asp Met Gly Phe Met Ser Tyr Val Ala Cys Leu His Ser
                85                  90                  95

Phe Gly Lys Ile Gln Asp Thr Pro Trp Glu Lys His Glu Ala Met Ile
            100                 105                 110

Asn Val Asn Val Val Thr Phe Leu Lys Cys Phe His His Tyr Met Arg
        115                 120                 125

Ile Phe Ala Ala Gln Asp Arg Gly Ala Val Ile Asn Val Ser Ser Met
    130                 135                 140

Thr Gly Ile Ser Ser Ser Pro Trp Asn Gly Gln Tyr Gly Ala Gly Lys
145                 150                 155                 160
```

Ala Phe Ile Leu Lys Met Thr Glu Ala Val Ala Cys Glu Cys Glu Gly
165 170 175

Thr Gly Val Asp Val Glu Val Ile Thr Leu Gly Thr Thr Leu Thr Pro
180 185 190

Ser Leu Leu Ser Asn Leu Pro Gly Gly Pro Gln Gly Glu Ala Val Met
195 200 205

Lys Ile Ala Leu Thr Pro Glu Glu Cys Val Asp Glu Ala Phe Glu Lys
210 215 220

Leu Gly Lys Glu Leu Ser Val Ile Ala Gly Gln Arg Asn Lys Asp Ser
225 230 235 240

Val His Asp Trp Lys Ala Asn His Thr Glu Asp Glu Tyr Ile Arg Tyr
245 250 255

Met Gly Ser Phe Tyr Arg Asp
260

<210> 10
<211> 263
<212> PRT
<213> Artificial Sequence

<220>
<223> 7β-HSDH Mutante

<400> 10

Met Asn Leu Arg Glu Lys Tyr Gly Glu Trp Gly Leu Ile Leu Gly Ala
1               5                   10                  15

Thr Glu Gly Val Gly Lys Ala Phe Cys Glu Lys Ile Ala Ala Gly Gly
            20                  25                  30

Met Asn Val Val Met Val Asp Ile Arg Glu Glu Lys Leu Asn Val Leu
        35                  40                  45

Ala Gly Glu Ile Arg Glu Thr Tyr Gly Val Glu Thr Lys Val Val Arg
    50                  55                  60

Ala Asp Phe Ser Gln Pro Gly Ala Ala Glu Thr Val Phe Ala Ala Thr
65                  70                  75                  80

Glu Gly Leu Asp Met Gly Phe Met Ser Tyr Val Ala Cys Leu His Ser
                85                  90                  95

Phe Gly Lys Ile Gln Asp Thr Pro Trp Glu Lys His Glu Ala Met Ile

```
                100                 105                 110

Asn Val Asn Val Val Thr Phe Leu Lys Cys Phe His His Tyr Met Arg
        115                 120                 125

Ile Phe Ala Ala Gln Asp Arg Gly Ala Val Ile Asn Val Ser Ser Met
    130                 135                 140

Thr Gly Ile Ser Ser Ser Pro Trp Asn Gly Gln Tyr Gly Ala Gly Lys
145                 150                 155                 160

Ala Phe Ile Leu Lys Met Thr Glu Ala Val Ala Cys Glu Cys Glu Gly
                165                 170                 175

Thr Gly Val Asp Val Glu Val Ile Thr Leu Gly Thr Thr Leu Thr Pro
            180                 185                 190

Ser Leu Leu Ser Asn Leu Pro Gly Gly Pro Gln Gly Glu Ala Val Met
        195                 200                 205

Lys Ile Ala Leu Thr Pro Glu Glu Cys Val Asp Glu Ala Phe Glu Lys
    210                 215                 220

Leu Gly Lys Glu Leu Ser Val Ile Ala Gly Gln Arg Asn Lys Asp Ser
225                 230                 235                 240

Val His Asp Trp Lys Ala Asn His Thr Glu Asp Glu Tyr Ile Arg Tyr
                245                 250                 255

Met Gly Ser Phe Tyr Arg Asp
            260
```

<210> 11
<211> 263
<212> PRT
<213> Artificial Sequence

<220>
<223> 7β-HSDH Mutante

<400> 11

```
Met Asn Leu Arg Glu Lys Tyr Gly Glu Trp Gly Leu Ile Leu Gly Ala
1               5                   10                  15

Thr Glu Gly Val Gly Lys Ala Phe Cys Glu Lys Ile Ala Ala Gly Gly
            20                  25                  30

Met Asn Val Val Met Val Asp Phe Arg Glu Glu Lys Leu Asn Val Leu
        35                  40                  45
```

```
Ala Gly Glu Ile Arg Glu Thr Tyr Gly Val Glu Thr Lys Val Val Arg
    50                  55                  60

Ala Asp Phe Ser Gln Pro Gly Ala Ala Glu Thr Val Phe Ala Ala Thr
65                  70                  75                  80

Glu Gly Leu Asp Met Gly Phe Met Ser Tyr Val Ala Cys Leu His Ser
                85                  90                  95

Phe Gly Lys Ile Gln Asp Thr Pro Trp Glu Lys His Glu Ala Met Ile
            100                 105                 110

Asn Val Asn Val Val Thr Phe Leu Lys Cys Phe His His Tyr Met Arg
        115                 120                 125

Ile Phe Ala Ala Gln Asp Arg Gly Ala Val Ile Asn Val Ser Ser Met
    130                 135                 140

Thr Gly Ile Ser Ser Ser Pro Trp Asn Gly Gln Tyr Gly Ala Gly Lys
145                 150                 155                 160

Ala Phe Ile Leu Lys Met Thr Glu Ala Val Ala Cys Glu Cys Glu Gly
                165                 170                 175

Thr Gly Val Asp Val Glu Val Ile Thr Leu Gly Thr Thr Leu Thr Pro
            180                 185                 190

Ser Leu Leu Ser Asn Leu Pro Gly Gly Pro Gln Gly Glu Ala Val Met
        195                 200                 205

Lys Ile Ala Leu Thr Pro Glu Glu Cys Val Asp Glu Ala Phe Glu Lys
    210                 215                 220

Leu Gly Lys Glu Leu Ser Val Ile Ala Gly Gln Arg Asn Lys Asp Ser
225                 230                 235                 240

Val His Asp Trp Lys Ala Asn His Thr Glu Asp Glu Tyr Ile Arg Tyr
                245                 250                 255

Met Gly Ser Phe Tyr Arg Asp
            260
```

<210> 12
<211> 263
<212> PRT
<213> Artificial Sequence

<220>

<223> 7β-HSDH Mutante

<400> 12

```
Met Asn Leu Arg Glu Lys Tyr Gly Glu Trp Gly Leu Ile Leu Gly Ala
1               5                   10                  15

Thr Glu Gly Val Gly Lys Ala Phe Cys Glu Lys Ile Ala Ala Gly Gly
            20                  25                  30

Met Asn Val Val Met Val Asp Phe Lys Glu Glu Lys Leu Asn Val Leu
        35                  40                  45

Ala Gly Glu Ile Arg Glu Thr Tyr Gly Val Glu Thr Lys Val Val Arg
    50                  55                  60

Ala Asp Phe Ser Gln Pro Gly Ala Ala Glu Thr Val Phe Ala Ala Thr
65                  70                  75                  80

Glu Gly Leu Asp Met Gly Phe Met Ser Tyr Val Ala Cys Leu His Ser
                85                  90                  95

Phe Gly Lys Ile Gln Asp Thr Pro Trp Glu Lys His Glu Ala Met Ile
            100                 105                 110

Asn Val Asn Val Val Thr Phe Leu Lys Cys Phe His His Tyr Met Arg
        115                 120                 125

Ile Phe Ala Ala Gln Asp Arg Gly Ala Val Ile Asn Val Ser Ser Met
    130                 135                 140

Thr Gly Ile Ser Ser Ser Pro Trp Asn Gly Gln Tyr Gly Ala Gly Lys
145                 150                 155                 160

Ala Phe Ile Leu Lys Met Thr Glu Ala Val Ala Cys Glu Cys Glu Gly
                165                 170                 175

Thr Gly Val Asp Val Glu Val Ile Thr Leu Gly Thr Thr Leu Thr Pro
            180                 185                 190

Ser Leu Leu Ser Asn Leu Pro Gly Gly Pro Gln Gly Glu Ala Val Met
        195                 200                 205

Lys Ile Ala Leu Thr Pro Glu Glu Cys Val Asp Glu Ala Phe Glu Lys
    210                 215                 220

Leu Gly Lys Glu Leu Ser Val Ile Ala Gly Gln Arg Asn Lys Asp Ser
225                 230                 235                 240
```

Val His Asp Trp Lys Ala Asn His Thr Glu Asp Glu Tyr Ile Arg Tyr
245 250 255

Met Gly Ser Phe Tyr Arg Asp
260

<210> 13
<211> 263
<212> PRT
<213> Artificial Sequence

<220>
<223> 7β-HSDH Mutante

<400> 13

Met Asn Leu Arg Glu Lys Tyr Gly Glu Trp Gly Leu Ile Leu Gly Ala
1 5 10 15

Thr Glu Gly Val Gly Lys Ala Phe Cys Glu Lys Ile Ala Ala Gly Gly
20 25 30

Met Asn Val Val Met Val Asp Phe Lys Glu Glu Gly Leu Asn Val Leu
35 40 45

Ala Gly Glu Ile Arg Glu Thr Tyr Gly Val Glu Thr Lys Val Val Arg
50 55 60

Ala Asp Phe Ser Gln Pro Gly Ala Ala Glu Thr Val Phe Ala Ala Thr
65 70 75 80

Glu Gly Leu Asp Met Gly Phe Met Ser Tyr Val Ala Cys Leu His Ser
85 90 95

Phe Gly Lys Ile Gln Asp Thr Pro Trp Glu Lys His Glu Ala Met Ile
100 105 110

Asn Val Asn Val Val Thr Phe Leu Lys Cys Phe His His Tyr Met Arg
115 120 125

Ile Phe Ala Ala Gln Asp Arg Gly Ala Val Ile Asn Val Ser Ser Met
130 135 140

Thr Gly Ile Ser Ser Ser Pro Trp Asn Gly Gln Tyr Gly Ala Gly Lys
145 150 155 160

Ala Phe Ile Leu Lys Met Thr Glu Ala Val Ala Cys Glu Cys Glu Gly
165 170 175

```
Thr Gly Val Asp Val Glu Val Ile Thr Leu Gly Thr Thr Leu Thr Pro
            180                 185                 190

Ser Leu Leu Ser Asn Leu Pro Gly Gly Pro Gln Gly Glu Ala Val Met
        195                 200                 205

Lys Ile Ala Leu Thr Pro Glu Glu Cys Val Asp Glu Ala Phe Glu Lys
    210                 215                 220

Leu Gly Lys Glu Leu Ser Val Ile Ala Gly Gln Arg Asn Lys Asp Ser
225                 230                 235                 240

Val His Asp Trp Lys Ala Asn His Thr Glu Asp Glu Tyr Ile Arg Tyr
                245                 250                 255

Met Gly Ser Phe Tyr Arg Asp
            260
```

<210> 14
<211> 26
<212> DNA
<213> Artificial Sequence

<220>
<223> PCR Primer

<400> 14
cgtcgtcatg gtcgactttc gcgagg 26

<210> 15
<211> 31
<212> DNA
<213> Artificial Sequence

<220>
<223> PCR Primer

<400> 15
ccgccgcatc cataccgcca gttgtttacc c 31

<210> 16
<211> 34
<212> DNA
<213> Artificial Sequence

<220>
<223> PCR Primer

<400> 16
cgtcgtcatg gtcgacttta aagaggagaa gctg 34

<210> 17
<211> 33
<212> DNA

<213> Artificial Sequence

<220>
<223> PCR Primer

<220>
<221> misc_feature
<222> (19)..(19)
<223> n is a, c, g, or t

<400> 17
gtcgacttta aagaggagnd tctgaacgtg ctc 33

<210> 18
<211> 7891
<212> DNA
<213> Artificial Sequence

<220>
<223> Plasmid p7(A)T3rG-A

<400> 18

```
atccggatat agttcctcct ttcagcaaaa aacccctcaa gacccgttta gaggccccaa      60
ggggttatgc tagttattgc tcagcggtgg cagcagccaa ctcagcttcc tttcgggctt     120
tgttagcagc cggatctcag tggtggtggt ggtggtgctc gagttaaccg cggcctgcct     180
ggaatgaagg atattgtgtc ataccgccgt ccgcgaataa cgtgatgcct gtgacgtagc     240
tggcttcctt cgaagcaagc caggctgcta ctgcggcgat ctcctccggt tcgccgatat     300
atcccattgg aatcatgctt tctacatcag ctttctgttt agggtcagcg aatttttcag     360
cattgattgg cgtgttgatc gcacctggcc caatattatt gacgcgaatg cccttcggcg     420
cgtattccaa cgctaatgtt tctgtcatca gctttatccc gcctttactt gccgcatagt     480
ggacaaataa cggccaagga atcacttcgt gcacactgga catgttaatg acatttccct     540
tgatatcgtt ttctacgaaa tatttaatcg cttcacggct tcctaaaaag gcacccgtta     600
agttcgtgcc gatgacttta tcccaatcct tgagcggcat ttcgtgagat ggcacaggat     660
tttcaagacc ggcattatta atcataatat cgagtgtgcc gaactcctta attgccgttt     720
gcacgatatt ttttacatct tcctctttcg tgacatctcc ttggacgaca acagcttcac     780
cgcccgcctt gatgacctct tcttttacct cgttcggatc ttgtttatta ctataatagt     840
tgataaccac ttttgcctgc tccttgccga agcgaatggc catcgccttt ccgagccctg     900
aagcagctcc tgtaatagcg acgacttttc cttttaaatc cggatacatg tatatctcct     960
tgcggccgct cagaactgtg tcgggcgcat caccgcatca atgccgccat caatgacgat    1020
ctgcgcgcca tgcacatagc ttgcggccgg gctcatcaaa aaggcgatga ccgacgccat    1080
ctcggacggc tcggcacggc ggcccatggg aggaacgaac ttggcaatgg attcgccata    1140
```

gcgcgggtcc tgcaggcccg cctgcagcaa gggagtctcg gttgcaccgg gggcgatggt 1200
gttcaggcgc acgccagcct cgccccaggc ggcggcgcgt ttgcgcacag ccaccgtcaa 1260
agcattcttg ctgcccgcat aggccagatt tccgccctgc tctcccgcat gttcgacaat 1320
ggcgcgggcc ttggcttcct cgccggcttc cagtgccagc gccagtgggt tcttgtcaaa 1380
agccagatgc gcggaagcca cggacgagat gacgacggct gcgggctgat ggcctttttt 1440
cagcgctggc aaaaaggcat ccatcagctc ggtcgcgcca aaataattga ccgaaaccac 1500
attgccaagc accttggtct gcggtcccag gccggcgcac agcaccaggc cgtccatgcc 1560
cttgctgcac ttcgccagta catcggcaat cgcctgcttt cgaccttcgg ccgtcgagag 1620
atcggcaatc acttccgcat cgcgtatatc gatgcctacg atctggtgac cggccgcctc 1680
caggaccttg cgcgtagcgg caccaatgcc ggtggcgcag ccgcttatca cgatgatgga 1740
catcatatgt atatctcctt cttatactta actaatatac taagatgggg aattgttatc 1800
cgctcacaat tcccctatag tgagtcgtat taatttcgat tatgcggccg tgtacaatac 1860
gattactttc tgttcgactt aagcattata agcttctagt cgcggtagaa cgaccccatg 1920
tagcggatgt actcgtcctc ggtgtggttt gccttccagt cgtggacgga gtccttgttg 1980
cgctggccgg cgatgacgga gagctcctta cccagcttct caaaggcctc gtcaacgcac 2040
tcctcggggg tgagggcgat cttcatgacg gcctcgccct gcgggccgcc ggggaggttg 2100
gacagcaggc tgggggttag ggtggtgccg agggtgatga cctcgacgtc gacgccggtg 2160
ccctcgcact cgcaggccac ggcctcggtc atcttgagga tgaaggcctt gcccgcgccg 2220
tactggccgt tccaggggct ggagctgatg ccggtcatcg acgagacgtt gatcacggcg 2280
ccgcggtcct gggcggcaaa gatccgcatg tagtggtgga agcacttgag gaaggtcacg 2340
acgttgacgt tgatcatggc ctcgtgcttc tcccaggggg tgtcctggat cttaccgaag 2400
ctgtgcaggc aggccacgta gctcatgaag cccatgtcca ggccctcggt cgcggcgaag 2460
acggtctcgg cagcgccggg ctggctaaag tcggcgcgca cgaccttggt ctccacgccg 2520
taggtctcgc ggatctcgcc tgcgagcacg ttcagcttct cctcgcgacg ggcgaccatg 2580
acgacgttca tgccgccggc ggcgatcttc tcgcagaacg ccttgccgac gccctcggtc 2640
gcgcccagga tcaggcccca ctcaccgtac ttctccctca ggttcatatg tatatctcct 2700
tcttaaagtt aaacaaaatt atttctagag gggaattgtt atccgctcac aattccccta 2760
tagtgagtcg tattaatttc gcgggatcga gatctcgatc ctctacgccg gacgcatcgt 2820
ggccggcatc accggcgcca caggtgcggt tgctggcgcc tatatcgccg acatcaccga 2880
tggggaagat cgggctcgcc acttcgggct catgagcgct tgtttcggcg tgggtatggt 2940
ggcaggcccc gtggccgggg gactgttggg cgccatctcc ttgcatgcac cattccttgc 3000
ggcggcggtg ctcaacggcc tcaacctact actgggctgc ttcctaatgc aggagtcgca 3060

taagggagag cgtcgagatc ccggacacca tcgaatggcg caaaaccttt cgcggtatgg 3120
catgatagcg cccggaagag agtcaattca gggtggtgaa tgtgaaacca gtaacgttat 3180
acgatgtcgc agagtatgcc ggtgtctctt atcagaccgt ttcccgcgtg gtgaaccagg 3240
ccagccacgt ttctgcgaaa acgcgggaaa aagtggaagc ggcgatggcg gagctgaatt 3300
acattcccaa ccgcgtggca caacaactgg cgggcaaaca gtcgttgctg attggcgttg 3360
ccacctccag tctggccctg cacgcgccgt cgcaaattgt cgcggcgatt aaatctcgcg 3420
ccgatcaact gggtgccagc gtggtggtgt cgatggtaga acgaagcggc gtcgaagcct 3480
gtaaagcggc ggtgcacaat cttctcgcgc aacgcgtcag tgggctgatc attaactatc 3540
cgctggatga ccaggatgcc attgctgtgg aagctgcctg cactaatgtt ccggcgttat 3600
ttcttgatgt ctctgaccag acacccatca acagtattat tttctcccat gaagacggta 3660
cgcgactggg cgtggagcat ctggtcgcat tgggtcacca gcaaatcgcg ctgttagcgg 3720
gcccattaag ttctgtctcg gcgcgtctgc gtctggctgg ctggcataaa tatctcactc 3780
gcaatcaaat tcagccgata gcggaacggg aaggcgactg gagtgccatg tccggttttc 3840
aacaaaccat gcaaatgctg aatgagggca tcgttcccac tgcgatgctg gttgccaacg 3900
atcagatggc gctgggcgca atgcgcgcca ttaccgagtc cgggctgcgc gttggtgcgg 3960
atatctcggt agtgggatac gacgataccg aagacagctc atgttatatc ccgccgttaa 4020
ccaccatcaa acaggatttt cgcctgctgg ggcaaaccag cgtggaccgc ttgctgcaac 4080
tctctcaggg ccaggcggtg aagggcaatc agctgttgcc cgtctcactg gtgaaaagaa 4140
aaaccaccct ggcgcccaat acgcaaaccg cctctccccg cgcgttggcc gattcattaa 4200
tgcagctggc acgacaggtt tcccgactgg aaagcgggca gtgagcgcaa cgcaattaat 4260
gtaagttagc tcactcatta ggcaccggga tctcgaccga tgcccttgag agccttcaac 4320
ccagtcagct ccttccggtg ggcgcggggc atgactatcg tcgccgcact tatgactgtc 4380
ttctttatca tgcaactcgt aggacaggtg ccggcagcgc tctgggtcat tttcggcgag 4440
gaccgctttc gctggagcgc gacgatgatc ggcctgtcgc ttgcggtatt cggaatcttg 4500
cacgccctcg ctcaagcctt cgtcactggt cccgccacca aacgtttcgg cgagaagcag 4560
gccattatcg ccggcatggc ggccccacgg gtgcgcatga tcgtgctcct gtcgttgagg 4620
acccggctag gctggcgggg ttgccttact ggttagcaga atgaatcacc gatacgcgag 4680
cgaacgtgaa gcgactgctg ctgcaaaacg tctgcgacct gagcaacaac atgaatggtc 4740
ttcggtttcc gtgtttcgta aagtctggaa acgcggaagt cagcgccctg caccattatg 4800
ttccggatct gcatcgcagg atgctgctgg ctaccctgtg gaacacctac atctgtatta 4860
acgaagcgct ggcattgacc ctgagtgatt tttctctggt cccgccgcat ccataccgcc 4920

agttgtttac cctcacaacg ttccagtaac cgggcatgtt catcatcagt aacccgtatc 4980

gtgagcatcc tctctcgttt catcggtatc attaccccca tgaacagaaa tcccccttac 5040

acggaggcat cagtgaccaa acaggaaaaa accgccctta acatggcccg ctttatcaga 5100

agccagacat taacgcttct ggagaaactc aacgagctgg acgcggatga acaggcagac 5160

atctgtgaat cgcttcacga ccacgctgat gagctttacc gcagctgcct cgcgcgtttc 5220

ggtgatgacg gtgaaaacct ctgacacatg cagctcccgg agacggtcac agcttgtctg 5280

taagcggatg ccgggagcag acaagcccgt cagggcgcgt cagcgggtgt tggcgggtgt 5340

cggggcgcag ccatgaccca gtcacgtagc gatagcggag tgtatactgg cttaactatg 5400

cggcatcaga gcagattgta ctgagagtgc accatatatg cggtgtgaaa taccgcacag 5460

atgcgtaagg agaaaatacc gcatcaggcg ctcttccgct tcctcgctca ctgactcgct 5520

gcgctcggtc gttcggctgc ggcgagcggt atcagctcac tcaaaggcgg taatacggtt 5580

atccacagaa tcaggggata acgcaggaaa gaacatgtga gcaaaaggcc agcaaaaggc 5640

caggaaccgt aaaaaggccg cgttgctggc gtttttccat aggctccgcc cccctgacga 5700

gcatcacaaa aatcgacgct caagtcagag gtggcgaaac ccgacaggac tataaagata 5760

ccaggcgttt ccccctggaa gctccctcgt gcgctctcct gttccgaccc tgccgcttac 5820

cggatacctg tccgcctttc tcccttcggg aagcgtggcg ctttctcata gctcacgctg 5880

taggtatctc agttcggtgt aggtcgttcg ctccaagctg ggctgtgtgc acgaaccccc 5940

cgttcagccc gaccgctgcg ccttatccgg taactatcgt cttgagtcca acccggtaag 6000

acacgactta tcgccactgg cagcagccac tggtaacagg attagcagag cgaggtatgt 6060

aggcggtgct acagagttct tgaagtggtg gcctaactac ggctacacta gaaggacagt 6120

atttggtatc tgcgctctgc tgaagccagt taccttcgga aaaagagttg gtagctcttg 6180

atccggcaaa caaaccaccg ctggtagcgg tggttttttt gtttgcaagc agcagattac 6240

gcgcagaaaa aaaggatctc aagaagatcc tttgatcttt tctacggggt ctgacgctca 6300

gtggaacgaa aactcacgtt aagggatttt ggtcatgaga ttatcaaaaa ggatcttcac 6360

ctagatcctt ttaaattaaa aatgaagttt taaatcaatc taaagtatat atgagtaaac 6420

ttggtctgac agttaccaat gcttaatcag tgaggcacct atctcagcga tctgtctatt 6480

tcgttcatcc atagttgcct gactccccgt cgtgtagata actacgatac gggagggctt 6540

accatctggc cccagtgctg caatgatacc gcgagaccca cgctcaccgg ctccagattt 6600

atcagcaata aaccagccag ccggaagggc cgagcgcaga agtggtcctg caactttatc 6660

cgcctccatc cagtctatta attgttgccg ggaagctaga gtaagtagtt cgccagttaa 6720

tagtttgcgc aacgttgttg ccattgctgc aggcatcgtg gtgtcacgct cgtcgtttgg 6780

tatggcttca ttcagctccg gttcccaacg atcaaggcga gttacatgat cccccatgtt 6840

gtgcaaaaaa gcggttagct ccttcggtcc tccgatcgtt gtcagaagta agttggccgc 6900

agtgttatca ctcatggtta tggcagcact gcataattct cttactgtca tgccatccgt 6960

aagatgcttt tctgtgactg gtgagtactc aaccaagtca ttctgagaat agtgtatgcg 7020

gcgaccgagt tgctcttgcc cggcgtcaat acgggataat accgcgccac atagcagaac 7080

tttaaaagtg ctcatcattg gaaaacgttc ttcggggcga aaactctcaa ggatcttacc 7140

gctgttgaga tccagttcga tgtaacccac tcgtgcaccc aactgatctt cagcatcttt 7200

tactttcacc agcgtttctg ggtgagcaaa aacaggaagg caaaatgccg caaaaaaggg 7260

aataagggcg acacggaaat gttgaatact catactcttc ctttttcaat attattgaag 7320

catttatcag ggttattgtc tcatgagcgg atacatattt gaatgtattt agaaaaataa 7380

acaaataggg gttccgcgca catttccccg aaaagtgcca cctgaaattg taaacgttaa 7440

tattttgtta aaattcgcgt taaatttttg ttaaatcagc tcatttttta accaataggc 7500

cgaaatcggc aaaatccctt ataaatcaaa agaatagacc gagatagggt tgagtgttgt 7560

tccagtttgg aacaagagtc cactattaaa gaacgtggac tccaacgtca aagggcgaaa 7620

aaccgtctat cagggcgatg gcccactacg tgaaccatca ccctaatcaa gtttttggg 7680

gtcgaggtgc cgtaaagcac taaatcggaa ccctaaaggg agcccccgat ttagagcttg 7740

acggggaaag ccggcgaacg tggcgagaaa ggaagggaag aaagcgaaag gagcgggcgc 7800

tagggcgctg gcaagtgtag cggtcacgct gcgcgtaacc accacacccg ccgcgcttaa 7860

tgcgccgcta cagggcgcgt cccattcgcc a 7891

<210> 19
<211> 7758
<212> DNA
<213> Artificial Sequence

<220>
<223> Plasmid p7(A)T3rG-K

<400> 19

```
atccggatat agttcctcct ttcagcaaaa aacccctcaa gacccgttta gaggccccaa        60
ggggttatgc tagttattgc tcagcggtgg cagcagccaa ctcagcttcc tttcgggctt       120
tgttagcagc cggatctcag tggtggtggt ggtggtgctc gagttaaccg cggcctgcct       180
ggaatgaagg atattgtgtc ataccgccgt ccgcgaataa cgtgatgcct gtgacgtagc       240
tggcttcctt cgaagcaagc caggctgcta ctgcggcgat ctcctccggt tcgccgatat       300
atcccattgg aatcatgctt tctacatcag ctttctgttt agggtcagcg aatttttcag       360
cattgattgg cgtgttgatc gcacctggcc caatattatt gacgcgaatg cccttcggcg       420
cgtattccaa cgctaatgtt tctgtcatca gctttatccc gcctttactt gccgcatagt       480
```

```
ggacaaataa cggccaagga atcacttcgt gcacactgga catgttaatg acatttccct      540
tgatatcgtt ttctacgaaa tatttaatcg cttcacggct tcctaaaaag gcacccgtta      600
agttcgtgcc gatgacttta tcccaatcct tgagcggcat ttcgtgagat ggcacaggat      660
tttcaagacc ggcattatta atcataatat cgagtgtgcc gaactcctta attgccgttt      720
gcacgatatt ttttacatct tcctctttcg tgacatctcc ttggacgaca acagcttcac      780
cgcccgcctt gatgacctct tcttttacct cgttcggatc ttgtttatta ctataatagt      840
tgataaccac ttttgcctgc tccttgccga agcgaatggc catcgccttt ccgagccctg      900
aagcagctcc tgtaatagcg acgacttttc cttttaaatc cggatacatg tatatctcct      960
tgcggccgct cagaactgtg tcgggcgcat caccgcatca atgccgccat caatgacgat     1020
ctgcgcgcca tgcacatagc ttgcggccgg gctcatcaaa aaggcgatga ccgacgccat     1080
ctcggacggc tcggcacggc ggcccatggg aggaacgaac ttggcaatgg attcgccata     1140
gcgcgggtcc tgcaggcccg cctgcagcaa gggagtctcg gttgcaccgg gggcgatggt     1200
gttcaggcgc acgccagcct cgccccaggc ggcggcgcgt ttgcgcacag ccaccgtcaa     1260
agcattcttg ctgcccgcat aggccagatt tccgccctgc tctcccgcat gttcgacaat     1320
ggcgcgggcc ttggcttcct cgccggcttc cagtgccagc gccagtgggt tcttgtcaaa     1380
agccagatgc gcggaagcca cggacgagat gacgacggct gcgggctgat ggcctttttt     1440
cagcgctggc aaaaaggcat ccatcagctc ggtcgcgcca aaataattga ccgaaaccac     1500
attgccaagc accttggtct gcggtcccag gccggcgcac agcaccaggc cgtccatgcc     1560
cttgctgcac ttcgccagta catcggcaat cgcctgcttt cgaccttcgg ccgtcgagag     1620
atcggcaatc acttccgcat cgcgtatatc gatgcctacg atctggtgac cggccgcctc     1680
caggaccttg cgcgtagcgg caccaatgcc ggtggcgcag ccgcttatca cgatgatgga     1740
catcatatgt atatctcctt cttatactta actaatatac taagatgggg aattgttatc     1800
cgctcacaat tcccctatag tgagtcgtat taatttcgat tatgcggccg tgtacaatac     1860
gattactttc tgttcgactt aagcattata agcttctagt cgcggtagaa cgaccccatg     1920
tagcggatgt actcgtcctc ggtgtggttt gccttccagt cgtggacgga gtccttgttg     1980
cgctggccgg cgatgacgga gagctcctta cccagcttct caaaggcctc gtcaacgcac     2040
tcctcggggg tgagggcgat cttcatgacg gcctcgccct gcgggccgcc ggggaggttg     2100
gacagcaggc tgggggttag ggtggtgccg agggtgatga cctcgacgtc gacgccggtg     2160
ccctcgcact cgcaggccac ggcctcggtc atcttgagga tgaaggcctt gcccgcgccg     2220
tactggccgt tccaggggct ggagctgatg ccggtcatcg acgagacgtt gatcacggcg     2280
ccgcggtcct gggcggcaaa gatccgcatg tagtggtgga agcacttgag gaaggtcacg     2340
acgttgacgt tgatcatggc ctcgtgcttc tcccaggggg tgtcctggat cttaccgaag     2400
```

ctgtgcaggc aggccacgta gctcatgaag cccatgtcca ggccctcggt cgcggcgaag 2460
acggtctcgg cagcgccggg ctggctaaag tcggcgcgca cgaccttggt ctccacgccg 2520
taggtctcgc ggatctcgcc tgcgagcacg ttcagcttct cctcgcgacg ggcgaccatg 2580
acgacgttca tgccgccggc ggcgatcttc tcgcagaacg ccttgccgac gccctcggtc 2640
gcgcccagga tcaggcccca ctcaccgtac ttctccctca ggttcatatg tatatctcct 2700
tcttaaagtt aaacaaaatt atttctagag gggaattgtt atccgctcac aattccccta 2760
tagtgagtcg tattaatttc gcgggatcga gatctcgatc ctctacgccg gacgcatcgt 2820
ggccggcatc accggcgcca caggtgcggt tgctggcgcc tatatcgccg acatcaccga 2880
tggggaagat cgggctcgcc acttcgggct catgagcgct tgtttcggcg tgggtatggt 2940
ggcaggcccc gtggccgggg gactgttggg cgccatctcc ttgcatgcac cattccttgc 3000
ggcggcggtg ctcaacggcc tcaacctact actgggctgc ttcctaatgc aggagtcgca 3060
taagggagag cgtcgagatc ccggacacca tcgaatggcg caaaaccttt cgcggtatgg 3120
catgatagcg cccggaagag agtcaattca gggtggtgaa tgtgaaacca gtaacgttat 3180
acgatgtcgc agagtatgcc ggtgtctctt atcagaccgt ttcccgcgtg gtgaaccagg 3240
ccagccacgt ttctgcgaaa acgcgggaaa aagtggaagc ggcgatggcg gagctgaatt 3300
acattcccaa ccgcgtggca caacaactgg cgggcaaaca gtcgttgctg attggcgttg 3360
ccacctccag tctggccctg cacgcgccgt cgcaaattgt cgcggcgatt aaatctcgcg 3420
ccgatcaact gggtgccagc gtggtggtgt cgatggtaga acgaagcggc gtcgaagcct 3480
gtaaagcggc ggtgcacaat cttctcgcgc aacgcgtcag tgggctgatc attaactatc 3540
cgctggatga ccaggatgcc attgctgtgg aagctgcctg cactaatgtt ccggcgttat 3600
ttcttgatgt ctctgaccag acacccatca acagtattat tttctcccat gaagacggta 3660
cgcgactggg cgtggagcat ctggtcgcat tgggtcacca gcaaatcgcg ctgttagcgg 3720
gcccattaag ttctgtctcg gcgcgtctgc gtctggctgg ctggcataaa tatctcactc 3780
gcaatcaaat tcagccgata gcggaacggg aaggcgactg gagtgccatg tccggttttc 3840
aacaaaccat gcaaatgctg aatgagggca tcgttcccac tgcgatgctg gttgccaacg 3900
atcagatggc gctgggcgca atgcgcgcca ttaccgagtc cgggctgcgc gttggtgcgg 3960
atatctcggt agtgggatac gacgataccg aagacagctc atgttatatc ccgccgttaa 4020
ccaccatcaa acaggatttt cgcctgctgg ggcaaaccag cgtggaccgc ttgctgcaac 4080
tctctcaggg ccaggcggtg aagggcaatc agctgttgcc cgtctcactg gtgaaaagaa 4140
aaaccaccct ggcgcccaat acgcaaaccg cctctccccg cgcgttggcc gattcattaa 4200
tgcagctggc acgacaggtt tcccgactgg aaagcgggca gtgagcgcaa cgcaattaat 4260

gtaagttagc tcactcatta ggcaccggga tctcgaccga tgcccttgag agccttcaac 4320
ccagtcagct ccttccggtg ggcgcggggc atgactatcg tcgccgcact tatgactgtc 4380
ttctttatca tgcaactcgt aggacaggtg ccggcagcgc tctgggtcat tttcggcgag 4440
gaccgctttc gctggagcgc gacgatgatc ggcctgtcgc ttgcggtatt cggaatcttg 4500
cacgccctcg ctcaagcctt cgtcactggt cccgccacca aacgtttcgg cgagaagcag 4560
gccattatcg ccggcatggc ggccccacgg gtgcgcatga tcgtgctcct gtcgttgagg 4620
acccggctag gctggcgggg ttgccttact ggttagcaga atgaatcacc gatacgcgag 4680
cgaacgtgaa gcgactgctg ctgcaaaacg tctgcgacct gagcaacaac atgaatggtc 4740
ttcggtttcc gtgtttcgta aagtctggaa acgcggaagt cagcgccctg caccattatg 4800
ttccggatct gcatcgcagg atgctgctgg ctaccctgtg gaacacctac atctgtatta 4860
acgaagcgct ggcattgacc ctgagtgatt tttctctggt cccgccgcat ccataccgcc 4920
agttgtttac cctcacaacg ttccagtaac cgggcatgtt catcatcagt aacccgtatc 4980
gtgagcatcc tctctcgttt catcggtatc attaccccca tgaacagaaa tcccccttac 5040
acggaggcat cagtgaccaa acaggaaaaa accgccctta acatggcccg ctttatcaga 5100
agccagacat taacgcttct ggagaaactc aacgagctgg acgcggatga acaggcagac 5160
atctgtgaat cgcttcacga ccacgctgat gagctttacc gcagctgcct cgcgcgtttc 5220
ggtgatgacg gtgaaaacct ctgacacatg cagctcccgg agacggtcac agcttgtctg 5280
taagcggatg ccgggagcag acaagcccgt cagggcgcgt cagcgggtgt tggcgggtgt 5340
cggggcgcag ccatgaccca gtcacgtagc gatagcggag tgtatactgg cttaactatg 5400
cggcatcaga gcagattgta ctgagagtgc accatatatg cggtgtgaaa taccgcacag 5460
atgcgtaagg agaaaatacc gcatcaggcg ctcttccgct tcctcgctca ctgactcgct 5520
gcgctcggtc gttcggctgc ggcgagcggt atcagctcac tcaaaggcgg taatacggtt 5580
atccacagaa tcaggggata acgcaggaaa gaacatgtga gcaaaaggcc agcaaaaggc 5640
caggaaccgt aaaaaggccg cgttgctggc gtttttccat aggctccgcc cccctgacga 5700
gcatcacaaa aatcgacgct caagtcagag gtggcgaaac ccgacaggac tataaagata 5760
ccaggcgttt ccccctggaa gctccctcgt gcgctctcct gttccgaccc tgccgcttac 5820
cggatacctg tccgcctttc tcccttcggg aagcgtggcg ctttctcata gctcacgctg 5880
taggtatctc agttcggtgt aggtcgttcg ctccaagctg ggctgtgtgc acgaaccccc 5940
cgttcagccc gaccgctgcg ccttatccgg taactatcgt cttgagtcca acccggtaag 6000
acacgactta tcgccactgg cagcagccac tggtaacagg attagcagag cgaggtatgt 6060
aggcggtgct acagagttct tgaagtggtg gcctaactac ggctacacta gaaggacagt 6120
atttggtatc tgcgctctgc tgaagccagt taccttcgga aaaagagttg gtagctcttg 6180

atccggcaaa caaaccaccg ctggtagcgg tggttttttt gtttgcaagc agcagattac 6240
gcgcagaaaa aaaggatctc aagaagatcc tttgatcttt tctacggggt ctgacgctca 6300
gtggaacgaa aactcacgtt aagggatttt ggtcatgaac aataaaactg tctgcttaca 6360
taaacagtaa tacaaggggt gttatgagcc atattcaacg ggaaacgtct tgctctaggc 6420
cgcgattaaa ttccaacatg gatgctgatt tatatgggta taaatgggct cgcgataatg 6480
tcgggcaatc aggtgcgaca atctatcgat tgtatgggaa gcccgatgcg ccagagttgt 6540
ttctgaaaca tggcaaaggt agcgttgcca atgatgttac agatgagatg gtcagactaa 6600
actggctgac ggaatttatg cctcttccga ccatcaagca ttttatccgt actcctgatg 6660
atgcatggtt actcaccact gcgatccccg ggaaaacagc attccaggta ttagaagaat 6720
atcctgattc aggtgaaaat attgttgatg cgctggcagt gttcctgcgc cggttgcatt 6780
cgattcctgt ttgtaattgt ccttttaaca gcgatcgcgt atttcgtctc gctcaggcgc 6840
aatcacgaat gaataacggt ttggttgatg cgagtgattt tgatgacgag cgtaatggct 6900
ggcctgttga acaagtctgg aaagaaatgc ataaactttt gccattctca ccggattcag 6960
tcgtcactca tggtgatttc tcacttgata accttatttt tgacgagggg aaattaatag 7020
gttgtattga tgttggacga gtcggaatcg cagaccgata ccaggatctt gccatcctat 7080
ggaactgcct cggtgagttt tctccttcat tacagaaacg gctttttcaa aaatatggta 7140
ttgataatcc tgatatgaat aaattgcagt ttcatttgat gctcgatgag tttttctaag 7200
aattaattca tgagcggata catatttgaa tgtatttaga aaaataaaca aataggggtt 7260
ccgcgcacat ttccccgaaa agtgccacct gaaattgtaa acgttaatat tttgttaaaa 7320
ttcgcgttaa atttttgtta aatcagctca ttttttaacc aataggccga aatcggcaaa 7380
atcccttata aatcaaaaga atagaccgag atagggttga gtgttgttcc agtttggaac 7440
aagagtccac tattaaagaa cgtggactcc aacgtcaaag ggcgaaaaac cgtctatcag 7500
ggcgatggcc cactacgtga accatcaccc taatcaagtt ttttggggtc gaggtgccgt 7560
aaagcactaa atcggaaccc taaagggagc ccccgattta gagcttgacg gggaaagccg 7620
gcgaacgtgg cgagaaagga agggaagaaa gcgaaaggag cgggcgctag ggcgctggca 7680
agtgtagcgg tcacgctgcg cgtaaccacc acacccgccg cgcttaatgc gccgctacag 7740
ggcgcgtccc attcgcca 7758

<210> 20
<211> 8019
<212> DNA
<213> Artificial Sequence

<220>
<223> Plasmid p7(A)T3TG-A

<400> 20

atccggatat agttcctcct ttcagcaaaa aacccctcaa gacccgttta gaggccccaa 60
ggggttatgc tagttattgc tcagcggtgg cagcagccaa ctcagcttcc tttcgggctt 120
tgttagcagc cggatctcag tggtggtggt ggtggtgctc gagttaaccg cggcctgcct 180
ggaatgaagg atattgtgtc ataccgccgt ccgcgaataa cgtgatgcct gtgacgtagc 240
tggcttcctt cgaagcaagc caggctgcta ctgcggcgat ctcctccggt tcgccgatat 300
atcccattgg aatcatgctt tctacatcag ctttctgttt agggtcagcg aatttttcag 360
cattgattgg cgtgttgatc gcacctggcc caatattatt gacgcgaatg cccttcggcg 420
cgtattccaa cgctaatgtt tctgtcatca gctttatccc gcctttactt gccgcatagt 480
ggacaaataa cggccaagga atcacttcgt gcacactgga catgttaatg acatttccct 540
tgatatcgtt ttctacgaaa tatttaatcg cttcacggct tcctaaaaag gcacccgtta 600
agttcgtgcc gatgacttta tcccaatcct tgagcggcat ttcgtgagat ggcacaggat 660
tttcaagacc ggcattatta atcataatat cgagtgtgcc gaactcctta attgccgttt 720
gcacgatatt ttttacatct tcctctttcg tgacatctcc ttggacgaca acagcttcac 780
cgcccgcctt gatgacctct tcttttacct cgttcggatc ttgtttatta ctataatagt 840
tgataaccac ttttgcctgc tccttgccga agcgaatggc catcgccttt ccgagccctg 900
aagcagctcc tgtaatagcg acgacttttc cttttaaatc cggatacata tgtatatctc 960
cttcttatac ttaactaata tactaagatg ggaattgtt atccgctcac aattccccta 1020
tagtgagtcg tattaatttc gattatgcgg ccgtgtacaa tacgattact ttctgttcga 1080
cttaagcatt atgcggccgc tcagaactgt gtcgggcgca tcaccgcatc aatgccgcca 1140
tcaatgacga tctgcgcgcc atgcacatag cttgcggccg ggctcatcaa aaaggcgatg 1200
accgacgcca tctcggacgg ctcggcacgg cggcccatgg gaggaacgaa cttggcaatg 1260
gattcgccat agcgcgggtc ctgcaggccc gcctgcagca agggagtctc ggttgcaccg 1320
ggggcgatgg tgttcaggcg cacgccagcc tcgccccagg cggcggcgcg tttgcgcaca 1380
gccaccgtca aagcattctt gctgcccgca taggccagat ttccgccctg ctctcccgca 1440
tgttcgacaa tggcgcgggc cttggcttcc tcgccggctt ccagtgccag cgccagtggg 1500
ttcttgtcaa aagccagatg cgcggaagcc acggacgaga tgacgacggc tgcgggctga 1560
tggccttttt tcagcgctgg caaaaaggca tccatcagct cggtcgcgcc aaaataattg 1620
accgaaacca cattgccaag caccttggtc tgcggtccca ggccggcgca cagcaccagg 1680
ccgtccatgc ccttgctgca cttcgccagt acatcggcaa tcgcctgctt tcgaccttcg 1740
gccgtcgaga gatcggcaat cacttccgca tcgcgtatat cgatgcctac gatctggtga 1800
ccggccgcct ccaggacctt gcgcgtagcg gcaccaatgc cggtggcgca gccgcttatc 1860

acgatgatgg acatatgtat atctccttct tatacttaac taatatacta agatggggaa 1920
ttgttatccg ctcacaattc ccctatagtg agtcgtatta atttcgatta tgcggccgtg 1980
tacaatacga ttactttctg ttcgacttaa gcattataag cttctagtcg cggtagaacg 2040
accccatgta gcggatgtac tcgtcctcgg tgtggtttgc cttccagtcg tggacggagt 2100
ccttgttgcg ctggccggcg atgacggaga gctccttacc cagcttctca aaggcctcgt 2160
caacgcactc ctcgggggtg agggcgatct tcatgacggc ctcgccctgc gggccgccgg 2220
ggaggttgga cagcaggctg gggttagggg tggtgccgag ggtgatgacc tcgacgtcga 2280
cgccggtgcc ctcgcactcg caggccacgg cctcggtcat cttgaggatg aaggccttgc 2340
ccgcgccgta ctggccgttc caggggctgg agctgatgcc ggtcatcgac gagacgttga 2400
tcacggcgcc gcggtcctgg gcggcaaaga tccgcatgta gtggtggaag cacttgagga 2460
aggtcacgac gttgacgttg atcatggcct cgtgcttctc ccaggggggtg tcctggatct 2520
taccgaagct gtgcaggcag gccacgtagc tcatgaagcc catgtccagg ccctcggtcg 2580
cggcgaagac ggtctcggca gcgccgggct ggctaaagtc ggcgcgcacg accttggtct 2640
ccacgccgta ggtctcgcgg atctcgcctg cgagcacgtt cagcttctcc tcgcgacggg 2700
cgaccatgac gacgttcatg ccgccggcgg cgatcttctc gcagaacgcc ttgccgacgc 2760
cctcggtcgc gcccaggatc aggccccact caccgtactt ctccctcagg ttcatatgta 2820
tatctccttc ttaaagttaa acaaaattat ttctagaggg gaattgttat ccgctcacaa 2880
ttcccctata gtgagtcgta ttaatttcgc gggatcgaga tctcgatcct ctacgccgga 2940
cgcatcgtgg ccggcatcac cggcgccaca ggtgcggttg ctggcgccta tatcgccgac 3000
atcaccgatg gggaagatcg ggctcgccac ttcgggctca tgagcgcttg tttcggcgtg 3060
ggtatggtgg caggccccgt ggccggggga ctgttgggcg ccatctcctt gcatgcacca 3120
ttccttgcgg cggcggtgct caacggcctc aacctactac tgggctgctt cctaatgcag 3180
gagtcgcata agggagagcg tcgagatccc ggacaccatc gaatggcgca aaacctttcg 3240
cggtatggca tgatagcgcc cggaagagag tcaattcagg gtggtgaatg tgaaaccagt 3300
aacgttatac gatgtcgcag agtatgccgg tgtctcttat cagaccgttt cccgcgtggt 3360
gaaccaggcc agccacgttt ctgcgaaaac gcgggaaaaa gtggaagcgg cgatggcgga 3420
gctgaattac attcccaacc gcgtggcaca acaactggcg ggcaaacagt cgttgctgat 3480
tggcgttgcc acctccagtc tggccctgca cgcgccgtcg caaattgtcg cggcgattaa 3540
atctcgcgcc gatcaactgg gtgccagcgt ggtggtgtcg atggtagaac gaagcggcgt 3600
cgaagcctgt aaagcggcgg tgcacaatct tctcgcgcaa cgcgtcagtg ggctgatcat 3660
taactatccg ctggatgacc aggatgccat tgctgtggaa gctgcctgca ctaatgttcc 3720

ggcgttattt cttgatgtct ctgaccagac acccatcaac agtattattt tctcccatga 3780

agacggtacg cgactgggcg tggagcatct ggtcgcattg ggtcaccagc aaatcgcgct 3840

gttagcgggc ccattaagtt ctgtctcggc gcgtctgcgt ctggctggct ggcataaata 3900

tctcactcgc aatcaaattc agccgatagc ggaacgggaa ggcgactgga gtgccatgtc 3960

cggttttcaa caaaccatgc aaatgctgaa tgagggcatc gttcccactg cgatgctggt 4020

tgccaacgat cagatggcgc tgggcgcaat gcgcgccatt accgagtccg ggctgcgcgt 4080

tggtgcggat atctcggtag tgggatacga cgataccgaa gacagctcat gttatatccc 4140

gccgttaacc accatcaaac aggattttcg cctgctgggg caaaccagcg tggaccgctt 4200

gctgcaactc tctcagggcc aggcggtgaa gggcaatcag ctgttgcccg tctcactggt 4260

gaaaagaaaa accaccctgg cgcccaatac gcaaaccgcc tctccccgcg cgttggccga 4320

ttcattaatg cagctggcac gacaggtttc ccgactggaa agcgggcagt gagcgcaacg 4380

caattaatgt aagttagctc actcattagg caccgggatc tcgaccgatg cccttgagag 4440

ccttcaaccc agtcagctcc ttccggtggg cgcggggcat gactatcgtc gccgcactta 4500

tgactgtctt ctttatcatg caactcgtag gacaggtgcc ggcagcgctc tgggtcattt 4560

tcggcgagga ccgctttcgc tggagcgcga cgatgatcgg cctgtcgctt gcggtattcg 4620

gaatcttgca cgccctcgct caagccttcg tcactggtcc cgccaccaaa cgtttcggcg 4680

agaagcaggc cattatcgcc ggcatggcgg ccccacgggt gcgcatgatc gtgctcctgt 4740

cgttgaggac ccggctaggc tggcggggtt gccttactgg ttagcagaat gaatcaccga 4800

tacgcgagcg aacgtgaagc gactgctgct gcaaaacgtc tgcgacctga gcaacaacat 4860

gaatggtctt cggtttccgt gtttcgtaaa gtctggaaac gcggaagtca gcgccctgca 4920

ccattatgtt ccggatctgc atcgcaggat gctgctggct accctgtgga acacctacat 4980

ctgtattaac gaagcgctgg cattgaccct gagtgatttt tctctggtcc cgccgcatcc 5040

ataccgccag ttgtttaccc tcacaacgtt ccagtaaccg ggcatgttca tcatcagtaa 5100

cccgtatcgt gagcatcctc tctcgtttca tcggtatcat taccccatg aacagaaatc 5160

cccttacac ggaggcatca gtgaccaaac aggaaaaaac cgcccttaac atggcccgct 5220

ttatcagaag ccagacatta acgcttctgg agaaactcaa cgagctggac gcggatgaac 5280

aggcagacat ctgtgaatcg cttcacgacc acgctgatga gctttaccgc agctgcctcg 5340

cgcgtttcgg tgatgacggt gaaaacctct gacacatgca gctcccggag acggtcacag 5400

cttgtctgta agcggatgcc gggagcagac aagcccgtca gggcgcgtca gcgggtgttg 5460

gcgggtgtcg gggcgcagcc atgacccagt cacgtagcga tagcggagtg tatactggct 5520

taactatgcg gcatcagagc agattgtact gagagtgcac catatatgcg gtgtgaaata 5580

ccgcacagat gcgtaaggag aaaataccgc atcaggcgct cttccgcttc ctcgctcact 5640

gactcgctgc gctcggtcgt tcggctgcgg cgagcggtat cagctcactc aaaggcggta 5700
atacggttat ccacagaatc aggggataac gcaggaaaga acatgtgagc aaaaggccag 5760
caaaaggcca ggaaccgtaa aaaggccgcg ttgctggcgt ttttccatag gctccgcccc 5820
cctgacgagc atcacaaaaa tcgacgctca agtcagaggt ggcgaaaccc gacaggacta 5880
taaagatacc aggcgtttcc ccctggaagc tccctcgtgc gctctcctgt tccgaccctg 5940
ccgcttaccg gatacctgtc cgcctttctc ccttcgggaa gcgtggcgct ttctcatagc 6000
tcacgctgta ggtatctcag ttcggtgtag gtcgttcgct ccaagctggg ctgtgtgcac 6060
gaaccccccg ttcagcccga ccgctgcgcc ttatccggta actatcgtct tgagtccaac 6120
ccggtaagac acgacttatc gccactggca gcagccactg gtaacaggat tagcagagcg 6180
aggtatgtag gcggtgctac agagttcttg aagtggtggc ctaactacgg ctacactaga 6240
aggacagtat ttggtatctg cgctctgctg aagccagtta ccttcggaaa aagagttggt 6300
agctcttgat ccggcaaaca aaccaccgct ggtagcggtg gtttttttgt ttgcaagcag 6360
cagattacgc gcagaaaaaa aggatctcaa gaagatcctt tgatcttttc tacggggtct 6420
gacgctcagt ggaacgaaaa ctcacgttaa gggattttgg tcatgagatt atcaaaaagg 6480
atcttcacct agatcctttt aaattaaaaa tgaagtttta aatcaatcta aagtatatat 6540
gagtaaactt ggtctgacag ttaccaatgc ttaatcagtg aggcacctat ctcagcgatc 6600
tgtctatttc gttcatccat agttgcctga ctccccgtcg tgtagataac tacgatacgg 6660
gagggcttac catctggccc cagtgctgca atgataccgc gagacccacg ctcaccggct 6720
ccagatttat cagcaataaa ccagccagcc ggaagggccg agcgcagaag tggtcctgca 6780
actttatccg cctccatcca gtctattaat tgttgccggg aagctagagt aagtagttcg 6840
ccagttaata gtttgcgcaa cgttgttgcc attgctgcag gcatcgtggt gtcacgctcg 6900
tcgtttggta tggcttcatt cagctccggt tcccaacgat caaggcgagt tacatgatcc 6960
cccatgttgt gcaaaaaagc ggttagctcc ttcggtcctc cgatcgttgt cagaagtaag 7020
ttggccgcag tgttatcact catggttatg gcagcactgc ataattctct tactgtcatg 7080
ccatccgtaa gatgcttttc tgtgactggt gagtactcaa ccaagtcatt ctgagaatag 7140
tgtatgcggc gaccgagttg ctcttgcccg gcgtcaatac gggataatac cgcgccacat 7200
agcagaactt taaaagtgct catcattgga aaacgttctt cggggcgaaa actctcaagg 7260
atcttaccgc tgttgagatc cagttcgatg taacccactc gtgcacccaa ctgatcttca 7320
gcatctttta ctttcaccag cgtttctggg tgagcaaaaa caggaaggca aaatgccgca 7380
aaaaagggaa taagggcgac acggaaatgt tgaatactca tactcttcct ttttcaatat 7440
tattgaagca tttatcaggg ttattgtctc atgagcggat acatatttga atgtatttag 7500

```
aaaaataaac aaataggggt tccgcgcaca tttccccgaa aagtgccacc tgaaattgta      7560
aacgttaata ttttgttaaa attcgcgtta aatttttgtt aaatcagctc atttttttaac      7620
caataggccg aaatcggcaa aatcccttat aaatcaaaag aatagaccga gatagggttg      7680
agtgttgttc cagtttggaa caagagtcca ctattaaaga acgtggactc caacgtcaaa      7740
gggcgaaaaa ccgtctatca gggcgatggc ccactacgtg aaccatcacc ctaatcaagt      7800
ttttggggt cgaggtgccg taaagcacta aatcggaacc ctaaagggag cccccgattt      7860
agagcttgac ggggaaagcc ggcgaacgtg gcgagaaagg aagggaagaa agcgaaagga      7920
gcgggcgcta gggcgctggc aagtgtagcg gtcacgctgc gcgtaaccac cacacccgcc      7980
gcgcttaatg cgccgctaca gggcgcgtcc cattcgcca                             8019
```

<210> 21
<211> 7886
<212> DNA
<213> Artificial Sequence

<220>
<223> Plasmid p7(A)T3TG-K

<400> 21

```
atccggatat agttcctcct ttcagcaaaa aacccctcaa gacccgttta gaggccccaa      60
ggggttatgc tagttattgc tcagcggtgg cagcagccaa ctcagcttcc tttcgggctt     120
tgttagcagc cggatctcag tggtggtggt ggtggtgctc gagttaaccg cggcctgcct     180
ggaatgaagg atattgtgtc ataccgccgt ccgcgaataa cgtgatgcct gtgacgtagc     240
tggcttcctt cgaagcaagc caggctgcta ctgcggcgat ctcctccggt tcgccgatat     300
atcccattgg aatcatgctt tctacatcag ctttctgttt agggtcagcg aatttttcag     360
cattgattgg cgtgttgatc gcacctggcc caatattatt gacgcgaatg cccttcggcg     420
cgtattccaa cgctaatgtt tctgtcatca gctttatccc gcctttactt gccgcatagt     480
ggacaaataa cggccaagga atcacttcgt gcacactgga catgttaatg acatttccct     540
tgatatcgtt ttctacgaaa tatttaatcg cttcacggct tcctaaaaag gcacccgtta     600
agttcgtgcc gatgacttta tcccaatcct tgagcggcat ttcgtgagat ggcacaggat     660
tttcaagacc ggcattatta atcataatat cgagtgtgcc gaactcctta attgccgttt     720
gcacgatatt ttttacatct tcctctttcg tgacatctcc ttggacgaca acagcttcac     780
cgcccgcctt gatgacctct tcttttacct cgttcggatc ttgtttatta ctataatagt     840
tgataaccac ttttgcctgc tccttgccga agcgaatggc catcgccttt ccgagccctg     900
aagcagctcc tgtaatagcg acgacttttc cttttaaatc cggatacata tgtatatctc     960
cttcttatac ttaactaata tactaagatg ggaattgtt atccgctcac aattccccta     1020
tagtgagtcg tattaatttc gattatgcgg ccgtgtacaa tacgattact ttctgttcga    1080
```

```
cttaagcatt atgcggccgc tcagaactgt gtcgggcgca tcaccgcatc aatgccgcca     1140
tcaatgacga tctgcgcgcc atgcacatag cttgcggccg ggctcatcaa aaaggcgatg     1200
accgacgcca tctcggacgg ctcggcacgg cggcccatgg gaggaacgaa cttggcaatg     1260
gattcgccat agcgcgggtc ctgcaggccc gcctgcagca agggagtctc ggttgcaccg     1320
ggggcgatgg tgttcaggcg cacgccagcc tcgccccagg cggcggcgcg tttgcgcaca     1380
gccaccgtca aagcattctt gctgcccgca taggccagat ttccgccctg ctctcccgca     1440
tgttcgacaa tggcgcgggc cttggcttcc tcgccggctt ccagtgccag cgccagtggg     1500
ttcttgtcaa aagccagatg cgcggaagcc acggacgaga tgacgacggc tgcgggctga     1560
tggccttttt tcagcgctgg caaaaaggca tccatcagct cggtcgcgcc aaaataattg     1620
accgaaacca cattgccaag caccttggtc tgcggtccca ggccggcgca cagcaccagg     1680
ccgtccatgc ccttgctgca cttcgccagt acatcggcaa tcgcctgctt tcgaccttcg     1740
gccgtcgaga gatcggcaat cacttccgca tcgcgtatat cgatgcctac gatctggtga     1800
ccggccgcct ccaggacctt gcgcgtagcg gcaccaatgc cggtggcgca gccgcttatc     1860
acgatgatgg acatatgtat atctccttct tatacttaac taatatacta agatggggaa     1920
ttgttatccg ctcacaattc ccctatagtg agtcgtatta atttcgatta tgcggccgtg     1980
tacaatacga ttactttctg ttcgacttaa gcattataag cttctagtcg cggtagaacg     2040
accccatgta gcggatgtac tcgtcctcgg tgtggtttgc cttccagtcg tggacggagt     2100
ccttgttgcg ctggccggcg atgacggaga gctccttacc cagcttctca aaggcctcgt     2160
caacgcactc ctcgggggtg agggcgatct tcatgacggc ctcgccctgc gggccgccgg     2220
ggaggttgga cagcaggctg gggttaggg tggtgccgag ggtgatgacc tcgacgtcga     2280
cgccggtgcc ctcgcactcg caggccacgg cctcggtcat cttgaggatg aaggccttgc     2340
ccgcgccgta ctggccgttc caggggctgg agctgatgcc ggtcatcgac gagacgttga     2400
tcacggcgcc gcggtcctgg gcggcaaaga tccgcatgta gtggtggaag cacttgagga     2460
aggtcacgac gttgacgttg atcatggcct cgtgcttctc ccaggggggtg tcctggatct     2520
taccgaagct gtgcaggcag gccacgtagc tcatgaagcc catgtccagg ccctcggtcg     2580
cggcgaagac ggtctcggca gcgccgggct ggctaaagtc ggcgcgcacg accttggtct     2640
ccacgccgta ggtctcgcgg atctcgcctg cgagcacgtt cagcttctcc tcgcgacggg     2700
cgaccatgac gacgttcatg ccgccggcgg cgatcttctc gcagaacgcc ttgccgacgc     2760
cctcggtcgc gcccaggatc aggccccact caccgtactt ctccctcagg ttcatatgta     2820
tatctccttc ttaaagttaa acaaaattat ttctagaggg gaattgttat ccgctcacaa     2880
ttcccctata gtgagtcgta ttaatttcgc gggatcgaga tctcgatcct ctacgccgga     2940
```

cgcatcgtgg ccggcatcac cggcgccaca ggtgcggttg ctggcgccta tatcgccgac 3000
atcaccgatg gggaagatcg ggctcgccac ttcgggctca tgagcgcttg tttcggcgtg 3060
ggtatggtgg caggccccgt ggccggggga ctgttgggcg ccatctcctt gcatgcacca 3120
ttccttgcgg cggcggtgct caacggcctc aacctactac tgggctgctt cctaatgcag 3180
gagtcgcata agggagagcg tcgagatccc ggacaccatc gaatggcgca aaacctttcg 3240
cggtatggca tgatagcgcc cggaagagag tcaattcagg gtggtgaatg tgaaaccagt 3300
aacgttatac gatgtcgcag agtatgccgg tgtctcttat cagaccgttt cccgcgtggt 3360
gaaccaggcc agccacgttt ctgcgaaaac gcgggaaaaa gtggaagcgg cgatggcgga 3420
gctgaattac attcccaacc gcgtggcaca acaactggcg ggcaaacagt cgttgctgat 3480
tggcgttgcc acctccagtc tggccctgca cgcgccgtcg caaattgtcg cggcgattaa 3540
atctcgcgcc gatcaactgg gtgccagcgt ggtggtgtcg atggtagaac gaagcggcgt 3600
cgaagcctgt aaagcggcgg tgcacaatct tctcgcgcaa cgcgtcagtg ggctgatcat 3660
taactatccg ctggatgacc aggatgccat tgctgtggaa gctgcctgca ctaatgttcc 3720
ggcgttattt cttgatgtct ctgaccagac acccatcaac agtattattt tctcccatga 3780
agacggtacg cgactgggcg tggagcatct ggtcgcattg ggtcaccagc aaatcgcgct 3840
gttagcgggc ccattaagtt ctgtctcggc gcgtctgcgt ctggctggct ggcataaata 3900
tctcactcgc aatcaaattc agccgatagc ggaacgggaa ggcgactgga gtgccatgtc 3960
cggttttcaa caaaccatgc aaatgctgaa tgagggcatc gttcccactg cgatgctggt 4020
tgccaacgat cagatggcgc tgggcgcaat gcgcgccatt accgagtccg ggctgcgcgt 4080
tggtgcggat atctcggtag tgggatacga cgataccgaa gacagctcat gttatatccc 4140
gccgttaacc accatcaaac aggattttcg cctgctgggg caaaccagcg tggaccgctt 4200
gctgcaactc tctcagggcc aggcggtgaa gggcaatcag ctgttgcccg tctcactggt 4260
gaaaagaaaa accaccctgg cgcccaatac gcaaaccgcc tctccccgcg cgttggccga 4320
ttcattaatg cagctggcac gacaggtttc ccgactggaa agcgggcagt gagcgcaacg 4380
caattaatgt aagttagctc actcattagg caccgggatc tcgaccgatg cccttgagag 4440
ccttcaaccc agtcagctcc ttccggtggg cgcggggcat gactatcgtc gccgcactta 4500
tgactgtctt ctttatcatg caactcgtag gacaggtgcc ggcagcgctc tgggtcattt 4560
tcggcgagga ccgctttcgc tggagcgcga cgatgatcgg cctgtcgctt gcggtattcg 4620
gaatcttgca cgccctcgct caagccttcg tcactggtcc cgccaccaaa cgtttcggcg 4680
agaagcaggc cattatcgcc ggcatggcgg ccccacgggt gcgcatgatc gtgctcctgt 4740
cgttgaggac ccggctaggc tggcggggtt gccttactgg ttagcagaat gaatcaccga 4800
tacgcgagcg aacgtgaagc gactgctgct gcaaaacgtc tgcgacctga gcaacaacat 4860

gaatggtctt cggtttccgt gtttcgtaaa gtctggaaac gcggaagtca gcgccctgca 4920
ccattatgtt ccggatctgc atcgcaggat gctgctggct accctgtgga acacctacat 4980
ctgtattaac gaagcgctgg cattgaccct gagtgatttt tctctggtcc cgccgcatcc 5040
ataccgccag ttgtttaccc tcacaacgtt ccagtaaccg ggcatgttca tcatcagtaa 5100
cccgtatcgt gagcatcctc tctcgtttca tcggtatcat tacccccatg aacagaaatc 5160
cccttacac ggaggcatca gtgaccaaac aggaaaaaac cgcccttaac atggcccgct 5220
ttatcagaag ccagacatta acgcttctgg agaaactcaa cgagctggac gcggatgaac 5280
aggcagacat ctgtgaatcg cttcacgacc acgctgatga gctttaccgc agctgcctcg 5340
cgcgtttcgg tgatgacggt gaaaacctct gacacatgca gctcccggag acggtcacag 5400
cttgtctgta agcggatgcc gggagcagac aagcccgtca gggcgcgtca gcgggtgttg 5460
gcgggtgtcg gggcgcagcc atgacccagt cacgtagcga tagcggagtg tatactggct 5520
taactatgcg gcatcagagc agattgtact gagagtgcac catatatgcg gtgtgaaata 5580
ccgcacagat gcgtaaggag aaaataccgc atcaggcgct cttccgcttc ctcgctcact 5640
gactcgctgc gctcggtcgt tcggctgcgg cgagcggtat cagctcactc aaaggcggta 5700
atacggttat ccacagaatc aggggataac gcaggaaaga acatgtgagc aaaaggccag 5760
caaaaggcca ggaaccgtaa aaaggccgcg ttgctggcgt ttttccatag gctccgcccc 5820
cctgacgagc atcacaaaaa tcgacgctca agtcagaggt ggcgaaaccc gacaggacta 5880
taaagatacc aggcgtttcc ccctggaagc tccctcgtgc gctctcctgt tccgaccctg 5940
ccgcttaccg gatacctgtc cgcctttctc ccttcgggaa gcgtggcgct ttctcatagc 6000
tcacgctgta ggtatctcag ttcggtgtag gtcgttcgct ccaagctggg ctgtgtgcac 6060
gaaccccccg ttcagcccga ccgctgcgcc ttatccggta actatcgtct tgagtccaac 6120
ccggtaagac acgacttatc gccactggca gcagccactg gtaacaggat tagcagagcg 6180
aggtatgtag gcggtgctac agagttcttg aagtggtggc ctaactacgg ctacactaga 6240
aggacagtat ttggtatctg cgctctgctg aagccagtta ccttcggaaa aagagttggt 6300
agctcttgat ccggcaaaca aaccaccgct ggtagcggtg gtttttttgt ttgcaagcag 6360
cagattacgc gcagaaaaaa aggatctcaa gaagatcctt tgatcttttc tacggggtct 6420
gacgctcagt ggaacgaaaa ctcacgttaa gggattttgg tcatgaacaa taaaactgtc 6480
tgcttacata aacagtaata caaggggtgt tatgagccat attcaacggg aaacgtcttg 6540
ctctaggccg cgattaaatt ccaacatgga tgctgattta tatgggtata aatgggctcg 6600
cgataatgtc gggcaatcag gtgcgacaat ctatcgattg tatgggaagc ccgatgcgcc 6660
agagttgttt ctgaaacatg gcaaaggtag cgttgccaat gatgttacag atgagatggt 6720

```
cagactaaac tggctgacgg aatttatgcc tcttccgacc atcaagcatt ttatccgtac     6780
tcctgatgat gcatggttac tcaccactgc gatccccggg aaaacagcat tccaggtatt     6840
agaagaatat cctgattcag gtgaaaatat tgttgatgcg ctggcagtgt tcctgcgccg     6900
gttgcattcg attcctgttt gtaattgtcc ttttaacagc gatcgcgtat ttcgtctcgc     6960
tcaggcgcaa tcacgaatga ataacggttt ggttgatgcg agtgattttg atgacgagcg     7020
taatggctgg cctgttgaac aagtctggaa agaaatgcat aaacttttgc cattctcacc     7080
ggattcagtc gtcactcatg gtgatttctc acttgataac cttatttttg acgaggggaa     7140
attaataggt tgtattgatg ttggacgagt cggaatcgca gaccgatacc aggatcttgc     7200
catcctatgg aactgcctcg gtgagttttc tccttcatta cagaaacggc tttttcaaaa     7260
atatggtatt gataatcctg atatgaataa attgcagttt catttgatgc tcgatgagtt     7320
tttctaagaa ttaattcatg agcggataca tatttgaatg tatttagaaa aataaacaaa     7380
taggggttcc gcgcacattt ccccgaaaag tgccacctga aattgtaaac gttaatattt     7440
tgttaaaatt cgcgttaaat ttttgttaaa tcagctcatt ttttaaccaa taggccgaaa     7500
tcggcaaaat cccttataaa tcaaaagaat agaccgagat agggttgagt gttgttccag     7560
tttggaacaa gagtccacta ttaaagaacg tggactccaa cgtcaaaggg cgaaaaaccg     7620
tctatcaggg cgatggccca ctacgtgaac catcacccta atcaagtttt ttggggtcga     7680
ggtgccgtaa agcactaaat cggaacccta aagggagccc ccgatttaga gcttgacggg     7740
gaaagccggc gaacgtggcg agaaaggaag ggaagaaagc gaaaggagcg ggcgctaggg     7800
cgctggcaag tgtagcggtc acgctgcgcg taaccaccac acccgccgcg cttaatgcgc     7860
cgctacaggg cgcgtcccat tcgcca                                          7886
```